# EUROPEAN PATENT APPLICATION

(11) **EP 3 090 751 A1**
(43) Date of publication of application: **09.11.2016**
(21) Application number: 15166627.8
(22) Date of filing: 06.05.2015
(51) Int. Cl.: A61K 35/17

(54) **MOLECULAR PROFILING OF CD8 T-CELLS IN AUTOCHTHONOUS MELANOMA IDENTIFIES MAF AS DRIVER OF EXHAUSTION**

(71) Applicant: Université de Lausanne, 1011 Lausanne (CH)
(72) Inventor: Verdeil, Grégory, 74500 Saint-Paul-en-Chablais (FR); Speiser, Daniel, 1273 Arzier (CH); Schmitt-Verhulst, Anne-Marie, 13007 Marseille (FR)
(74) Representative: KATZAROV S.A.

(57) **Abstract**

The invention relates to a pharmaceutical composition comprising empowered T cells for use in a method for treating cancer in said patient, wherein said empowered T cells do not express c-Maf or in which the expression or activity of c-Maf is abolished or inhibited.

## Description

### Field of the Invention

The invention relates to a pharmaceutical composition comprising patient's empowered autologous T cells for use in a method for treating cancer in said patient.

### BACKGROUND OF THE INVENTION

The adaptive immune system plays a central, yet complex role in controlling tumor growth. It can suppress tumors by destroying cancer cells or inhibiting their growth. It can also promote tumor progression either by selecting for tumor cells that are more fit to survive an immune attack or by establishing conditions within the tumor microenvironment that facilitate tumor outgrowth. Nevertheless the presence of CD8 T-cells associated with expression of effector molecules, like Granzyme B (GZMB) or perforin, inside primary tumors has been correlated with a better prognosis for patients affected with a wide variety of cancers. In spite of this, T-cells infiltrating tumors often share features with exhausted T-cells in chronic infection. The molecular bases characterizing this state have been thoroughly detailed in previous studies using LCMV and other viral chronic infections. In both cases CD8 T-cells express inhibitory receptors including PD-1, Lag-3 and CTLA-4. It is now well recognized that these receptors inhibit T-cell mediated protection from chronic infections and tumors. In particular, blockade of PD-1 has been shown to restore function in exhausted CD8 T-cells during chronic viral infection. Accordingly, the therapeutic use of "blocking" antibodies specific for CTLA-4, PD-1 and PD-L1 is increasingly useful for cancer patients. This approach represents a breakthrough for the treatment of patients with various solid tumors. However, except for inhibitory receptors, mechanisms that regulate tumor-induced T-cell exhaustion are poorly documented. While several transcription factors (T-bet, Blimp-1 and Batf) have been shown to play a role in chronic infection, little is known concerning the establishment of T-cell exhaustion in cancer.

US 2014/0127268 (Sieweke, INSERM) relates to an ex vivo method for expanding monocytes, macrophages or dendritic cells, which method comprises inhibiting the expression or the activity of MafB and c-Maf in monocytes, macrophages or dendritic cells; and expanding the cells in the presence of at least one cytokine or an agonist of cytokine receptor signalling. Myeloid cells (including monocytes, macrophages and dendritic cells) naturally express MafB and c-Maf however it is not the case for CD8 T lymphocytes. T lymphocytes ("anti-tumor T cells") can eliminate cancer and chronic infections, provided that they are sufficiently "empowered" for robust and prolonged action against disease. Naturally in patients, however, these T cells are frequently "exhausted", i.e. they are dysfunctional.

Adoptive cell transfer therapy has shown some great success in treating patients with solid tumors, especially in the case of melanoma. It consists in harvesting T cells from a patient, amplify tumor specific T cells ex-vivo and, after reaching a high enough number of cells, transfer back these cells to the same patient. However, one major problem to solve to increase the efficiency of this therapy is to increase the survival and functions of the transferred cells in the patient and their resistance to the immunosuppressive environment created by the tumor.

### BRIEF DESCRIPTION OF THE INVENTION

It is one goal of the present invention to solve this problem by inactivating the expression of the gene encoding for MAF before transferring the cells back in the patient. Applicants have surprisingly shown that Maf is a transcription factor that dampens survival and effector function of CD8 T cells during a response versus a tumor and that inactivating this gene in tumor specific CD8 T cells leads to an increased response toward a tumor and an increased survival of the cells in vivo.

Contrary to the teaching of US 2014/0127268 Applicants target c-Maf in CD8 T lymphocytes to increase survival and anti-tumor response in a protocol of adoptive immunotherapy. Expression of c-Maf in CD8 T cells has never been established in vivo. Applicants showed for the first time that c-Maf is expressed in dysfunctional CD8 T cells infiltrated in tumors and that this expression prevents an efficient response of the CD8 T cells toward the tumor, i.e. its elimination.

One of the objects of the present invention is to provide a pharmaceutical composition comprising patient's empowered autologous T cells, preferably CD8 T cells; for use in a method for treating cancer in said patient, wherein said empowered autologous T cells do not express c-Maf or in which the expression or activity of c-Maf is abolished or inhibited.

Another object of the present invention is to provide an ex vivo method for adoptive cell transfer, which method comprises inhibiting the expression or the activity of c-Maf in patients' autologous T cells, preferably CD8 T cells; and expanding said cells.

An object of the invention is also a T cell population, preferably CD8 T cells obtainable by the above method.

Another object of the invention is a T cell population, preferably CD8 T cells, which does not express c-Maf or in which the expression or activity of MafB and c-Maf is abolished or inhibited.

Such empowered T cells, preferably empowered CD8 T cells are useful in a pharmaceutical composition, where they are in combination with a pharmaceutically acceptable carrier.

A yet further object is an *ex vivo* method for empowering T cells in adoptive cell transfer, the method comprises:
collecting patients' autologous T cells,
*ex vivo* inhibiting the expression or the activity of c-Maf in said patients' autologous T cells and expanding said T cells to be suitable for a subsequent adoptive cell transfer to said patient.

Also provided are inhibitors of c-Maf expression in patient's T cells for use in a method for treating cancer in said patient, wherein said patient's T cells are ex vivo empowered by said inhibitors of c-Maf expression so as not to express c-Maf and whereas said empowered patient's T cells are suitable for adoptive cell transfer in said patient.

Other objects and advantages of the invention will become apparent to those skilled in the art from a review of the ensuing detailed description, which proceeds with reference to the following illustrative drawings, and the attendant claims.

### BRIEF DESCRIPTION OF THE FIGURES

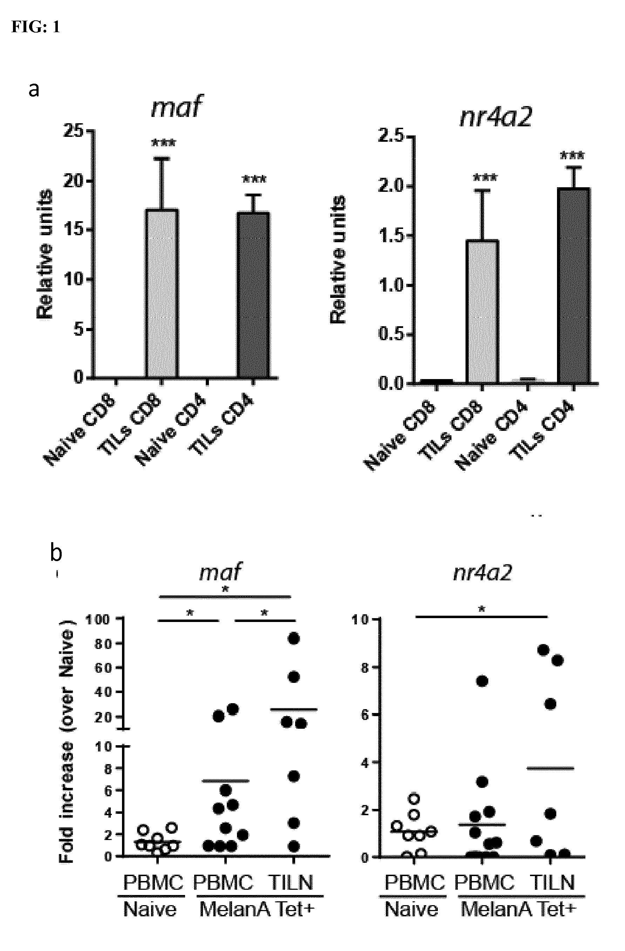

**Figure 1****: illustrates that *Maf* and *nr4a2* are overexpressed in mouse and human CD8+ TILs.**
   (a) After the analysis of the microarrays, fold change between exhausted TILs (TILs) and naive (naive) CD8 T-cells and between exhausted TILs and activated (act) CD8 T-cells were calculated. Some genes of interest with a p-value lower than 0.05 in both conditions and a log₂ fold change greater than 1 in both conditions are shown. (b) CD4 and CD8 T-cells were sorted from tumors of TiRP mice (3 independent samples). RNA levels for *maf* and *nr4a2* from these cells (Exh) were compared to those from naive CD4 and CD8 T-cells by RT-QPCR. (c) Applicants compared by quantitative RT-PCR the level of *Maf* and *Nr4a2* in Naive T cells isolated from Peripheral Blood Mononuclear Cells (PBMC) and Melan-A/MART-1 specific CD8 T cells from PBMC or metastasized LN (TILN) of melanoma patients.

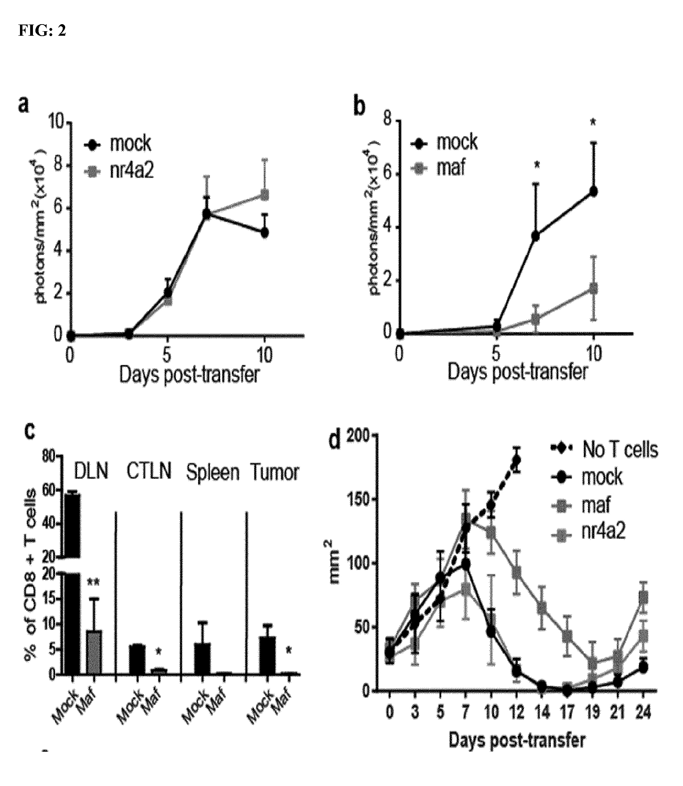

**Figure 2****: shows the overexpression of *maf*, but not *nr4a2,* dampens the anti-tumor response by CD8 T-cells.** (a-d) TCRP1A-luc CD8 T-cells were activated and transduced *in-vitro* with a retrovirus encoding either *maf* (maf) or *nr4a2* (nr4a2) coupled to GFP expression or with a construct coding for GFP only (Mock). Two days after transduction, GFP-expressing CD8 T-cells were FACs sorted and 5.10⁴ of GFP+ TCRP1A-luc CD8 T-cells were injected in Rag^{-/-}B10.D2 mice, previously injected with 10⁶ P511 mastocytoma (7 days before). (a, b) P511 infiltration by TCRP1A-luc T-cells transduced with the indicated construct was monitored every 2-3 days by bioluminescence. (c) At day 7 post-transfer, percentages of TCRP1A CD8 T-cells among CD45+ cells (Mock- or maf-transduced) from draining LN (DLN), contralateral LN (CTLN), spleen and TILs from 2 pooled experiments were determined. (d) Tumor growth was monitored every 2-3 days using a caliper.

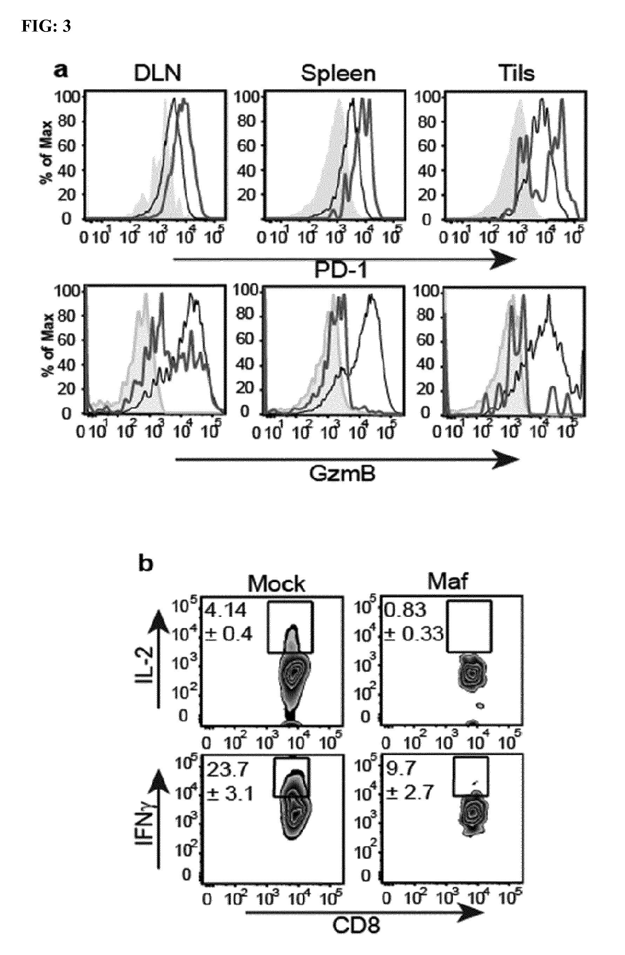

**Figure 3****: shows the overexpression of *maf* during anti-tumor response polarizes CD8 T-cells toward an exhausted phenotype.** (a-e) TCRP1A CD8 T-cells were activated and transduced *in-vitro* with a retrovirus encoding either *maf* (maf - grey) coupled to GFP expression or with a construct encoding GFP only (Mock - black). Two days after transduction GFP-expressing CD8 T-cells were FACS-sorted and injected (5.10⁴) in Rag^{-/-} B10.D2 mice, previously inoculated with 10⁶ P511 mastocytoma (7 days earlier). At day 7 post-transfer, mice were sacrificed. Cells from tumor draining LN (DLN), spleen or TILs were directly labeled (a) or restimulated and labeled (b) for the indicated molecules and analyzed by flow cytometry. (b) Numbers indicate the average of positive cells from 8 mice per condition from 2 independent experiments.

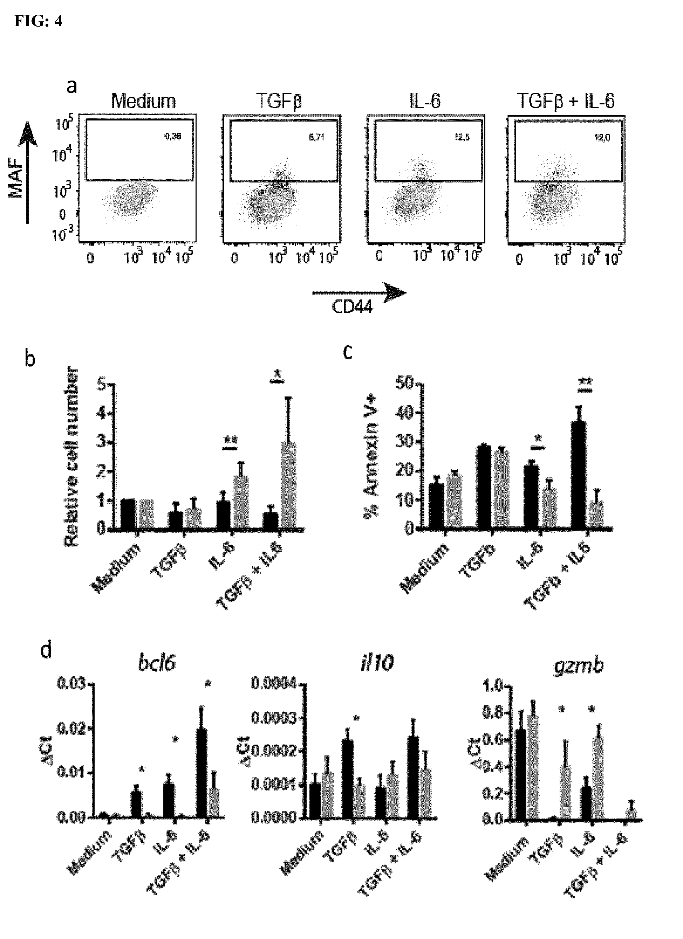

**Figure 4****: illustrates the presence of TGFβ or/and IL-6 during CD8 T-cell activation induces *maf* expression.** (a) P14 T-cells were activated *in-vitro* with GP33 peptide (10⁻⁶M) in the presence of IL-6 (10 ng/ml) and/or TGFβ (10ng/ml) for 72h. CD8 T cells were labelled with the indicated Ab and analyzed by flow cytometry. Levels of MAF in P14 T cells from maT-Cre+ maf^{fl/fl} mice (grey) or from Cre- littermates (black)(a) and average of the percentage of AnnexinV+ cells in the various conditions of culture (c) are shown. P14 T cell numbers were determined 72h post-stimulation and normalized according to the peptide only condition in 3 independent experiments (b). In (d) RNA from the two types of P14 T cells were analyzed by RT-QPCR. Pooled relative expressions compared to b2-microglobulin (ΔCt) for the indicated molecules and from 3 independent experiments are shown.

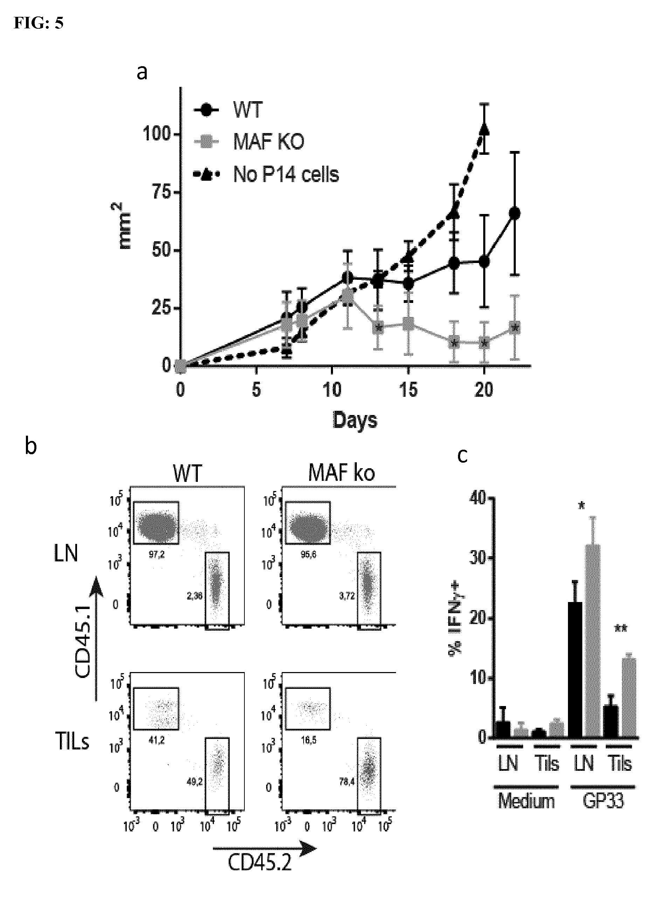

**Figure 5****: shows the inactivation of *maf* in tumor specific CD8 T cells increases tumor infiltration and elimination**
   C57Bl/6 CD45.1+ mice were injected s.c with 0.3x10⁵ B16-GP33 melanoma cells. One week after mice were adoptively transferred with 5.10⁶ *in vitro* pre-activated CD45.2+ P14 T cells from maT-Cre+ maf^{fl/fl} mice (8 mice - grey) or from Cre- littermates (8 mice - black) or left untreated (4 mice- dotted line). Tumor growth was assessed with a caliper every 2/3 days (a). In a similar experiment mice were sacrificed on the sixth day after transfer of P14 T cells. Percentage of CD45.1+ and CD45.2+ CD8 T cells was determined by FACs analysis. (b) Representative data from 4 mice per condition are shown. The same samples were restimulated for 6 hours with GP33 peptide and labelled with the indicated Ab. Pooled data from 4 mice per group are shown (c).

### BRIEF DESCRIPTION OF THE SEQUENCES

SEQ ID NO: 1 corresponds to a 6384 nucleotides long DNA molecule coding for a human v-maf avian musculoaponeurotic fibrosarcoma oncogene homolog (maf) polypeptide.
SEQ ID NO: 2 corresponds to a 1122 nucleotides long DNA molecule coding for a human c-MAF polypeptide.
SEQ ID NO: 3 corresponds to a 6384 nucleotides long DNA molecule coding for a human c-MAF polypeptide.
SEQ ID NO: 4 corresponds to the protein sequence the human c-MAF.
SEQ ID NO: 5 corresponds to a 4450 nucleotides long DNA molecule coding for a human c-MAF polypeptide.
SEQ ID NO: 6 corresponds to a 812 nucleotides long DNA molecule coding for a human c-MAF polypeptide.
SEQ ID NO: 7 corresponds to a 6384 nucleotides long DNA molecule coding for a human c-MAF polypeptide.
SEQ ID NO: 8 corresponds to a 6360 nucleotides long DNA molecule coding for a mouse MAF polypeptide
SEQ ID NO: 9 to a 1113 nucleotides long DNA molecule coding for a mouse MAF polypeptide
SEQ ID NO: 10 corresponds to a 6360 nucleotides long DNA molecule coding for a mouse MAF polypeptide
SEQ ID NO: 11 corresponds to the protein sequence of the mouse c-MAF.
SEQ ID NO: 12 corresponds to a 4316 nucleotides long DNA molecule coding for a mouse MAF polypeptide
SEQ ID NO: 13 corresponds to a 931 nucleotides long DNA molecule coding for a mouse MAF polypeptide
SEQ ID NO: 14 corresponds to a 6360 nucleotides long DNA molecule coding for a mouse MAF polypeptide
SEQ ID NO: 15 corresponds to a DNA sequence of an artificial construct containing the mouse DNA coding for MAF polypeptide and the DNA coding for the green fluorescent protein (GFP)
SEQ ID NO: 16 corresponds to a DNA sequence of an artificial construct containing the mouse DNA coding for the NR4A2 polypeptide and the DNA coding for the green fluorescent protein (GFP)
SEQ ID NO: 17 corresponds to a nucleotide sequence homologous to a portion of the mouse DNA coding for the beta-2-microglubulin polypeptide
SEQ ID NO: 18 corresponds to a nucleotide sequence homologous to a portion of the mouse DNA coding for the beta-2-microglubulin polypeptide
SEQ ID NO: 19 corresponds to a nucleotide sequence homologous to a portion of the mouse DNA coding for the c-MAF polypeptide
SEQ ID NO: 20 corresponds to a nucleotide sequence homologous to a portion of the mouse DNA coding for the c-MAF polypeptide
SEQ ID NO: 21 corresponds to a nucleotide sequence homologous to a portion of the mouse DNA coding for the interleukin-10 polypeptide
SEQ ID NO: 22 corresponds to a nucleotide sequence homologous to a portion of the mouse DNA coding for the interleukin-10 polypeptide
SEQ ID NO: 23 corresponds to a nucleotide sequence homologous to a portion of the mouse DNA coding for the BCL6 polypeptide
SEQ ID NO: 24 corresponds to a nucleotide sequence homologous to a portion of the mouse DNA coding for the BCL6 polypeptide
SEQ ID NO: 25 corresponds to a nucleotide sequence homologous to a portion of the mouse DNA coding for the NR4A2 polypeptide
SEQ ID NO: 26 corresponds to a nucleotide sequence homologous to a portion of the mouse DNA coding for the NR4A2 polypeptide
SEQ ID NO: 27 corresponds to a nucleotide sequence homologous to a portion of the mouse DNA coding for the GZMB polypeptide
SEQ ID NO: 28 corresponds to a nucleotide sequence homologous to a portion of the mouse DNA coding for the GZMB polypeptide
SEQ ID NO: 29 corresponds to a nucleotide sequence homologous to a portion of the human DNA coding for the GAPDH polypeptide
SEQ ID NO: 30 corresponds to a nucleotide sequence homologous to a portion of the human DNA coding for the GAPDH polypeptide
SEQ ID NO: 31 corresponds to a nucleotide sequence homologous to a portion of the human DNA coding for the c-MAF polypeptide
SEQ ID NO: 32 corresponds to a nucleotide sequence homologous to a portion of the human DNA coding for the c-MAF polypeptide
SEQ ID NO: 33 corresponds to a nucleotide sequence homologous to a portion of the human DNA coding for the NR4A2 polypeptide
SEQ ID NO: 34 corresponds to a nucleotide sequence homologous to a portion of the human DNA coding for the NR4A2 polypeptide

### DETAILED DESCRIPTION OF THE INVENTION

T lymphocytes ("anti-tumor T cells") can eliminate cancer and chronic infections, provided that they are sufficiently "empowered" for robust and prolonged action against disease. Naturally in patients, however, these T cells are frequently "exhausted", i.e. they are dysfunctional. To determine the reasons for exhaustion, Applicants analyzed their transcriptome in patients and in an especially designed mouse model. The T cells presented strong similarities with those described in chronic infection. However, notable differences appeared. Applicants focused their studies on the two transcriptional regulators with the highest fold increase in exhausted compared to naive CD8 T cells, nr4a2 and maf. While nr4a2 was highly expressed in both virus- and tumor-induced exhaustion, maf was highly over-expressed only in tumor-exhausted CD8 T cells. Anti-tumor CD8 T cells transduced to express maf showed dampened anti-tumor efficiency upon adoptive transfer. Maf-expressing CD8 T cells showed unaltered homeostasis but failed to accumulate in tumor-bearing hosts and developed defective anti-tumor secondary responses. Maf expression in CD8 T cells induced part of the transcriptional program associated with tumor-induced exhaustion. This included elevated expression of genes encoding inhibitory receptors (PD-1, GP49a), antiinflammatory proteins (IL-10, A20), negative regulators of chemokine receptors (Rgs-1, Rgs-16), as well as transcription factors diverting CD8 T cells from a cytolytic program (Bcl6, Stat3). Surprisingly, the identified genes and the underlying mechanisms represent novel targets for novel therapies for improving immunotherapy against cancer.

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" is generally used in the sense of include, that is to say permitting the presence of one or more features or components.

As used in the specification and claims, the singular forms "a", "an" and "the" include plural references unless the context clearly dictates otherwise.

As used herein the terms "subject" or "patient" are well-recognized in the art, and, are used interchangeably herein to denotes a vertebrate, preferably a mammal, including dog, cat, rat, mouse, monkey, cow, horse, goat, sheep, pig, camel, and, most preferably, a human. In some embodiments, the subject is a subject in need of treatment or a subject with a disease or disorder. However, in other embodiments, the subject can be a normal subject. The term does not denote a particular age or sex. Thus, adult and newborn subjects, whether male or female, are intended to be covered. Most preferably the T cells according to the method of the invention are thus human cells.

In the context of the invention, the term "treating" or "treatment", as used herein, means reversing, alleviating, inhibiting the progress of, or preventing the disease or condition to which such term applies, or one or more symptoms of such disease or condition.

The term "an effective amount" refers to an amount necessary to obtain a physiological effect. The physiological effect may be achieved by one application dose or by repeated applications. The dosage administered may, of course, vary depending upon known factors, such as the physiological characteristics of the particular composition; the age, health and weight of the subject; the nature and extent of the symptoms; the kind of concurrent treatment; the frequency of treatment; and the effect desired and can be adjusted by a person skilled in the art.

The terms "nucleic acid," "polynucleotide," and "oligonucleotide" are used interchangeably and refer to a desoxyribonucleotide or ribonucleotide polymer, in linear or circular conformation, and in either single- or double-stranded form. For the purposes of the present disclosure, these terms are not to be construed as limiting with respect to the length of a polymer. The terms can encompass known analogues of natural nucleotides, as well as nucleotides that are modified in the base, sugar and/or phosphate moieties (e.g. phosphorothioate backbones). In general, an analogue of a particular nucleotide has the same base-pairing specificity; i.e., an analogue of A will base-pair with T.

The terms "polypeptide," "peptide" and "protein" are used interchangeably to refer to a polymer of amino acid residues. The term also applies to amino acid polymers in which one or more amino acids are chemical analogues or modified derivatives of a corresponding naturally-occurring amino acids.

"Recombination" refers to a process of exchange of genetic information between two polynucleotides. For the purposes of this disclosure, "homologous recombination (HR)" refers to the specialized form of such exchange that takes place, for example, during repair of double-strand breaks in cells via homology-directed repair mechanisms. This process requires nucleotide sequence homology, uses a "donor" molecule to template repair of a "target" molecule (i.e. the one that experienced the double-strand break), and is variously known as "non- crossover gene conversion" or "short tract gene conversion," because it leads to the transfer of genetic information from the donor to the target. Without wishing to be bound by any particular theory, such transfer can involve mismatch correction of heteroduplex DNA that forms between the broken target and the donor, and/or "synthesis-dependent strand annealing," in which the donor is used to resynthesize genetic information that will become part of the target, and/or related processes. Such specialized HR often results in an alteration of the sequence of the target molecule such that part or all of the sequence of the donor polynucleotide is incorporated into the target polynucleotide.

A "reporter gene" or "reporter sequence" refers to any sequence that produces a protein product that is easily measured, preferably although not necessarily in a routine assay. Suitable reporter genes include, but are not limited to, sequences encoding proteins that mediate antibiotic resistance (e.g., ampicillin resistance, neomycin resistance, G418 resistance, puromycin resistance), sequences encoding colored or fluorescent or luminescent proteins (e.g., green fluorescent protein, enhanced green fluorescent protein, red fluorescent protein, luciferase), and proteins which mediate enhanced cell growth and/or gene amplification (e.g., dihydrofolate reductase). Epitope tags include, for example, one or more copies of FLAG, His, myc, Tap, HA or any detectable amino acid sequence. "Expression tags" include sequences that encode reporters that may be operably linked to a desired gene sequence in order to monitor expression of the gene of interest.

"The CRISPR" (Clustered Regularly Interspaced Short Palindromic Repeats)/Cas (CRISPR Associated) nuclease system is a recently engineered nuclease system based on a bacterial system that can be used for genome engineering. It is based on part of the adaptive immune response of many bacteria and archea. When a virus or plasmid invades a bacterium, segments of the invader's DNA are converted into CRISPR RNAs (crRNA) by the 'immune' response. This crRNA then associates, through a region of partial complementarity, with another type of RNA called tracrRNA to guide the Cas9 nuclease to a region homologous to the crRNA in the target DNA called a "protospacer." Cas9 cleaves the DNA to generate blunt ends at the DSB at sites specified by a 20-nucleotide guide sequence contained within the crRNA transcript. Cas9 requires both the crRNA and the tracrRNA for site specific DNA recognition and cleavage. This system has now been engineered such that the crRNA and tracrRNA can be combined into one molecule (the "single guide RNA"), and the crRNA equivalent portion of the single guide RNA can be engineered to guide the Cas9 nuclease to target any desired sequence (see Jineket al (2012) Science 337, p. 816-821, Jineket al, (2013), eLife 2:e00471, and David Segal, (2013) eLife 2:e00563). Thus, the CRISPR/Cas system can be engineered to create a DSB at a desired target in a genome, and repair of the DSB can be influenced by the use of repair inhibitors to cause an increase in error prone repair.

"Adoptive cell transfer" can refer to the transfer of cells, most commonly immune-derived cells, back into the same patient or into a new recipient host with the goal of transferring the immunologic functionality and characteristics into the new host. If possible, use of autologous cells helps the recipient by minimizing GVHD issues. For isolation of immune cells for adpotive transfer, a phlebotomist draws blood into tubes containing anticoagulant and the PBM (buffy coat) cells are isolated, typically by density barrier centrifugation. In T cell-based therapies, these cells are expanded in vitro using cell culture methods relying heavily on the immunomodulatory action of interleukin-2 and returned to the patient in large numbers intravenously in an activated state.

The term "expression" when used in the context of expression of a gene or nucleic acid refers to the conversion of the information, contained in a gene, into a gene product. A gene product can be the direct transcriptional product of a gene (e.g., mRNA, tRNA, rRNA, antisense RNA, ribozyme, structural RNA or any other type of RNA) or a protein produced by translation of a mRNA. Gene products also include messenger RNAs which are modified, by processes such as capping, polyadenylation, methylation, and editing, and proteins (e.g., MafB or c-Maf) modified by, for example, methylation, acetylation, phosphorylation, ubiquitination, SUMOylation, ADP-ribosylation, myristilation, and glycosylation.

An "inhibitor of expression" refers to a natural or synthetic compound that reduces or suppresses the expression of a gene.

An "inhibitor of activity" has its general meaning in the art, and refers to a compound (natural or not) which has the capability of reducing or suppressing the activity of a protein.

The term "c-Maf" denotes the c-Maf proto-onocogene, which is identical in sequence to the v-Maf oncogene of AS42 virus and can transform chicken embryo fibroblasts (Nishizawa et al. PNAS 1989). C-Maf and other Maf family members form homodimers and heterodimers with each other and with Fos and Jun, consistent with the known ability of the AP-1 proteins to pair with each other (Kerppola, T. K. and Curran, T. (1994) Oncogene 9:675-684; Kataoka, K. et al. (1994) Mol. Cell. Biol. 14:700-712). The DNA target sequence to which c-Maf homodimers bind, termed the c-Maf response element (MARE), is a 13 or 14 bp element which contains a core TRE (T-MARE) or CRE (C-MARE) palindrome respectively, but c-Maf may also bind to DNA sequences diverging from these consensus sites including composite AP-1/MARE sites and MARE half sites with 5' AT rich extensions (Yoshida et al., NAR2005).

By "purified" and "isolated" it is meant, when referring to a polypeptide or a nucleotide sequence, that the indicated molecule is present in the substantial absence of other biological macromolecules. When referring to a cell or a population of cells, the term means that said cell or said population of cells is present in the substantial absence of other cells or population of cells. The term "purified" as used herein preferably means at least 75% by weight or number, more preferably at least 85% by weight or number, still preferably at least 95% by weight or number, and most preferably at least 98% by weight or number, of biological macromolecules or cells of the same type are present. An "isolated" nucleic acid molecule, which encodes a particular polypeptide refers to a nucleic acid molecule which is substantially free of other nucleic acid molecules that do not encode the subject polypeptide; however, the molecule may include some additional bases or moieties which do not deleteriously affect the basic characteristics of the composition.

T-cells infiltrating neoplasms express surface molecules typical of chronically virus-stimulated T-cells, often termed "exhausted" T-cells. Applicants compared the transcriptome of "exhausted" CD8 T-cells infiltrating autochthonous melanomas to those of naive and acutely stimulated CD8 T-cells. Despite strong similarities between transcriptional signatures of tumor- and virally-induced exhausted CD8 T-cells, notable differences appeared. Among transcriptional regulators, Nr4a2 and Maf were highly over-expressed in tumor-exhausted T-cells and significantly upregulated in CD8 T-cells from human melanoma metastases. Transduction of murine tumor-specific CD8 T-cells to express Maf partially reproduced the transcriptional program associated with tumor-induced exhaustion. Upon adoptive transfer, the transduced cells showed normal homeostasis but failed to accumulate in tumor-bearing hosts and developed defective anti-tumor secondary responses. Applicants further identified TGFβ and IL-6 as main contributors to Maf expression in CD8 T-cells and showed that Maf-deleted tumor specific CD8 T-cells were much more potent to restrain tumor growth in vivo. Therefore the melanoma microenvironment contributes to skewing of CD8 T-cell differentiation programs, in part by TGFβ/IL-6 mediated induction of Maf.

Applicants developed a mouse model of induced melanoma based on conditional deletion of tumor suppressor genes with concomitant expression of a natural mouse tumor antigen (TiRP mice) (Huijbers et al, 2006). In this model tumor-intrinsic factors control the development of aggressive tumors and their expression of an inflammatory/immunosuppressive program. Applicants showed that intra-tumor T-cells expressed high level of inhibitory receptors such as PD-1 and had poor capacity to produce interferon gamma (IFN-γ) upon restimulation, concluding that they were exhausted. Taking advantage of this model, Applicants established the gene expression signature associated with CD8 T-cell exhaustion during autochthonous melanoma development. Applicants show that tumor- and virus-induced exhaustion share many features, with expression of genes encoding molecules such as inhibitory receptors or particular transcription factors. Among the latter *Nr4a2,* encoding an orphan nuclear receptor, was highly expressed in both virus- and tumor-induced exhaustion, whereas *Maf* was highly over-expressed in tumor-exhausted CD8 T-cells and very weakly during chronic viral infection. Applicants confirmed the overexpression for both genes in Melan-A/MART-1 specific CD8 T-cells isolated from metastasized lymph nodes (LN) from melanoma patients. Over-expression of *Maf* by retroviral transduction of CD8 T-cells dampened their intra-tumor accumulation and anti-tumor activity, while over-expression of *Nr4a2* did not affect CD8 T-cell properties in the same assays. Surprisingly, Applicants showed that *Maf* expression in anti-tumor CD8 T-cells contributes to their polarization toward an exhausted phenotype. In addition, Applicants show that the presence of TGFβ and IL-6 are capable of inducing *Maf* expression in CD8 T-cells in vitro and that *Maf-*depleted tumor specific CD8 T-cells have heightened capacity to eliminate melanoma cells *in vivo.*

It is an object of the present invention to provide a pharmaceutical composition comprising empowered T cells, preferably patient's empowered autologous T cells, for use in a method for treating cancer in a patient, wherein said empowered T cells, preferably said empowered autologous T cells, do not express c-Maf or in which the expression or activity of c-Maf is abolished or inhibited.

According to a preferred embodiment of the invention, the T cells are CD8 T cells.

In the scope of the invention, the cancer includes melanoma, sarcomas and carcinomas derived from lung, kidney, colon, stomach, brain, breast, uterus, ovary, prostate, bladder, pancreas and the head and neck region. In addition, hematological malignancies such as leukemia and lymphomas can also be treated with the method of the invention. Preferably the cancer is a TGFβ and/or IL-6 mediated or induced cancer and most preferably the cancer to be treated is a melanoma.

The pharmaceutical composition of the invention is in combination with a pharmaceutically acceptable carrier. Such compositions comprise a therapeutically effective amount of a patient's empowered autologous T cells, preferably CD8 T cell, produced according to the invention, and a pharmaceutically acceptable carrier or excipient. By a "therapeutically effective amount" of patient's empowered autologous T cells as above described is meant a sufficient amount of said empowered T cell to treat a disease or disorder at a reasonable benefit/risk ratio applicable to any medical treatment. It will be understood, however, that the total daily usage of compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific cells employed; and like factors well known in the medical arts.

Pharmaceutically acceptable carrier or excipient includes but is not limited to saline, buffered saline, dextrose, water, glycerol and combinations thereof. The carrier and composition can be sterile. The formulation should suit the mode of administration. The composition, if desired, can also contain minor amounts of wetting or emulsifying agents, or pH buffering agents. The composition can be a liquid solution, suspension, or emulsion. In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to ease pain at the site of the injection.

Inhibition of the expression or the activity of c-Maf may be achieved by any technique known to the skilled in the art.

According to a preferred embodiment of the present invention, the expression or activity of c-Maf is abolished or inhibited by using inhibitors of c-Maf selected among the group comprising c-Maf specific siRNA oligonucleotide, c-Maf specific antisense oligonucleotide, c-Maf specific shRNA oligonucleotide, c-Maf specific ribozymes oligonucleotide, c-Maf specific Zinc finger nuclease oligonucleotide, c-Maf specific TALENs oligonucleotide, c-Maf specific Crispr-Cas9 oligonucleotide sequences, dominant negative form of MAF and/or chemical inhibitors of MAF.

More preferably, inactivation of maf in T cells is carried out by Crispr-Cas9 technology.

For the sake of clarity, the following techniques are described as follows:
- Crispr-Cas9 technology:
   this system uses a nuclease, CRISPR-associated (Cas9), that complexes with small RNAs as guides (gRNAs) to cleave DNA in a sequence-specific manner upstream of the protospacer adjacent motif (PAM) in any genomic location.
- TALENs:
   TALENs are fusions of transcription activator-like (TAL) proteins and a FokI nuclease. TAL proteins are composed of 33-34 amino acid repeating motifs with two variable positions that have a strong recognition for specific nucleotides. By assembling arrays of these TALs and
   fusing them to a FokI nuclease, specific cutting of the genome can be achieved. When two TALENs bind and meet, the FokI domains induce a double-strand break which can inactivate a gene, or can be used to insert DNA of interest.
- Zinc finger nuclease (ZFN) :
   A ZFN is a hybrid molecule that couples the DNA binding domain of a zinc-finger protein with the DNA-cleaving nuclease domain of the restriction endonuclease FokI. The DNA binding motif specified by the zinc fingers directs the ZFN to a specific (targeted) locus in the genome. A pair of ZFNs is required to cleave double-stranded DNA. Each ZFN recognizes a different 12-18 base pair target sequence, and these target sequences must be separated by 4-7 base pairs to allow formation of the catalytically active FokI dimer. These positional constraints drive a very high degree of specificity.
- Ribozymes can also function as inhibitors of expression of c-Maf for use in the present invention. Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of c-Maf mRNA sequences are thereby useful within the scope of the present invention. Specific ribozyme cleavage sites within any potential RNA target are initially identified by scanning the target molecule for ribozyme cleavage sites, which typically include the following sequences, GUA, GuU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable. The suitability of candidate targets can also be evaluated by testing their accessibility to hybridization with complementary oligonucleotides, using, e.g., ribonuclease protection assays.
- siRNA:
   siRNA is formed in the cell from shRNA or from long synthetic dsRNA by the Dicer enzyme, and is later separated into two short strands, one of which binds to the target mRNA and cleaves it, preventing the unwanted protein from being made. In the siRNA approach, specific siRNAs are synthesized in the laboratory to silence specific proteins in target cells which are implicated in disease.
- shRNA:
   shRNA is short hairpin RNA, double stranded RNA (dsRNA) which is created in the cell from a DNA construct encoding a sequence of single stranded RNA and its complement, separated by a stuffer fragment, allowing the RNA molecule to fold back on itself, creating a dsRNA molecule with a hairpin loop. The target cell can be directed to produce shRNA by specific DNA sequences introduced to the cell via a small gene cassette which travels to the nucleus. Here the introduced DNA either becomes part of the cell's own DNA or persists in the nucleus, and instructs the cell to produce the specific shRNA, which is then processed by Dicer to siRNA and continues along the RNAi pathway via RISC to silence the gene.
- Dominant negative form of MAF:
   a dominant negative form of c-maf, termed Ac-maf, has been created by replacing the basic DNA binding region of c-maf with an acidic region while retaining the leucine zipper. Retroviruses or lentiviruses can be used to transduce Ac-maf into cells.
- Chemical inhibitors of MAF (see Xialiang Mao et al, Blood ISSN 0006-4971, 2007 110: 4047-4054, "A chemical biology screen identifies glucocorticoids that regulate c-maf expression by increasing its proteosomal degradation through up-regulation of ubiquitin"): Some chemical inhibitors can increase the degradation of maf in cells.

In particular, the chemical inhibitors of MAF are identified as c-maf-dependent cyclin D2 inhibitors consisting of corticosteroid derivatives selected among the group comprising budesonide, flumethasone, betamethasone, diflorasone, flurandrenoline, flunisolide, methylprednisolone, prednisolone, triamcinolone, fluocinonide, isoflupredone acetate, dexamethasone acetate, corticosterone, fluorometholone, hydrocortisone base, halcinonide, prednicarbate, fludrocortisone acetate, clocortolone pivalate, alclometasone dipropionate, beclomethasone dipropionate, hydrocortisone, deoxycorticosterone, medrysone.

Preferably, the most potent chemical inhibitors of MAF are glucocorticoids such as dexamethasone acetate and dexamethasone.

Methods for delivering siRNAs, ribozymes and/or antisense oligonucleotides into T cells are well known in the art and include but are not limited to transfection, electroporation, microinj ection, lipofection, calcium phosphate mediated transfection or infection with a viral vector containing the gene sequences, cell fusion, chromosome-mediated gene transfer, microcell-mediated gene transfer, spheroplast fusion, etc. Numerous techniques are known in the art for the introduction of foreign genes into cells and may be used in accordance with the present invention, provided that the necessary developmental and physiological functions of the recipient cells are not disrupted. The technique may provide for the stable transfer of the gene to the cell, so that the gene is expressible by the cell, heritable and expressible by its cell progeny. Usually, the method of transfer includes the transfer of a selectable marker to the cells. The cells are then placed under selection to isolate those cells that have taken up and are expressing the transferred gene. Those cells are then delivered to a subject. A variation of the technique may provide for transient transfer of oligonucleotides or oligonucleotide coding genes to T cells to enable temporary expansion of T cells ex vivo or in vivo without permanent genetic modification.

Another object of the present invention is to provide an *ex vivo* method for empowering T cells that can be used in adoptive cell transfer, wherein said method comprises:
collecting patients' autologous T cells,
*ex vivo* inhibiting the expression or the activity of c-Maf in said patients' autologous T cells and expanding said T cells to be suitable for a subsequent adoptive cell transfer or administration to said patient.

Preferably the T cells are CD8 T cells.

In particular, the expression or activity of c-Maf is abolished or inhibited by using inhibitors of c-Maf selected among the group comprising c-Maf specific siRNA oligonucleotide, c-Maf specific antisense oligonucleotide, c-Maf specific shRNA oligonucleotide, c-Maf specific ribozymes oligonucleotide, c-Maf specific Zinc finger nuclease oligonucleotide, c-Maf specific TALENs oligonucleotide, c-Maf specific Crispr-Cas9 oligonucleotide sequences, dominant negative form of MAF and/or chemical inhibitors of MAF as described above.

The present invention also provides for an empowered T cells, obtainable by the ex vivo method for adoptive cell transfer of the invention.

Said empowered T cell, preferably CD8 T cells, do not express c-Maf or in which the expression or activity of c-Maf is abolished or inhibited.

It is another object of the invention to provide for inhibitors of c-Maf expression in patient's T cells for use in a method for treating cancer in said patient, wherein said patient's T cells are ex vivo empowered by said inhibitors of c-Maf expression so as not to express c-Maf and whereas said empowered patient's T cells are suitable or to be used in adoptive cell transfer in said patient (cell therapy). More preferably said T cells are patient's autologous T cells and even more preferably patient's autologous CD8 T cells.

Selecting and expanding the empowered specific T cells "ex vivo" can circumvent some of the regulatory and tolerising effects of the tumour environment that are seen in active vaccination. In addition, T cells are manipulated "ex vivo" before adoptive transfer to enhance their numbers, specificity, and function.

Preferably said inhibitors of c-Maf are selected among the group comprising c-Maf specific siRNA oligonucleotide, c-Maf specific antisense oligonucleotide, c-Maf specific shRNA oligonucleotide, c-Maf specific ribozymes oligonucleotide, c-Maf specific Zinc finger nuclease oligonucleotide, c-Maf specific TALENs oligonucleotide, c-Maf specific Crispr-Cas9 oligonucleotide sequences, dominant negative form of MAF and/or chemical inhibitors of MAF.

More preferably the chemical inhibitors of MAF are identified as c-maf-dependent cyclin D2 inhibitors consisting of corticosteroid derivatives selected among the group comprising budesonide, flumethasone, betamethasone, diflorasone, flurandrenoline, flunisolide, methylprednisolone, prednisolone, triamcinolone, fluocinonide, isoflupredone acetate, dexamethasone acetate, corticosterone, fluorometholone, hydrocortisone base, halcinonide, prednicarbate, fludrocortisone acetate, clocortolone pivalate, alclometasone dipropionate, beclomethasone dipropionate, hydrocortisone, deoxycorticosterone, medrysone.

The genes that Applicants newly identified can be manipulated in T cells from patients, making these T cells more powerful with regard to their anti-tumor activity. After "empowering" of the patient's ("autologous") T cells, they are transferred back to the patient in the so-called adoptive cell therapy, an approach that can be used for treating patients with metastatic cancer.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein.

Various references are cited throughout this specification, each of which is incorporated herein by reference in its entirety.

The foregoing description will be more fully understood with reference to the following Examples. Such Examples, are, however, exemplary of methods of practising the present invention and are not intended to limit the scope of the invention.

### Examples

### Materials and methods:

**Mice and cell lines.** TiRP-10B; Ink4a/Arf flox/flox mice, "TiRP mice," were kept on a B10.D2 background and treated with 40H-tamoxifen as previously described. Mice heterozygous for the H-2L^{d}/P1A₃₅₋₄₃-specific TCR-transgene (TCRP1A) (Shanker et al, 2007) were kept on the Rag-1^{-/-}B10.D2 background. Luciferase expressing TCRP1A mice (TCRP1A-luc) were generated as previously described. TCR transgenic P14 mice (TCR specific for the LCMV gp33 peptide and H-2D^{b}) with selective deletion of *maf* in mature T cells were obtained by crossing maf floxed/floxed mice with P14 maT-Cre mice (Giordano et al, 2014). Rag-1^{-/-}B10.D2, C57BL/6 CD45.2 and C57BL/6 CD45.1 mice were also used. All these mice were bred in the CIML animal facility. Animal experiments respected French and European directives.

**Cell preparation.** CD8 T-cells were prepared from LN or spleen of TCRP1A Rag-1^{-/-} B10.D2 mice according to standard procedures. When prepared from immunocompetent B10.D2 mice, CD8 T-cells were enriched using Mouse CD8 negative selection kit (Dynal, Invitrogen) according to the manufacturer's instructions. For analysis of TILs, tumors were cut into small pieces and incubated with collagenase I for 45 minutes. Tumors were dispersed into a single cell suspension and passed over Ficoll-Paque™ solution (Amersham Biosciences AB).

**CD8 T-cells activation and retroviral infections.** Murine *maf* cDNA was amplified using the Stratagene HerculaseII Kit (with the following primers: acgcgtcgacaagcttcagaactggcaatgaacaattcc / cgcggatcctctagatcacatgaaaaattcgggagaggaagg) and cloned into pRc/CMV-3X-Flag-GFP vector. FL cDNA vector encoding *nr4a2* (IRCKp5014E0514Q) was obtained from ImaGenes GmbH. This cDNA was cloned into the retroviral vector pMX-IRES-GFP after PCR amplification using PFUultra HF (Agilent) using the following primers tcggaaatataccaaagc / ttttccttttgcggccgcttctttgacgtgcttgg. Retroviral particles were produced as previously described (Verdeil et al, 2006). TCRP1A T-cells were activated for 72h with 10⁻⁷M of P1A₃₅₋₄₃ (LPYLGWLVF) peptide. 20h after initial stimulation, CD8 T-cells were retrovirally-transduced as previously described (Verdeil et al, 2006). Cultures were then continued for another 48h. P14 T CD8 T cells were stimulated with GP33 peptide (KAVYNFATC - 10⁻⁶M) for 48h before adoptive transfer experiments and up to 72h *for in vitro* analysis.

**Flow cytometry.** Antibodies were from BD Biosciences, except anti-GzmB mAb (Invitrogen) and anti-Maf mAb (eBiosciences). Cells (10⁶) were analyzed on a LSR2 UV or a LSR2 561 cytometer (BD Biosciences). Data were analyzed using FlowJo (Treestar Inc., CA) or Diva (BD Biosciences) software. For intracellular cytokine staining, CD8 T-cells were stimulated ex-vivo for 4h with ionomycin (400ng/ml) and PMA (40ng/ml) in the presence of Golgi stop (BD) and permeabilized using the Cytofix/Cytoperm kit (BD Biosciences). For intracellular labelling with anti-Maf mAb, FoxP3 permeabilization buffer (eBiosciences) was used according to the manufacturer's protocol. Cells were labelled using Cell Trace Violet (life technologies) according to manufacturer's protocol.

**Bioluminescence.** The infiltration of the luciferase-expressing TCRP1A T-cells was monitored by bioluminescence imaging. After i.p. luciferin (3 mg/mouse) injection, the mice were anesthetized in a chamber flushed with a mixture of isofluorane (4% in air) and placed in the NightOwl LB981 (Berthold Technologies) under continuous anaesthetization as previously described (Shanker et al, 2007).

**Transcriptome analyses.** Naive CD8 T cells from lymph nodes of TCR transgenic TCRP1A B10.D2 mice were sorted by flow cytometry (CD8+, CD3+, CD44-, Topro3-). For AdP1A samples, naive CD8 T cells from lymph nodes of TCR transgenic TCRP1A B10.D2 (Ly5.2) were transferred into Ly5.1 B10.D2 mice. One day after adoptive transfer, mice were infected intra-dermally with the adenovirus AdP1At (coding for P1A, the antigen recognized by TCRP1A CD8 T cells). Four days after infection draining lymph nodes were recovered and activated TCRP1A CD8 T cells were sorted by flow cytometry (CD8+, Ly5.2+, CD44+, Topro3-). For TILs samples, 2 to 3 melanoma tumors from TIRP mice were pooled. After centrifugation on Ficoll-Paque™ solution (Amersham Biosciences AB), CD8 T cells were sorted by flow cytometry (CD3+, CD8+, Topro3-). Triplicates were used for Naive and AdP1A (activated) T cells. For TILs, T cells coming from 4 independent sortings (with 2 to 3 pooled tumors each time) were used. SuperAmp RNA amplification was performed according to Miltenyi Biotec's undisclosed procedure and amplified cDNA labeled with Cy3 were hybridized on Agilent Whole Mouse Genome Oligo Microarrays 8 x 60K. Microarray data have been submitted to NCBI GEO database and are accessible with the following link: (http://www.ncbi.nlm.nih.gov/geo/query/acc.cgi?acc=GSE42824). All the data were normalized by quantiles. The statistical analysis for detecting differentially expressed genes was made using LIMMA (Linear Model for Microarray Data) based on an empirical Bayes model. Probes whose maximal gene expression value did not exceed 5 (log₂ scale) were removed. The remaining list contained 24 562 probes used for GSEA analysis. Genes with logFC >1 (or <-1) and p-value <0.05 were selected as regulated. All these selected genes were constituted as gene set for the subsequent GSEA analysis. To find GO terms that were overrepresented in exhausted T cells we used GOrilla (http://cbl-gorilla.cs.technion.ac.il/). Gene Ontology terms were summarized and visualized by using REVIGO (http://revigo.irb.hr/).

**GSEA Analysis.** Data from GSE30431 were extracted from GEO. "CD8 D30 chronic" and "naïve" samples were selected. Data were normalized by RMA. 21 938 probes were taken into account. Fold changes between CD8 D30 chronic" and "naïve" samples were calculated and probes were ranked according to these ratios. All these selected genes were constituted as gene sets for the subsequent GSEA analysis.

**Quantitative RT-PCR.** Total RNA was isolated using RNeasy kit (Quiagen). RNA from sorted cell populations was reverse-transcribed using High Capacity cDNA Reverse Transcription Kit (Applied Biosystems). Quantitative real-time PCR was done by nonspecific detection (Power SYBR®Green, Applied Biosystems) of cDNA. For each gene, primer pairs generated a single product and were amplified in a linear relationship with the housekeeping beta-2-microglobulin gene.

Naive CD8 T cells and Melan-A/MART-1 specific T cells were sorted and processed as in (Baitsch et al, 2011).

**Statistical analysis.** Sample means were compared using Student's t test (2 tailed comparison) or multiple T Test using Sidak-Bonferoni method when multiple time points were compared. P-value is represented as following: P < 0.05 (*) P<0.01 (**), P<0.001 (***), P<0.0001 (****). Error bars display standard deviation.

### Example 1:

### Molecular characterization of T infiltrated lymphocytes (TILs) from autochthonous melanomas

To explore the molecular bases of tumor-induced exhaustion, Applicants performed a transcriptomic analysis of CD8 T-cells sorted by flow cytometry from autochthonous melanomas of TiRP mice. Applicants compared their profile with the transcriptomic profile of sorted naive CD44 low CD8 T-cells and of activated T Cell Receptor (TCR) transgenic CD8 T-cells (TCRP1A) obtained 4 days after infection with an adenovirus expressing P1A, the antigen recognized by the TCRP1A. Using a cut-off of a two-fold change in comparison to naive CD8 T-cells with a statistically significant p-value (p<0.05), 2192 known genes were found upregulated in activated CD8 T-cells versus 629 in CD8 TILs (hereafter also called exhausted T-cells) and 1630 were down regulated in activated CD8 T-cells versus 656 in exhausted CD8 T-cells. More than half of the genes upregulated in CD8 TILs (330) were also upregulated at similar levels in activated CD8 T-cells while about a third of the genes down regulated in CD8 TILs were also down regulated in activated CD8 T-cells.

Exhaustion has been associated with high level expression of inhibitory receptors that are normally transiently upregulated on effector T-cells but are rapidly down regulated when the pathogen is cleared. In the present setting Applicants confirmed that exhausted CD8 T-cells inside the melanomas expressed high level of PD-1 (*Pdcd1*)*,* LAG-3 *(Lag3),* Tim-3 (*Havcr2*), GP49b (*Lilrb4*), NKG2A (*Klrc1*) and CTLA-4 (*Ctla4*) transcripts compared to naive CD8 T-cells. These transcripts were expressed at a higher (CTLA-4, GP49b), a similar (PD-1, NKG2A) or a lower (TIM-3, LAG-3) level in exhausted compared to activated CD8 T-cells.

Applicants then looked at genes specifically up- or down-regulated in exhausted CD8 T-cells compared to both naive and activated CD8 T-cells (Figure 1a). Applicants studied the enrichment of Gene Ontology terms associated with the genes from these two lists. The most represented group of genes with an upregulated expression consisted in "negative regulation of biological/cellular processes", followed by "homeostatic process and regulation of gene expression". Among the genes falling into the category of negative regulation, Applicants found genes encoding phosphatases like *Ptpre* and *Ptprj*, whose products regulate MAPK phosphorylation, a gene (*Tnfaip3*) encoding an inhibitor of the NF-κB pathway (Giordano et al, 2014), as well as genes encoding transcription factors (*Nr4a2, Nr4a3*, *Maf, Nfat5*)*.* CD101, a surface molecule which inhibits TCR/CD3 phosphorylation in humans and expressed on highly suppressive regulatory T cells (Tregs) in mice, was also specifically expressed on exhausted CD8 T-cells. *Lilrb4,* encoding for the inhibitory receptor GP49 (ILT3), was also overexpressed on CD8+ TILs.

In summary the transcriptional signature of exhausted CD8 T-cells showed a high level of inhibitory receptors, with the expression of numerous genes involved in negative regulation of biological processes and in regulation of transcription as well as a strong downregulation of genes involved in metabolic processes.

### Example 2:

### Nr4a2 and Maf are overexpressed in both mouse and human CD8 TILs

One aim of this study was to determine potential transcriptional regulators favoring exhaustion establishment in TILs. Applicants chose to focus studies on the two transcriptional regulators with the highest fold increase in exhausted CD8 T-cells compared to naive CD8 T-cells, *Nr4a2* and *Maf.* Applicants validated the microarray data by measuring the level of expression of those two transcripts in CD8 and CD4 TILs obtained from TiRP melanomas and evaluated by flow cytometry the level of MAF in CD4 and CD8 T-cells in tumor free or TiRP mice. In the spleens of tumor free animals, Applicants found very few MAF+ CD8 T-cells (1.9%) and some MAF+ CD4 T-cells (around 10%; Figure 1a). In TiRP mice that had developed a tumor, the percentage of MAF+ CD4 T-cells was slightly increased in the spleen (17%) and was further raised in CD4 TILs (35.6%). MAF+ CD8 T-cells were only found among TILs, ranging from 6 to 42% of the CD8 TILs (average of 22%).

Applicants then determined whether the findings in a melanoma mouse model was applicable to humans. Therefore, Applicants used RNA from sorted naive T cells (CD8+, CD45RA+, CCR7+, CD27+, CD28+) from healthy donors and from Melan-A/MART-1 specific CD8 T-cells isolated from the blood or from metastasized LNs of melanoma patients as previously described (Baitsch et al, 2011). Applicants measured the relative level of *MAF* and *NR4A2* in those samples. For both genes, Applicants found a significant increase in tumor infiltrated CD8 T-cells compered to naive T-cells, with an average of a 25 and a 4 fold increase compared to the median value obtained in naive T-cells, for *MAF* and *NR4A2* respectively (Figure 1b). Blood derived Melan-A/MART-1 specific CD8 T-cells showed an intermediate level of expression for *MAF* (7 fold increase compared to Naive T-cells) and a level of *NR4A2* expression that was similar to that of Naive T-cells.

These results validate the transcriptomic data for CD8 T-cells and show that there is convergence of gene expression in both CD4 and CD8 TILs in mouse and between human and mouse CD8 TILs, at least for the expression of *Nr4a2* and *Maf.*

### Example 3:

### Overexpression of Maf dampens CD8 T-cell anti-tumor response

Applicants further focused our study on the effects of *Nr4a2* and *Maf* expression in CD8 T-cells. To test whether forced expression of the transcription factors in CD8 T-cells would dampen their anti-tumor efficiency, Applicants needed to use a tumor model in which untransduced CD8 T-cells were able to induce tumor regression. This was not the case in the TiRP mouse model. Applicants therefore took advantage of a model using the P511 mastocytoma naturally expressing the P1A-encoded antigen. In this model as few as 10⁴ naive tumor specific CD8 T-cells were capable of inducing a strong but transient regression of the tumor burden. Applicants hypothesized that if their gene of interest played a role in the establishment/maintenance of a dysfunctional state in tumor infiltrating CD8 T-cells, their expression would dampen the anti-tumor response. Rag-/-B10.D2 mice were injected subcutaneously with P511 cells. One week later, pre-activated T-cells expressing a transgenic TCR recognizing the P1A-encoded Ag (TCRP1A T-cells) were adoptively transferred into the animals. Tumors reach their maximum size 7 days after the T-cell transfer and generally shrink and disappear 15 days after transfer (Figure 2d). Applicants used TCRP1A T-cells that are also transgenic for the luciferase encoding gene (TCRP1A-luc) to follow the accumulation of T-cells inside the tumor. TCRP1A-luc cells were activated *in vitro* and transduced with retroviruses encoding *Maf* and *green fluorescent protein (gfp)* (maf), *Nr4a2* and *gfp* (nr4a2) or *gfp* alone (mock). After 3 days, T-cells were FACS sorted according to their expression of GFP. GFP^{high} TCRP1A-luc T-cells were transferred into tumor bearing mice. Applicants monitored by bioluminescence the infiltration of the tumors by the TCRP1A-luc T-cells (Figure 2a, b). Photon emission measured in the tumors showed no difference when TCRP1A T-cells were transduced with nr4a2 compared to mock (Figure 2a). However, Applicants detected a much lower signal for maf-transduced as compared to mock-transduced TCRP1A-luc T-cells (Figure 2b). This shows a strong decrease in intra-tumor accumulation of *Maf-*overexpressing CD8 T-cells. This was confirmed by determining the proportion of GFP+ maf-transduced TCRP1A T-cells at day 7, which was much lower in LN, spleen and tumor compared to their mock-transduced counterparts (Figure 2c). The overall tumor regression induced by adoptively transferred T-cells was also less pronounced for maf-transduced as compared to mock-transduced TCRP1A T-cells, whereas nr4a2-transduced TCRP1A T-cells were as effective as their control counterparts (Figure 2d). Tumor shrinking was delayed for 5 days when maf-transduced as compared to mock-transduced T-cells were used in the adoptive transfer and the tumor never totally disappeared from the animal (Figure 2d).

Altogether the results presented here illustrate that maf-expressing CD8 T-cells were unaltered in their homeostasis, at least up to 7 days post-transfer in non-tumor bearing hosts, but developed a defective tumor-induced secondary response as compared to control T-cells and failed to accumulate in tumor-bearing hosts.

### Example 4:

### Overexpression of maf polarizes CD8 T-cell differentiation toward an exhausted phenotype.

To determine how maf expression influences the T-cell anti-tumor response, Applicants analyzed by flow cytometry TCRP1A T-cells *ex-vivo* (from LN, spleen or TILs), 7 days after transfer into P511 bearing mice. Maf-transduced GFP+ CD8 T-cells expressed a higher level of PD-1 and a lower level of GzmB compared to mock-transduced T-cells (Figure 3a). To test if the functional capacities of these cells were further affected, Applicants stimulated the cells from the tumor draining LN with PMA/Ionomycine *ex-vivo.* Clearly fewer maf-transduced than mock-transduced CD8 T-cells produced IFNγ (9.7 compared to 23.7%) and IL-2 (0.83 compared to 4.1 %) (Figure 3b).

### Example 5:

### TGFβ and IL-6 induce Maf expression in CD8 T-cells

In CD4 T-cells *Maf* expression has been associated to Th2, Th17, Tr1 and follicular helper T-cells (Tfh) differentiation in response to TCR signaling plus various stimuli including the presence of cytokines like IL-4, IL-6, TGFβ or IL-27 among others. However, scant information exists on the control of *Maf* expression in CD8 T-cells. In the TiRP model, the tumor itself produces high level of TGFβ, IL-6 and IL-10(Soudja et al, 2010; Wehbe et al, 2012). Applicants tested if one of these cytokines could induce *Maf* expression during CD8 T-cell *in vitro* activation. We never detected MAF by flow cytometry or at the RNA level after stimulation of TCR transgenic T cells without additional cytokines (Figure 4a). At day 3 post-activation, Applicants observed a strong increase in MAF+ population in the presence of TGFb, IL-6 or a combination of both (from 5 to 20% of the CD8 T-cells depending on the cytokine - Figure 4a). Altogether these data show that TGFβ and IL-6 in the tumor microenvironment contribute to the induction of *Maf* expression in exhausted CD8 T-cells.

### Example 6:

### Maf deletion in CD8 T-cells dampens the effects of TGFβ and IL-6 on their activation program

To further characterize the importance of MAF expression during the response of CD8 T-cells to antigenic stimulation, Applicants generated TCR transgenic P14 mice (TCR specific for the LCMV gp33 peptide and H-2D^{b}) with selective deletion of *maf* in mature T cells by crossing maf floxed/floxed mice with P14 maT-Cre mice (Giordano et al, 2014) (P14 maT-Cre-maf^{fl/fl} mice). Applicants evaluated the capacity of *maf* deficient CD8 T-cells to respond to stimulation with GP33 peptide *in vitro* in the presence of TGFb or/and IL-6. When stimulated with peptide alone, Applicants did not detect noticeable differences between Wild Type (WT) and maf deficient CD8 T-cells 3 days post-stimulation with regard to cell number or AnnexinV labeling (Fig.4 b, c). Maf deficient T-cells were however producing more IL-2 and *ifng* than their WT counterparts. Presence of 10 ng/ml TGFβ in the culture medium dampened CD8 T-cell stimulation, affecting cell number but not their survival (Figure 5b, c). All of the effector functions were dampened in this condition: production of IL-2, expression of *ifng* and *gzmb* (Figure 4d). On the opposite Applicants detected a significant increase in *il10* and *bcl6* expression (Figure 4d). In the maf deficient CD8 T-cells the proliferation was partially rescued as well as IL-2 production. The increased expression of *bcl6* and *il10* in WT P14 T-cells in the presence of either TGFb or IL-6 was not detectable in the maf-deleted CD8 T-cells (Figure 4d). The presence of 10 ng/ml IL-6 did not affect cell proliferation in either WT or maf-deficient CD8 T-cells. The number of recovered cells was however increased by twofold for the maf-deficient CD8 T-cells (Figure 4b). This correlates with a decreased labeling with AnnexinV, suggesting a better survival of the maf-deficient cells in the presence of IL-6 (Figure 4c). IL-6 induced the expression of *bcl-6,* but not *il-10,* abrogated the expression of *ifng* and partially decreased the expression of *gzmb* in WT cells (Figure 4d). In maf-deficient CD8 T-cells IL-6 failed to induce expression of *bcl6* and *gzmb* expression was partially restored. When IL-6 and TGFβ were combined, their effects on WT T cells were more dramatic with decreased proliferation, increased apoptosis, high level of *bcl6, il10* and decreased level of *ifng* and IL-2 production (Figure 4b, c, d). Maf deletion strongly restored IL-2 production, cell survival and proliferation (Figure 4c), decreased *bcl6* and *il10* expression but failed to restore significant levels of *ifng* or *gzmb* (Figure 4d). Altogether these data show that the effects of TGFβ and IL-6 on CD8 T-cell activation are partially regulated by their induction of MAF in CD8 T-cells.

### Example 7:

### Maf deletion in tumor specific CD8 T-cells increased their capacity to eliminate melanoma cells in vivo

Applicants next evaluated the capacity of adoptively transferred maf-deficient tumor specific CD8 T-cells to induce melanoma regression. Because of the C57BL/6 background of P14 maT-Cre maf^{fl/fl} mice, Applicants had to use a different melanoma model. C57BL/6 (CD45.1) mice were injected s.c with B16F10 melanoma cells expressing the LCMV GP33 epitope (B16-GP33). One week later, WT or maf-deficient *in vitro* pre-activated P14 T-cells (on a C57BL/6 (CD45.2) background) were adoptively transferred into the mice. The presence of WT P 14 T-cells only moderately affected tumor growth in this model. However maf-deficient P14 T-cells induced a strong regression of the tumor burden (Figure 5a). One week post-transfer Applicants analyzed the endogenous CD45.1 CD8 T-cells and the transferred CD45.2 P14 T-cells from either P14 maT-Cre+ maf^{fl/fl} mice (maf-KO P14 CD8 T-cells) or from P14 maT-Cre- maf^{fl/fl} littermates (WT P14 CD8 T-cells). There was a slight increase of maf-deficient P14 T-cells among total CD8 T-cells compared to WT P14 T-cells in the tumor-draining LN. In TILs Applicants detected a much higher percentage of maf-deficient P14 T-cells, increasing the ratio of P14 CD8 T-cell/endogenous CD8 T-cells from 1 to 1 for WT P14 to 4 to 1 for maf-deficient P14 T-cells (Figure 5b). When tested for their capacity to produce cytokines upon gp33 peptide restimulation, maf-KO P14 T-cells from the tumor-draining LN showed a higher capacity to produce IFNγ, IL-2 or TNFα compared to WT P14 T-cells (Figure 5b). In P14 T-cells from TILs, Applicants detected similar levels of Granzyme B, IL-2 or TNFα but higher capacity to produce IFNγ in maf-deficient cells (Figure 5b). At the cell surface from TILs, similar levels of CD44 but lower expression of the inhibitory receptors PD-1 and LAG-3 were detected for maf-deficient compared to WT P14 T-cells.

In summary the higher capacity of maf-deficient tumor specific CD8 T-cells to accumulate inside the tumor and their higher potential for IFNγ production with lower surface expression of PD-1 correlate with increased capacity to eliminate tumor burden in vivo.

### References:

Baitsch L, Baumgaertner P, Devevre E, Raghav SK, Legat A, Barba L, Wieckowski S, Bouzourene H, Deplancke B, Romero P, Rufer N, Speiser DE (2011) Exhaustion of tumor-specific CD8(+) T cells in metastases from melanoma patients. J Clin Invest 121: 2350-2360.
Giordano M, Roncagalli R, Bourdely P, Chasson L, Buferne M, Yamasaki S, Beyaert R, van Loo G, Auphan-Anezin N, Schmitt-Verhulst AM, Verdeil G (2014) The tumor necrosis factor alpha-induced protein 3 (TNFAIP3, A20) imposes a brake on antitumor activity of CD8 T cells. Proc Natl Acad Sci U S A 111: 11115-11120.
Huijbers IJ, Krimpenfort P, Chomez P, van der Valk MA, Song JY, Inderberg-Suso EM, Schmitt-Verhulst AM, Berns A, Van den Eynde BJ (2006) An inducible mouse model of melanoma expressing a defined tumor antigen. Cancer Res 66: 3278-3286.
Shanker A, Verdeil G, Buferne M, Inderberg-Suso EM, Puthier D, Joly F, Nguyen C, Leserman L, Auphan-Anezin N, Schmitt-Verhulst AM (2007) CD8 T cell help for innate antitumor immunity. J Immunol 179: 6651-6662.
Soudja SM, Wehbe M, Mas A, Chasson L, de Tenbossche CP, Huijbers I, Van den Eynde B, Schmitt-Verhulst AM (2010) Tumor-initiated inflammation overrides protective adaptive immunity in an induced melanoma model in mice. Cancer Res 70: 3515-3525.
Verdeil G, Puthier D, Nguyen C, Schmitt-Verhulst AM, Auphan-Anezin N (2006) STAT5-mediated signals sustain a TCR-initiated gene expression program toward differentiation of CD8 T cell effectors. J Immunol 176: 4834-4842.
Wehbe M, Soudja SM, Mas A, Chasson L, Guinamard R, de Tenbossche CP, Verdeil G, Van den Eynde B, Schmitt-Verhulst AM (2012) Epithelial-Mesenchymal-Transition-Like and TGFbeta Pathways Associated with Autochthonous Inflammatory Melanoma Development in Mice. PLoS One 7: e49419.

## Claims

1. A pharmaceutical composition comprising empowered T cells for use in a method for treating cancer in a patient, **characterized in that** said empowered T cells do not express c-Maf or in which the expression or activity of c-Maf is abolished or inhibited.

2. The pharmaceutical composition of claim 1, wherein the empowered T cells are patient's empowered autologous T cells consisting of CD8 T cells.

3. The pharmaceutical composition of claims 1 or 2, wherein the cancer is a TGFβ and/or IL-6 induced cancer.

4. The pharmaceutical composition of claim 3, wherein the cancer is a melanoma.

5. The pharmaceutical composition of any one of the preceding claims, wherein the expression or activity of c-Maf is abolished or inhibited by using inhibitors of c-Maf selected among the group comprising c-Maf specific siRNA oligonucleotide, c-Maf specific antisense oligonucleotide, c-Maf specific shRNA oligonucleotide, c-Maf specific ribozymes oligonucleotide, c-Maf specific Zinc finger nuclease oligonucleotide, c-Maf specific TALENs oligonucleotide, c-Maf specific Crispr-Cas9 oligonucleotide sequences, dominant negative form of MAF and/or chemical inhibitors of MAF.

6. The pharmaceutical composition of claim 5, wherein the chemical inhibitors of MAF are identified as c-maf-dependent cyclin D2 inhibitors consisting of corticosteroid derivatives selected among the group comprising budesonide, flumethasone, betamethasone, diflorasone, flurandrenoline, flunisolide, methylprednisolone, prednisolone, triamcinolone, fluocinonide, isoflupredone acetate, dexamethasone acetate, corticosterone, fluorometholone, hydrocortisone base, halcinonide, prednicarbate, fludrocortisone acetate, clocortolone pivalate, alclometasone dipropionate, beclomethasone dipropionate, hydrocortisone, deoxycorticosterone, medrysone.

7. The pharmaceutical composition of claim 5, wherein the chemical inhibitors of MAF is dexamethasone acetate or dexamethasone.

8. The pharmaceutical composition of any of claims 1-7, in combination with a pharmaceutically acceptable carrier.

9. An *ex vivo* method for empowering T cells in adoptive cell transfer, **characterized in that** the method comprises:
collecting patients' autologous T cells,
*ex vivo* inhibiting the expression or the activity of c-Maf in said patients' autologous T cells and
expanding said T cells to be suitable for a subsequent adoptive cell transfer to said patient.

10. The *ex vivo* method for empowering T cells in adoptive cell transfer of claim 9, wherein the patients' autologous T cells are CD8 T cells.

11. The *ex vivo* method for empowering T cells in adoptive cell transfer of any of claims 9-10, wherein the expression or activity of c-Maf is abolished or inhibited by using inhibitors of c-Maf selected among the group comprising c-Maf specific siRNA oligonucleotide, c-Maf specific antisense oligonucleotide, c-Maf specific shRNA oligonucleotide, c-Maf specific ribozymes oligonucleotide, c-Maf specific Zinc finger nuclease oligonucleotide, c-Maf specific TALENs oligonucleotide, c-Maf specific Crispr-Cas9 oligonucleotide sequences, dominant negative form of MAF and/or chemical inhibitors of MAF.

12. An empowered T cells, obtainable by the *ex vivo* method for adoptive cell transfer of any of claims 9-11 and wherein said empowered T cell does not express c-Maf or in which the expression or activity of c-Maf is abolished or inhibited.

13. Inhibitors of c-Maf expression in patient's T cells for use in a method for treating cancer in said patient, **characterized in that** said patient's T cells are ex vivo empowered by said inhibitors of c-Maf expression so as not to express c-Maf and wherein said empowered patient's T cells are suitable for adoptive cell transfer in said patient.

14. The inhibitors of c-Maf expression of claim 13, wherein said inhibitors of c-Maf are selected among the group comprising c-Maf specific siRNA oligonucleotide, c-Maf specific antisense oligonucleotide, c-Maf specific shRNA oligonucleotide, c-Maf specific ribozymes oligonucleotide, c-Maf specific Zinc finger nuclease oligonucleotide, c-Maf specific TALENs oligonucleotide, c-Maf specific Crispr-Cas9 oligonucleotide sequences, dominant negative form of MAF and/or chemical inhibitors of MAF.

15. The inhibitors of c-Maf expression of claim 14, **characterized in that** the chemical inhibitors of MAF are identified as c-maf-dependent cyclin D2 inhibitors consisting of corticosteroid derivatives selected among the group comprising budesonide, flumethasone, betamethasone, diflorasone, flurandrenoline, flunisolide, methylprednisolone, prednisolone, triamcinolone, fluocinonide, isoflupredone acetate, dexamethasone acetate, corticosterone, fluorometholone, hydrocortisone base, halcinonide, prednicarbate, fludrocortisone acetate, clocortolone pivalate, alclometasone dipropionate, beclomethasone dipropionate, hydrocortisone, deoxycorticosterone, medrysone.


SEQUENCE LISTING

<110>  Université de Lausanne UNIL

<120>  Molecular profiling of CD8 T-cells in autochthonous melanoma
       identifies Maf as driver of exhaustion

<130>  50040/EP

<140>  EP 15166627.8
<141>  2015-05-06

<160>  40

<170>  PatentIn version 3.5

<210>  1
<211>  6384
<212>  DNA
<213>  Homo sapiens

<400>  1
atcttttttc atcttctagc tgtagctcgg gctgcttgtc ggcttggcct ccccctcccc      60

cctttgctct ctgcctcgtc tttccccagg acttcgctat tttgcttttt taaaaaaagg     120

caagaaagaa ctaaactccc ccctccctct cctccagtcg ggctgcacct ctgccttgca     180

ctttgcacag aggtagagag cgcgcgaggg agagagagga aagaaaaaaa ataataaaga     240

gagccaagca gaagaggagg cgagaagcat gaagtgttaa ctcccccgtg ccaaggcccg      300

cgccgcccgg acagacgccc gccgcgcctc cagccccgag cggacgccgc gcgcgccctg     360

cctgcagccc gggccggcga ggcgagccct tccttatgca aagcgcgcag cggagcggcg     420

agcgggggac gccgcgcacc gggccgggct cctccagctt cgccgccgca gccaccaccg     480

ccgccaccgc agctcgcgga ggatcttccc gagcctgaag ccgccggctc ggcgcgcaag     540

gaggcgagcg agcaaggagg ggccggggcg agcgagggag cacattggcg tgagcagggg     600

ggagggaggg cgggcgcggg gggcgcgggc agggcggggg ggtgtgtgtg tgagcgcgct     660

cggaggtttc gggccagcca ccgccgcgca agctagaagc gccccagccc ggcaagctgg     720

ctcacccgct ggccacccag cacagcccgc tggcccctct cctgcagccc atctggcgga     780

gcggcggcgg cggcggcggc ggcggcagga gaatggcatc agaactggca atgagcaact     840

ccgacctgcc caccagtccc ctggccatgg aatatgttaa tgacttcgat ctgatgaagt     900

ttgaagtgaa aaaggaaccg gtggagaccg accgcatcat cagccagtgc ggccgtctca     960

tcgccggggg ctcgctgtcc tccaccccca tgagcacgcc gtgcagctcg gtgccccctt    1020

cccccagctt ctcggcgccc agcccgggct cgggcagcga gcagaaggcg cacctggaag    1080

actactactg gatgaccggc tacccgcagc agctgaaccc cgaggcgctg ggcttcagcc    1140

ccgaggacgc ggtcgaggcg ctcatcagca acagccacca gctccagggc ggcttcgatg    1200

gctacgcgcg cggggcgcag cagctggccg cggcggccgg ggccggtgcc ggcgcctcct 1260

tgggcggcag cggcgaggag atgggccccg ccgccgccgt ggtgtccgcc gtgatcgccg 1320

cggccgccgc gcagagcggc gcgggcccgc actaccacca ccaccaccac cacgccgccg 1380

gccaccacca ccacccgacg gccggcgcgc ccggcgccgc gggcagcgcg gccgcctcgg 1440

ccggtggcgc tgggggcgcg ggcggcggtg gcccggccag cgctgggggc ggcggcggcg 1500

gcggcggcgg cggaggcggc gggggcgcgg cgggggcggg gggcgccctg cacccgcacc 1560

acgccgccgg cggcctgcac ttcgacgacc gcttctccga cgagcagctg gtgaccatgt 1620

ctgtgcgcga gctgaaccgg cagctgcgcg gggtcagcaa ggaggaggtg atccggctga 1680

agcagaagag gcggaccctg aaaaaccgcg gctatgccca gtcctgccgc ttcaagaggg 1740

tgcagcagag acacgtcctg gagtcggaga agaaccagct gctgcagcaa gtcgaccacc 1800

tcaagcagga gatctccagg ctggtgcgcg agagggacgc gtacaaggag aaatacgaga 1860

agttggtgag cagcggcttc cgagaaaacg gctcgagcag cgacaacccg tcctctcccg 1920

agtttttcat gtgagtctga cacgcgattc cagctagcca ccctgataag tgctccgcgg 1980

gggtccggct cgggtgtggg cttgctagtt ctagagccat gctcgccacc acctcaccac 2040

ccccaccccc accgagtttg gcccccttgg ccccctacac acacacaaac ccgcacgcac 2100

acaccacaca cacacacaca cacacacaca caccccacac cctgctcgag tttgtggtgg 2160

tggtggctgt tttaaactgg ggagggaatg ggtgtctggc tcatggattg ccaatctgaa 2220

attctccata acttgctagc ttgttttttt ttttttttta cacccccccg ccccaccccc 2280

ggacttgcac aatgttcaat gatctcagca gagttcttca tgtgaaacgt tgatcacctt 2340

tgaagcctgc atcattcaca tattttttct tcttcttccc cttcagttca tgaactggtg 2400

ttcattttct gtgtgtgtgt gtgttttatt ttgtttggat tttttttttt aattttactt 2460

ttagagcttg ctgtgttgcc cacctttttt ccaacctcca ccctcactcc ttctcaaccc 2520

atctcttccg agatgaaaga aaaaaaaaag caaagttttt ttttcttctc ctgagttctt 2580

catgtgagat tgagcttgca aaggaaaaaa aaatgtgaaa tgttatagac ttgcagcgtg 2640

ccgagttcca tcgggttttt tttttagcat tgttatgcta aaatagagaa aaaaatcctc 2700

atgaaccttc cacaatcaag cctgcatcaa ccttctgggt gtgacttgtg agttttggcc 2760

ttgtgatgcc aaatctgaga gtttagtctg ccattaaaaa aactcattct catctcatgc 2820

attattatgc ttgctacttt gtcttagcaa caatgaacta taactgtttc aaagacttta 2880

tggaaaagag acattatatt aataaaaaaa aaaagcctgc atgctggaca tgtatggtat 2940

aattattttt tccttttttt ttccttttgg cttggaaatg gacgttcgaa gacttatagc 3000

atggcattca tacttttgtt ttattgcctc atgacttttt tgagtttaga acaaaacagt 3060

gcaaccgtag agccttcttc ccatgaaatt ttgcatctgc tccaaaactg ctttgagtta 3120

ctcagaactt caacctccca atgcactgaa ggcattcctt gtcaaagata ccagaatggg 3180

ttacacattt aacctggcaa acattgaaga actcttaatg ttttcttttt aataagaatg 3240

acgccccact ttggggacta aaattgtgct attgccgaga agcagtctaa aatttatttt 3300

ttaaaaagag aaactgcccc attatttttg gtttgtttta tttttatttt atattttttg 3360

gcttttggtc attgtcaaat gtggaatgct ctgggtttct agtatataat ttaattctag 3420

tttttataat ctgttagccc agttaaaatg tatgctacag ataaaggaat gttatagata 3480

aatttgaaag agttaggtct gtttagctgt agatttttta aacgattgat gcactaaatt 3540

gtttactatt gtgatgttaa ggggggtaga gtttgcaagg ggactgttta aaaaaagtag 3600

cttatacagc atgtgcttgc aacttaaata taagttgggt atgtgtagtc tttgctatac 3660

cactgactgt attgaaaacc aaagtattaa gaggggaaac gcccctgttt atatctgtag 3720

gggtatttta cattcaaaaa tgtatgtttt tttttctttt caaaattaaa gtatttggga 3780

ctgaattgca ctaagatata acctgcaagc atataataca aaaaaaaatt gcaaaactgt 3840

ttagaacgct aataaaattt atgcagttat aaaaatggca ttactgcaca gttttaagat 3900

gatgcagatt tttttacagt tgtattgtgg tgcagaactg gattttctgt aacttaaaaa 3960

aaaatccaca gttttaaagg caataatcag taaatgttat tttcagggac tgacatcctg 4020

tctttaaaaa gaaatgaaaa gtaaatctta ccacaataaa tataaaaaaa tcttgtcagt 4080

tacttttctt ttacatattt tgctgtgcaa aattgtttta tatcttgagt tactaactaa 4140

ccacgcgtgt tgttcctatg tgcttttctt tcattttcaa ttctggttat atcaagaaaa 4200

gaataatcta caataataaa cggcattttt ttttgattct gtactcagtt tcttagtgta 4260

cagtttaact gggcccaaca acctcgttaa aagtgtaaaa tgcatccttt tctccagtgg 4320

aaggattcct ggaggaatag ggagacagta attcagggtg aaattatagg ctgttttttg 4380

aagtgaggag gctggcccca tatactgatt agcaatattt aatatagatg taaattatga 4440

cctcattttt ttctccccaa agttttcagt tttcaaatga gttgagccat aattgccctt 4500

ggtaggaaaa acaaaacaaa acagtggaac taggcttcct gagcatggcc ctacacttct 4560

gatcaggagc aaagccatcc atagacagag gagccggaca aatatggcgc atcagaggtg 4620

gcttgcgcac atatgcattg aacggtaaag agaaacagcg cttgcctttt cactaaagtt 4680

gactattttt ccttcttctc ttacacaccg agatttcttg ttagcaaggc cctgacaaga 4740

tttaacataa acatgacaaa tcatagttgt ttgttttgtt ttgcttttct ctttaacact 4800

gaagatcatt tgtcttaaat aggaaaaaga aaatccactc cttacttcca tatttccaag 4860

tacatatctg gtttaaacta tgttatcaaa tcatatttca ccgtgaatat tcagtggaga 4920

acttctctac ctggatgagc tagtaatgat ttcagatcat gctatcccca gaaataaaag 4980

caaaaaataa tacctgtgtg gaatataggc tgtgctttga tttactggta tttaccccaa 5040

aataggctgt gtatgggggc tgacttaaag atcccttgga aagactcaaa actaccttca 5100

ctagtaggac tcctaagcgc tgacctattt ttaaatgaca caaattcatg aaactaatgt 5160

tacaaattca tgcagtttgc actcttagtc atcttcccct agcacaccaa tagaatgtta 5220

gacaaagcca gcactgtttt gaaaatacag ccaaacacga tgacttttgt tttgttttct 5280

gccgttctta aaagaaaaaa agataatatt gcaactctga ctgaaagact tatttttaag 5340

aaaacaggtt gtgtttggtg ctgctaagtt ctggccagtt tatcatctgg ccttcctgcc 5400

tattttttac aaaacacgaa gacagtgtgt aacctcgaca ttttgacctt cctttatgtg 5460

ctagtttaga caggctcctg aatccacact taattttgct taacaaaagt cttaatagta 5520

aacctcccct catgagcttg aagtcaagtg ttcttgactt cagatatttc tttccttttt 5580

tttttttttt cctcatcaca actaagagat acacaaactc tgaagaagca gaaatggaga 5640

gaatgctttt aacaaaaaag catctgatga aagattttag gcaaacattc tcaaaataag 5700

agtgatattc tggatgtagt tattgcagtt atctcatgac aaatgaggcc tggattggaa 5760

ggaaaatata gttgtgtaga attaagcatt ttgataggaa tctacaaggt agttgaatat 5820

aataagcagg tttgggcccc caaactttag aaaatcaaat gcaaaggtgc tggcaaaaat 5880

gaggtttgag tggctggctg taagagaagg ttaactccta gtaaaaggca tttttagaaa 5940

taacaattac tgaaaacttt gaagtatagt gggagtagca aacaaataca tgtttttttt 6000

ttcttacaaa gaactcctaa atcctgagta agtgccattc attacaataa gtctctaaat 6060

ttaaaaaaaa aaaaatcata tgaggaaatc tagctttccc ctttacgctg cgtttgatct 6120

ttgtctaaat agtgttaaaa ttcctttcat tccaattaca gaactgagcc cactcgcaag 6180

ttggagccat cagtgggata cgccacattt tggaagcccc agcatcgtgt acttaccagt 6240

gtgttcacaa aatgaaattt gtgtgagagc tgtacattaa aaaaaatcat cattattatt 6300

attatttgca gtcatggaga accacctacc cctgacttct gtttagtctc ctttttaaat 6360

aaaaattact gtgttagaga agaa 6384

<210> 2
<211> 1122
<212> DNA
<213> Homo sapiens

<400> 2
atggcatcag aactggcaat gagcaactcc gacctgccca ccagtcccct ggccatggaa 60

tatgttaatg acttcgatct gatgaagttt gaagtgaaaa aggaaccggt ggagaccgac 120

cgcatcatca gccagtgcgg ccgtctcatc gccgggggct cgctgtcctc cacccccatg 180

agcacgccgt gcagctcggt gcccccttcc cccagcttct cggcgcccag cccgggctcg 240

ggcagcgagc agaaggcgca cctggaagac tactactgga tgaccggcta cccgcagcag 300

ctgaaccccg aggcgctggg cttcagcccc gaggacgcgg tcgaggcgct catcagcaac 360

agccaccagc tccagggcgg cttcgatggc tacgcgcgcg gggcgcagca gctggccgcg 420

gcggccgggg ccggtgccgg cgcctccttg ggcggcagcg gcgaggagat gggccccgcc 480

gccgccgtgg tgtccgccgt gatcgccgcg gccgccgcgc agagcggcgc gggcccgcac 540

taccaccacc accaccacca cgccgccggc caccaccacc acccgacggc cggcgcgccc 600

ggcgccgcgg gcagcgcggc cgcctcggcc ggtggcgctg gggcgcgggc cggcggtggc 660

ccggccagcg ctgggggcgg cggcggcggc ggcggcggcg gaggcggcgg gggcgcggcg 720

ggggcggggg gcgccctgca cccgcaccac gccgccggcg gcctgcactt cgacgaccgc 780

ttctccgacg agcagctggt gaccatgtct gtgcgcgagc tgaaccggca gctgcgcggg 840

gtcagcaagg aggaggtgat ccggctgaag cagaagaggc ggaccctgaa aaaccgcggc 900

tatgcccagt cctgccgctt caagagggtg cagcagagac acgtcctgga gtcggagaag 960

aaccagctgc tgcagcaagt cgaccacctc aagcaggaga tctccaggct ggtgcgcgag 1020

agggacgcgt acaaggagaa atacgagaag ttggtgagca gcggcttccg agaaaacggc 1080

tcgagcagcg acaacccgtc ctctcccgag tttttcatgt ga 1122

<210> 3
<211> 6384
<212> DNA
<213> Homo sapiens

<400> 3
   atcttttttc atcttctagc tgtagctcgg gctgcttgtc ggcttggcct ccccctcccc 60
   cctttgctct ctgcctcgtc tttccccagg acttcgctat tttgcttttt taaaaaaagg 120
   caagaaagaa ctaaactccc ccctccctct cctccagtcg ggctgcacct ctgccttgca 180
   ctttgcacag aggtagagag cgcgcgaggg agagagagga aagaaaaaaa ataataaaga 240
   gagccaagca gaagaggagg cgagaagcat gaagtgttaa ctcccccgtg ccaaggcccg 300
   cgccgcccgg acagacgccc gccgcgcctc cagccccgag cggacgccgc gcgcgccctg 360
   cctgcagccc gggccggcga ggcgagccct tccttatgca aagcgcgcag cggagcggcg 420
   agcgggggac gccgcgcacc gggccgggct cctccagctt cgccgccgca gccaccaccg 480
   ccgccaccgc agctcgcgga ggatcttccc gagcctgaag ccgccggctc ggcgcgcaag 540
   gaggcgagcg agcaaggagg ggccggggcg agcgagggag cacattggcg tgagcagggg 600
   ggagggaggg cgggcgcggg gggcgcgggc agggcggggg ggtgtgtgtg tgagcgcgct 660
   cggaggtttc gggccagcca ccgccgcgca agctagaagc gccccagccc ggcaagctgg 720
   ctcacccgct ggccacccag cacagcccgc tggcccctct cctgcagccc atctggcgga 780
   gcggcggcgg cggcggcggc ggcggcagga gaatggcatc agaactggca atgagcaact 840
   ccgacctgcc caccagtccc ctggccatgg aatatgttaa tgacttcgat ctgatgaagt 900
   ttgaagtgaa aaaggaaccg gtggagaccg accgcatcat cagccagtgc ggccgtctca 960
   tcgccggggg ctcgctgtcc tccaccccca tgagcacgcc gtgcagctcg gtgccccctt 1020
   cccccagctt ctcggcgccc agcccgggct cgggcagcga gcagaaggcg cacctggaag 1080
   actactactg gatgaccggc tacccgcagc agctgaaccc cgaggcgctg ggcttcagcc 1140
   ccgaggacgc ggtcgaggcg ctcatcagca acagccacca gctccagggc ggcttcgatg 1200
   gctacgcgcg cggggcgcag cagctggccg cggcggccgg ggccggtgcc ggcgcctcct 1260
   tgggcggcag cggcgaggag atgggccccg ccgccgccgt ggtgtccgcc gtgatcgccg 1320
   cggccgccgc gcagagcggc gcgggcccgc actaccacca ccaccaccac cacgccgccg 1380
   gccaccacca ccacccgacg gccggcgcgc ccggcgccgc gggcagcgcg gccgcctcgg 1440
   ccggtggcgc tgggggcgcg ggcggcggtg gcccggccag cgctgggggc ggcggcggcg 1500
   gcggcggcgg cggaggcggc gggggcgcgg cgggggcggg gggcgccctg cacccgcacc 1560
   acgccgccgg cggcctgcac ttcgacgacc gcttctccga cgagcagctg gtgaccatgt 1620
   ctgtgcgcga gctgaaccgg cagctgcgcg gggtcagcaa ggaggaggtg atccggctga 1680
   agcagaagag gcggaccctg aaaaaccgcg gctatgccca gtcctgccgc ttcaagaggg 1740
   tgcagcagag acacgtcctg gagtcggaga agaaccagct gctgcagcaa gtcgaccacc 1800
   tcaagcagga gatctccagg ctggtgcgcg agagggacgc gtacaaggag aaatacgaga 1860
   agttggtgag cagcggcttc cgagaaaacg gctcgagcag cgacaacccg tcctctcccg 1920
   agtttttcat gtgagtctga cacgcgattc cagctagcca ccctgataag tgctccgcgg 1980
   gggtccggct cgggtgtggg cttgctagtt ctagagccat gctcgccacc acctcaccac 2040
   ccccaccccc accgagtttg gcccccttgg ccccctacac acacacaaac ccgcacgcac 2100
   acaccacaca cacacacaca cacacacaca caccccacac cctgctcgag tttgtggtgg 2160
   tggtggctgt tttaaactgg ggagggaatg ggtgtctggc tcatggattg ccaatctgaa 2220
   attctccata acttgctagc ttgttttttt ttttttttta cacccccccg ccccaccccc 2280
   ggacttgcac aatgttcaat gatctcagca gagttcttca tgtgaaacgt tgatcacctt 2340
   tgaagcctgc atcattcaca tattttttct tcttcttccc cttcagttca tgaactggtg 2400
   ttcattttct gtgtgtgtgt gtgttttatt ttgtttggat tttttttttt aattttactt 2460
   ttagagcttg ctgtgttgcc cacctttttt ccaacctcca ccctcactcc ttctcaaccc 2520
   atctcttccg agatgaaaga aaaaaaaaag caaagttttt ttttcttctc ctgagttctt 2580
   catgtgagat tgagcttgca aaggaaaaaa aaatgtgaaa tgttatagac ttgcagcgtg 2640
   ccgagttcca tcgggttttt tttttagcat tgttatgcta aaatagagaa aaaaatcctc 2700
   atgaaccttc cacaatcaag cctgcatcaa ccttctgggt gtgacttgtg agttttggcc 2760
   ttgtgatgcc aaatctgaga gtttagtctg ccattaaaaa aactcattct catctcatgc 2820
   attattatgc ttgctacttt gtcttagcaa caatgaacta taactgtttc aaagacttta 2880
   tggaaaagag acattatatt aataaaaaaa aaaagcctgc atgctggaca tgtatggtat 2940
   aattattttt tccttttttt ttccttttgg cttggaaatg gacgttcgaa gacttatagc 3000
   atggcattca tacttttgtt ttattgcctc atgacttttt tgagtttaga acaaaacagt 3060
   gcaaccgtag agccttcttc ccatgaaatt ttgcatctgc tccaaaactg ctttgagtta 3120
   ctcagaactt caacctccca atgcactgaa ggcattcctt gtcaaagata ccagaatggg 3180
   ttacacattt aacctggcaa acattgaaga actcttaatg ttttcttttt aataagaatg 3240
   acgccccact ttggggacta aaattgtgct attgccgaga agcagtctaa aatttatttt 3300
   ttaaaaagag aaactgcccc attatttttg gtttgtttta tttttatttt atattttttg 3360
   gcttttggtc attgtcaaat gtggaatgct ctgggtttct agtatataat ttaattctag 3420
   tttttataat ctgttagccc agttaaaatg tatgctacag ataaaggaat gttatagata 3480
   aatttgaaag agttaggtct gtttagctgt agatttttta aacgattgat gcactaaatt 3540
   gtttactatt gtgatgttaa ggggggtaga gtttgcaagg ggactgttta aaaaaagtag 3600
   cttatacagc atgtgcttgc aacttaaata taagttgggt atgtgtagtc tttgctatac 3660
   cactgactgt attgaaaacc aaagtattaa gaggggaaac gcccctgttt atatctgtag 3720
   gggtatttta cattcaaaaa tgtatgtttt tttttctttt caaaattaaa gtatttggga 3780
   ctgaattgca ctaagatata acctgcaagc atataataca aaaaaaaatt gcaaaactgt 3840
   ttagaacgct aataaaattt atgcagttat aaaaatggca ttactgcaca gttttaagat 3900
   gatgcagatt tttttacagt tgtattgtgg tgcagaactg gattttctgt aacttaaaaa 3960
   aaaatccaca gttttaaagg caataatcag taaatgttat tttcagggac tgacatcctg 4020
   tctttaaaaa gaaatgaaaa gtaaatctta ccacaataaa tataaaaaaa tcttgtcagt 4080
   tacttttctt ttacatattt tgctgtgcaa aattgtttta tatcttgagt tactaactaa 4140
   ccacgcgtgt tgttcctatg tgcttttctt tcattttcaa ttctggttat atcaagaaaa 4200
   gaataatcta caataataaa cggcattttt ttttgattct gtactcagtt tcttagtgta 4260
   cagtttaact gggcccaaca acctcgttaa aagtgtaaaa tgcatccttt tctccagtgg 4320
   aaggattcct ggaggaatag ggagacagta attcagggtg aaattatagg ctgttttttg 4380
   aagtgaggag gctggcccca tatactgatt agcaatattt aatatagatg taaattatga 4440
   cctcattttt ttctccccaa agttttcagt tttcaaatga gttgagccat aattgccctt 4500
   ggtaggaaaa acaaaacaaa acagtggaac taggcttcct gagcatggcc ctacacttct 4560
   gatcaggagc aaagccatcc atagacagag gagccggaca aatatggcgc atcagaggtg 4620
   gcttgcgcac atatgcattg aacggtaaag agaaacagcg cttgcctttt cactaaagtt 4680
   gactattttt ccttcttctc ttacacaccg agattttctt gttagcaagg cctgacaaga 4740
   tttaacataa acatgacaaa tcatagttgt ttgttttgtt ttgcttttct ctttaacact 4800
   gaagatcatt tgtcttaaat aggaaaaaga aaatccactc cttacttcca tatttccaag 4860
   tacatatctg gtttaaacta tgttatcaaa tcatatttca ccgtgaatat tcagtggaga 4920
   acttctctac ctggatgagc tagtaatgat ttcagatcat gctatcccca gaaataaaag 4980
   caaaaaataa tacctgtgtg gaatataggc tgtgctttga tttactggta tttaccccaa 5040
   aataggctgt gtatgggggc tgacttaaag atcccttgga aagactcaaa actaccttca 5100
   ctagtaggac tcctaagcgc tgacctattt ttaaatgaca caaattcatg aaactaatgt 5160
   tacaaattca tgcagtttgc actcttagtc atcttcccct agcacaccaa tagaatgtta 5220
   gacaaagcca gcactgtttt gaaaatacag ccaaacacga tgacttttgt tttgttttct 5280
   gccgttctta aaagaaaaaa agataatatt gcaactctga ctgaaagact tatttttaag 5340
   aaaacaggtt gtgtttggtg ctgctaagtt ctggccagtt tatcatctgg ccttcctgcc 5400
   tattttttac aaaacacgaa gacagtgtgt aacctcgaca ttttgacctt cctttatgtg 5460
   ctagtttaga caggctcctg aatccacact taattttgct taacaaaagt cttaatagta 5520
   aacctcccct catgagcttg aagtcaagtg ttcttgactt cagatatttc tttccttttt 5580
   tttttttttt cctcatcaca actaagagat acacaaactc tgaagaagca gaaatggaga 5640
   gaatgctttt aacaaaaaag catctgatga aagattttag gcaaacattc tcaaaataag 5700
   agtgatattc tggatgtagt tattgcagtt atctcatgac aaatgaggcc tggattggaa 5760
   ggaaaatata gttgtgtaga attaagcatt ttgataggaa tctacaaggt agttgaatat 5820
   aataagcagg tttgggcccc caaactttag aaaatcaaat gcaaaggtgc tggcaaaaat 5880
   gaggtttgag tggctggctg taagagaagg ttaactccta gtaaaaggca tttttagaaa 5940
   taacaattac tgaaaacttt gaagtatagt gggagtagca aacaaataca tgtttttttt 6000
   ttcttacaaa gaactcctaa atcctgagta agtgccattc attacaataa gtctctaaat 6060
   ttaaaaaaaa aaaaatcata tgaggaaatc tagctttccc ctttacgctg cgtttgatct 6120
   ttgtctaaat agtgttaaaa ttcctttcat tccaattaca gaactgagcc cactcgcaag 6180
   ttggagccat cagtgggata cgccacattt tggaagcccc agcatcgtgt acttaccagt 6240
   gtgttcacaa aatgaaattt gtgtgagagc tgtacattaa aaaaaatcat cattattatt 6300
   attatttgca gtcatggaga accacctacc cctgacttct gtttagtctc ctttttaaat 6360
   aaaaattact gtgttagaga agaa 6384

<210>  4
<211>  373
<212>  PRT
<213>  Homo sapiens

<400>  4

Met Ala Ser Glu Leu Ala Met Ser Asn Ser Asp Leu Pro Thr Ser Pro
1               5                   10                  15

Leu Ala Met Glu Tyr Val Asn Asp Phe Asp Leu Met Lys Phe Glu Val
            20                  25                  30

Lys Lys Glu Pro Val Glu Thr Asp Arg Ile Ile Ser Gln Cys Gly Arg
        35                  40                  45

Leu Ile Ala Gly Gly Ser Leu Ser Ser Thr Pro Met Ser Thr Pro Cys
    50                  55                  60

Ser Ser Val Pro Pro Ser Pro Ser Phe Ser Ala Pro Ser Pro Gly Ser
65                  70                  75                  80

Gly Ser Glu Gln Lys Ala His Leu Glu Asp Tyr Tyr Trp Met Thr Gly
                85                  90                  95

Tyr Pro Gln Gln Leu Asn Pro Glu Ala Leu Gly Phe Ser Pro Glu Asp
            100                 105                 110

Ala Val Glu Ala Leu Ile Ser Asn Ser His Gln Leu Gln Gly Gly Phe
        115                 120                 125

Asp Gly Tyr Ala Arg Gly Ala Gln Gln Leu Ala Ala Ala Ala Gly Ala
    130                 135                 140

Gly Ala Gly Ala Ser Leu Gly Gly Ser Gly Glu Glu Met Gly Pro Ala
145                 150                 155                 160

Ala Ala Val Val Ser Ala Val Ile Ala Ala Ala Ala Ala Gln Ser Gly
                165                 170                 175

Ala Gly Pro His Tyr His His His His His His Ala Ala Gly His His
            180                 185                 190

His His Pro Thr Ala Gly Ala Pro Gly Ala Ala Gly Ser Ala Ala Ala
        195                 200                 205

Ser Ala Gly Gly Ala Gly Gly Ala Gly Gly Gly Gly Pro Ala Ser Ala
    210                 215                 220

Gly Gly Gly Gly Gly Gly Gly Gly Gly Gly Gly Gly Gly Gly Ala Ala
225                 230                 235                 240

Gly Ala Gly Gly Ala Leu His Pro His His Ala Ala Gly Gly Leu His
                245                 250                 255

Phe Asp Asp Arg Phe Ser Asp Glu Gln Leu Val Thr Met Ser Val Arg
            260                 265                 270

Glu Leu Asn Arg Gln Leu Arg Gly Val Ser Lys Glu Glu Val Ile Arg
        275                 280                 285

Leu Lys Gln Lys Arg Arg Thr Leu Lys Asn Arg Gly Tyr Ala Gln Ser
    290                 295                 300

Cys Arg Phe Lys Arg Val Gln Gln Arg His Val Leu Glu Ser Glu Lys
305                 310                 315                 320

Asn Gln Leu Leu Gln Gln Val Asp His Leu Lys Gln Glu Ile Ser Arg
                325                 330                 335

Leu Val Arg Glu Arg Asp Ala Tyr Lys Glu Lys Tyr Glu Lys Leu Val
            340                 345                 350

Ser Ser Gly Phe Arg Glu Asn Gly Ser Ser Ser Asp Asn Pro Ser Ser
        355                 360                 365

Pro Glu Phe Phe Met
    370

<210>  5
<211>  4450
<212>  DNA
<213>  Homo sapiens

<400>  5
gtctgacacg cgattccagc tagccaccct gataagtgct ccgcgggggt ccggctcggg      60

tgtgggcttg ctagttctag agccatgctc gccaccacct caccaccccc acccccaccg     120

agtttggccc ccttggcccc ctacacacac acaaacccgc acgcacacac cacacacaca     180

cacacacaca cacacacacc ccacaccctg ctcgagtttg tggtggtggt ggctgtttta     240

aactggggag ggaatgggtg tctggctcat ggattgccaa tctgaaattc tccataactt     300

gctagcttgt tttttttttt tttttacacc cccccgcccc acccccggac ttgcacaatg     360

ttcaatgatc tcagcagagt tcttcatgtg aaacgttgat cacctttgaa gcctgcatca     420

ttcacatatt ttttcttctt cttccccttc agttcatgaa ctggtgttca ttttctgtgt     480

gtgtgtgtgt tttattttgt ttggattttt ttttttaatt ttacttttag agcttgctgt     540

gttgcccacc ttttttccaa cctccaccct cactccttct caacccatct cttccgagat 600

gaaagaaaaa aaaaagcaaa gttttttttt cttctcctga gttcttcatg tgagattgag 660

cttgcaaagg aaaaaaaaat gtgaaatgtt atagacttgc agcgtgccga gttccatcgg 720

gttttttttt tagcattgtt atgctaaaat agagaaaaaa atcctcatga accttccaca 780

atcaagcctg catcaacctt ctgggtgtga cttgtgagtt ttggccttgt gatgccaaat 840

ctgagagttt agtctgccat taaaaaaact cattctcatc tcatgcatta ttatgcttgc 900

tactttgtct tagcaacaat gaactataac tgtttcaaag actttatgga aaagagacat 960

tatattaata aaaaaaaaaa gcctgcatgc tggacatgta tggtataatt attttttcct 1020

ttttttttcc ttttggcttg gaaatggacg ttcgaagact tatagcatgg cattcatact 1080

tttgttttat tgcctcatga cttttttgag tttagaacaa aacagtgcaa ccgtagagcc 1140

ttcttcccat gaaattttgc atctgctcca aaactgcttt gagttactca gaacttcaac 1200

ctcccaatgc actgaaggca ttccttgtca aagataccag aatgggttac acatttaacc 1260

tggcaaacat tgaagaactc ttaatgtttt ctttttaata agaatgacgc cccactttgg 1320

ggactaaaat tgtgctattg ccgagaagca gtctaaaatt tattttttaa aaagagaaac 1380

tgccccatta tttttggttt gttttatttt tattttatat tttttggctt ttggtcattg 1440

tcaaatgtgg aatgctctgg gtttctagta tataatttaa ttctagtttt tataatctgt 1500

tagcccagtt aaaatgtatg ctacagataa aggaatgtta tagataaatt tgaaagagtt 1560

aggtctgttt agctgtagat tttttaaacg attgatgcac taaattgttt actattgtga 1620

tgttaagggg ggtagagttt gcaaggggac tgtttaaaaa aagtagctta tacagcatgt 1680

gcttgcaact taaatataag ttgggtatgt gtagtctttg ctataccact gactgtattg 1740

aaaaccaaag tattaagagg ggaaacgccc ctgtttatat ctgtaggggt attttacatt 1800

caaaaatgta tgtttttttt tcttttcaaa attaaagtat ttgggactga attgcactaa 1860

gatataacct gcaagcatat aatacaaaaa aaaattgcaa aactgtttag aacgctaata 1920

aaatttatgc agttataaaa atggcattac tgcacagttt taagatgatg cagatttttt 1980

tacagttgta ttgtggtgca gaactggatt ttctgtaact taaaaaaaaa tccacagttt 2040

taaaggcaat aatcagtaaa tgttattttc agggactgac atcctgtctt taaaaagaaa 2100

tgaaaagtaa atcttaccac aataaatata aaaaaatctt gtcagttact tttcttttac 2160

atattttgct gtgcaaaatt gttttatatc ttgagttact aactaaccac gcgtgttgtt 2220

cctatgtgct tttctttcat tttcaattct ggttatatca agaaaagaat aatctacaat 2280

aataaacggc attttttttt gattctgtac tcagtttctt agtgtacagt ttaactgggc 2340

ccaacaacct cgttaaaagt gtaaaatgca tccttttctc cagtggaagg attcctggag 2400

gaatagggag acagtaattc agggtgaaat tataggctgt tttttgaagt gaggaggctg 2460

gccccatata ctgattagca atatttaata tagatgtaaa ttatgacctc atttttttct 2520

ccccaaagtt ttcagttttc aaatgagttg agccataatt gcccttggta ggaaaaacaa 2580

aacaaaacag tggaactagg cttcctgagc atggccctac acttctgatc aggagcaaag 2640

ccatccatag acagaggagc cggacaaata tggcgcatca gaggtggctt gcgcacatat 2700

gcattgaacg gtaaagagaa acagcgcttg ccttttcact aaagttgact atttttcctt 2760

cttctcttac acaccgagat tttcttgtta gcaaggcctg acaagattta acataaacat 2820

gacaaatcat agttgtttgt tttgttttgc ttttctcttt aacactgaag atcatttgtc 2880

ttaaatagga aaaagaaaat ccactcctta cttccatatt tccaagtaca tatctggttt 2940

aaactatgtt atcaaatcat atttcaccgt gaatattcag tggagaactt ctctacctgg 3000

atgagctagt aatgatttca gatcatgcta tccccagaaa taaaagcaaa aaataatacc 3060

tgtgtggaat ataggctgtg ctttgattta ctggtattta ccccaaaata ggctgtgtat 3120

gggggctgac ttaaagatcc cttggaaaga ctcaaaacta ccttcactag taggactcct 3180

aagcgctgac ctatttttaa atgacacaaa ttcatgaaac taatgttaca aattcatgca 3240

gtttgcactc ttagtcatct tcccctagca caccaataga atgttagaca aagccagcac 3300

tgttttgaaa atacagccaa acacgatgac ttttgttttg ttttctgccg ttcttaaaag 3360

aaaaaaagat aatattgcaa ctctgactga aagacttatt tttaagaaaa caggttgtgt 3420

ttggtgctgc taagttctgg ccagtttatc atctggcctt cctgcctatt ttttacaaaa 3480

cacgaagaca gtgtgtaacc tcgacatttt gaccttcctt tatgtgctag tttagacagg 3540

ctcctgaatc cacacttaat tttgcttaac aaaagtctta atagtaaacc tcccctcatg 3600

agcttgaagt caagtgttct tgacttcaga tatttctttc cttttttttt ttttttcctc 3660

atcacaacta agagatacac aaactctgaa gaagcagaaa tggagagaat gcttttaaca 3720

aaaaagcatc tgatgaaaga ttttaggcaa acattctcaa aataagagtg atattctgga 3780

tgtagttatt gcagttatct catgacaaat gaggcctgga ttggaaggaa aatatagttg 3840

tgtagaatta agcatttgga taggaatcta caaggtagtt gaatataata agcaggtttg 3900

ggcccccaaa ctttagaaaa tcaaatgcaa aggtgctggc aaaaatgagg tttgagtggc 3960

tggctgtaag agaaggttaa ctcctagtaa aaggcatttt tagaaataac aattactgaa 4020

aactttgaag tatagtggga gtagcaaaca aatacatgtt tttttttctt tacaaagaac 4080

tcctaaatcc tgagtaagtg ccattcatta caataagtct ctaaatttaa aaaaaaaaaa 4140

atcatatgag gaaatctagc tttccccttt acgctgcgtt tgatctttgt ctaaatagtg 4200

ttaaaattcc tttcattcca attacagaac tgagcccact cgcaagttgg agccatcagt 4260

gggatacgcc acattttgga agccccagca tcgtgtactt accagtgtgt tcacaaaatg 4320

aaatttgtgt gagagctgta cattaaaaaa aatcatcatt attattatta tttgcagtca 4380

tggagaacca cctacccctg acttctgttt agtctccttt ttaaataaaa attactgtgt 4440

tagagaagaa 4450

<210> 6
<211> 812
<212> DNA
<213> Homo sapiens

<400> 6
atcttttttc atcttctagc tgtagctcgg gctgcttgtc ggcttggcct ccccctcccc 60

cctttgctct ctgcctcgtc tttccccagg acttcgctat tttgcttttt taaaaaaagg 120

caagaaagaa ctaaactccc ccctccctct cctccagtcg ggctgcacct ctgccttgca 180

ctttgcacag aggtagagag cgcgcgaggg agagagagga aagaaaaaaa ataataaaga 240

gagccaagca gaagaggagg cgagaagcat gaagtgttaa ctcccccgtg ccaaggcccg 300

cgccgcccgg acagacgccc gccgcgcctc cagccccgag cggacgccgc gcgcgccctg 360

cctgcagccc gggccggcga ggcgagccct tccttatgca aagcgcgcag cggagcggcg 420

agcgggggac gccgcgcacc gggccgggct cctccagctt cgccgccgca gccaccaccg 480

ccgccaccgc agctcgcgga ggatcttccc gagcctgaag ccgccggctc ggcgcgcaag 540

gaggcgagcg agcaaggagg ggccggggcg agcgagggag cacattggcg tgagcagggg 600

ggagggaggg cgggcgcggg gggcgcgggc agggcggggg ggtgtgtgtg tgagcgcgct 660

cggaggtttc gggccagcca ccgccgcgca agctagaagc gccccagccc ggcaagctgg 720

ctcacccgct ggccacccag cacagcccgc tggcccctct cctgcagccc atctggcgga 780

gcggcggcgg cggcggcggc ggcggcagga ga 812

<210> 7
<211> 6384
<212> DNA
<213> Homo sapiens

<400> 7
atcttttttc atcttctagc tgtagctcgg gctgcttgtc ggcttggcct ccccctcccc 60

cctttgctct ctgcctcgtc tttccccagg acttcgctat tttgcttttt taaaaaaagg 120

caagaaagaa ctaaactccc ccctccctct cctccagtcg ggctgcacct ctgccttgca 180

ctttgcacag aggtagagag cgcgcgaggg agagagagga aagaaaaaaa ataataaaga 240

gagccaagca gaagaggagg cgagaagcat gaagtgttaa ctcccccgtg ccaaggcccg 300

cgccgcccgg acagacgccc gccgcgcctc cagccccgag cggacgccgc gcgcgccctg 360

cctgcagccc gggccggcga ggcgagccct tccttatgca aagcgcgcag cggagcggcg 420

agcgggggac gccgcgcacc gggccgggct cctccagctt cgccgccgca gccaccaccg 480

ccgccaccgc agctcgcgga ggatcttccc gagcctgaag ccgccggctc ggcgcgcaag 540

gaggcgagcg agcaaggagg ggccggggcg agcgagggag cacattggcg tgagcagggg 600

ggagggaggg cgggcgcggg gggcgcgggc agggcggggg ggtgtgtgtg tgagcgcgct 660

cggaggtttc gggccagcca ccgccgcgca agctagaagc gccccagccc ggcaagctgg 720

ctcacccgct ggccacccag cacagcccgc tggcccctct cctgcagccc atctggcgga 780

gcggcggcgg cggcggcggc ggcggcagga gaatggcatc agaactggca atgagcaact 840

ccgacctgcc caccagtccc ctggccatgg aatatgttaa tgacttcgat ctgatgaagt 900

ttgaagtgaa aaaggaaccg gtggagaccg accgcatcat cagccagtgc ggccgtctca 960

tcgccggggg ctcgctgtcc tccaccccca tgagcacgcc gtgcagctcg gtgccccctt 1020

cccccagctt ctcggcgccc agcccgggct cgggcagcga gcagaaggcg cacctggaag 1080

actactactg gatgaccggc tacccgcagc agctgaaccc cgaggcgctg ggcttcagcc 1140

ccgaggacgc ggtcgaggcg ctcatcagca acagccacca gctccagggc ggcttcgatg 1200

gctacgcgcg cggggcgcag cagctggccg cggcggccgg ggccggtgcc ggcgcctcct 1260

tgggcggcag cggcgaggag atgggccccg ccgccgccgt ggtgtccgcc gtgatcgccg 1320

cggccgccgc gcagagcggc gcgggcccgc actaccacca ccaccaccac cacgccgccg 1380

gccaccacca ccacccgacg gccggcgcgc ccggcgccgc gggcagcgcg gccgcctcgg 1440

ccggtggcgc tgggggcgcg ggcggcggtg gcccggccag cgctgggggc ggcggcggcg 1500

gcggcggcgg cggaggcggc gggggcgcgg cgggggcggg gggcgccctg cacccgcacc 1560

acgccgccgg cggcctgcac ttcgacgacc gcttctccga cgagcagctg gtgaccatgt 1620

ctgtgcgcga gctgaaccgg cagctgcgcg gggtcagcaa ggaggaggtg atccggctga 1680

agcagaagag gcggaccctg aaaaaccgcg gctatgccca gtcctgccgc ttcaagaggg 1740

tgcagcagag acacgtcctg gagtcggaga agaaccagct gctgcagcaa gtcgaccacc 1800

tcaagcagga gatctccagg ctggtgcgcg agagggacgc gtacaaggag aaatacgaga 1860

agttggtgag cagcggcttc cgagaaaacg gctcgagcag cgacaacccg tcctctcccg 1920

agtttttcat gtgagtctga cacgcgattc cagctagcca ccctgataag tgctccgcgg 1980

gggtccggct cgggtgtggg cttgctagtt ctagagccat gctcgccacc acctcaccac 2040

ccccaccccc accgagtttg gcccccttgg ccccctacac acacacaaac ccgcacgcac 2100

acaccacaca cacacacaca cacacacaca caccccacac cctgctcgag tttgtggtgg 2160

tggtggctgt tttaaactgg ggagggaatg ggtgtctggc tcatggattg ccaatctgaa 2220

attctccata acttgctagc ttgttttttt ttttttttta cacccccccg ccccaccccc 2280

ggacttgcac aatgttcaat gatctcagca gagttcttca tgtgaaacgt tgatcacctt 2340

tgaagcctgc atcattcaca tattttttct tcttcttccc cttcagttca tgaactggtg 2400

ttcattttct gtgtgtgtgt gtgttttatt ttgtttggat tttttttttt aattttactt 2460

ttagagcttg ctgtgttgcc cacctttttt ccaacctcca ccctcactcc ttctcaaccc 2520

atctcttccg agatgaaaga aaaaaaaaag caaagttttt ttttcttctc ctgagttctt 2580

catgtgagat tgagcttgca aaggaaaaaa aaatgtgaaa tgttatagac ttgcagcgtg 2640

ccgagttcca tcgggttttt tttttagcat tgttatgcta aaatagagaa aaaaatcctc 2700

atgaaccttc cacaatcaag cctgcatcaa ccttctgggt gtgacttgtg agttttggcc 2760

ttgtgatgcc aaatctgaga gtttagtctg ccattaaaaa aactcattct catctcatgc 2820

attattatgc ttgctacttt gtcttagcaa caatgaacta taactgtttc aaagacttta 2880

tggaaaagag acattatatt aataaaaaaa aaaagcctgc atgctggaca tgtatggtat 2940

aattattttt tccttttttt ttccttttgg cttggaaatg gacgttcgaa gacttatagc 3000

atggcattca tacttttgtt ttattgcctc atgacttttt tgagtttaga acaaaacagt 3060

gcaaccgtag agccttcttc ccatgaaatt ttgcatctgc tccaaaactg ctttgagtta 3120

ctcagaactt caacctccca atgcactgaa ggcattcctt gtcaaagata ccagaatggg 3180

ttacacattt aacctggcaa acattgaaga actcttaatg ttttcttttt aataagaatg 3240

acgccccact ttggggacta aaattgtgct attgccgaga agcagtctaa aatttatttt 3300

ttaaaaagag aaactgcccc attatttttg gtttgtttta tttttatttt atattttttg 3360

gcttttggtc attgtcaaat gtggaatgct ctgggtttct agtatataat ttaattctag 3420

tttttataat ctgttagccc agttaaaatg tatgctacag ataaaggaat gttatagata 3480

aatttgaaag agttaggtct gtttagctgt agatttttta aacgattgat gcactaaatt 3540

gtttactatt gtgatgttaa ggggggtaga gtttgcaagg ggactgttta aaaaaagtag 3600

cttatacagc atgtgcttgc aacttaaata taagttgggt atgtgtagtc tttgctatac 3660

cactgactgt attgaaaacc aaagtattaa gaggggaaac gcccctgttt atatctgtag 3720

gggtatttta cattcaaaaa tgtatgtttt tttttctttt caaaattaaa gtatttggga 3780

ctgaattgca ctaagatata acctgcaagc atataataca aaaaaaaatt gcaaaactgt 3840

ttagaacgct aataaaattt atgcagttat aaaaatggca ttactgcaca gttttaagat 3900

gatgcagatt tttttacagt tgtattgtgg tgcagaactg gattttctgt aacttaaaaa 3960

aaaatccaca gttttaaagg caataatcag taaatgttat tttcagggac tgacatcctg 4020

tctttaaaaa gaaatgaaaa gtaaatctta ccacaataaa tataaaaaaa tcttgtcagt 4080

tacttttctt ttacatattt tgctgtgcaa aattgtttta tatcttgagt tactaactaa 4140

ccacgcgtgt tgttcctatg tgcttttctt tcattttcaa ttctggttat atcaagaaaa 4200

gaataatcta caataataaa cggcattttt ttttgattct gtactcagtt tcttagtgta 4260

cagtttaact gggcccaaca acctcgttaa aagtgtaaaa tgcatccttt tctccagtgg 4320

aaggattcct ggaggaatag ggagacagta attcagggtg aaattatagg ctgttttttg 4380

aagtgaggag gctggcccca tatactgatt agcaatattt aatatagatg taaattatga 4440

cctcattttt ttctccccaa agttttcagt tttcaaatga gttgagccat aattgccctt 4500

ggtaggaaaa acaaaacaaa acagtggaac taggcttcct gagcatggcc ctacacttct 4560

gatcaggagc aaagccatcc atagacagag gagccggaca aatatggcgc atcagaggtg 4620

gcttgcgcac atatgcattg aacggtaaag agaaacagcg cttgcctttt cactaaagtt 4680

gactattttt ccttcttctc ttacacaccg agatttcttt gttagcaagg cctgacaaga 4740

tttaacataa acatgacaaa tcatagttgt ttgttttgtt ttgcttttct ctttaacact 4800

gaagatcatt tgtcttaaat aggaaaaaga aaatccactc cttacttcca tatttccaag 4860

tacatatctg gtttaaacta tgttatcaaa tcatatttca ccgtgaatat tcagtggaga 4920

acttctctac ctggatgagc tagtaatgat ttcagatcat gctatcccca gaaataaaag 4980

caaaaaataa tacctgtgtg gaatataggc tgtgctttga tttactggta tttaccccaa 5040

aataggctgt gtatgggggc tgacttaaag atcccttgga aagactcaaa actaccttca 5100

ctagtaggac tcctaagcgc tgacctattt ttaaatgaca caaattcatg aaactaatgt 5160

tacaaattca tgcagtttgc actcttagtc atcttcccct agcacaccaa tagaatgtta 5220

gacaaagcca gcactgtttt gaaaatacag ccaaacacga tgacttttgt tttgttttct 5280

gccgttctta aaagaaaaaa agataatatt gcaactctga ctgaaagact tatttttaag 5340

aaaacaggtt gtgtttggtg ctgctaagtt ctggccagtt tatcatctgg ccttcctgcc 5400

tattttttac aaaacacgaa gacagtgtgt aacctcgaca ttttgacctt cctttatgtg 5460

ctagtttaga caggctcctg aatccacact taattttgct taacaaaagt cttaatagta 5520

aacctcccct catgagcttg aagtcaagtg ttcttgactt cagatatttc tttccttttt 5580

tttttttttt cctcatcaca actaagagat acacaaactc tgaagaagca gaaatggaga 5640

gaatgctttt aacaaaaaag catctgatga aagattttag gcaaacattc tcaaaataag 5700

agtgatattc tggatgtagt tattgcagtt atctcatgac aaatgaggcc tggattggaa 5760

ggaaaatata gttgtgtaga attaagcatt ttgataggaa tctacaaggt agttgaatat 5820

aataagcagg tttgggcccc caaactttag aaaatcaaat gcaaaggtgc tggcaaaaat 5880

gaggtttgag tggctggctg taagagaagg ttaactccta gtaaaaggca tttttagaaa 5940

taacaattac tgaaaacttt gaagtatagt gggagtagca aacaaataca tgtttttttt 6000

ttcttacaaa gaactcctaa atcctgagta agtgccattc attacaataa gtctctaaat 6060

ttaaaaaaaa aaaaatcata tgaggaaatc tagctttccc ctttacgctg cgtttgatct 6120

ttgtctaaat agtgttaaaa ttcctttcat tccaattaca gaactgagcc cactcgcaag 6180

ttggagccat cagtgggata cgccacattt tggaagcccc agcatcgtgt acttaccagt 6240

gtgttcacaa aatgaaattt gtgtgagagc tgtacattaa aaaaaatcat cattattatt 6300

attatttgca gtcatggaga accacctacc cctgacttct gtttagtctc ctttttaaat 6360

aaaaattact gtgttagaga agaa 6384

<210> 8
<211> 6360
<212> DNA
<213> Mus musculus

<400> 8
ggagctgtag ctcgggctgc tcgtccgctc ggcctctccc cccaaccctt ttgctctcgg 60

ctttgccttg gcccaggact tgcaattttg ctttaaaaaa ataataagaa agaaaaaaag 120

aaaagaaaag aaaaaccaaa accaaaagaa aaaaagtggg ggaagaaaga acttaactcc 180

cccctccctt gcctccagcc ggcctgcacc tcggtcttgc actttgcacg gagggagagg 240

gtgagagaga gagagagaga gagagagagc gcgcgcacgc gagggatccg gagagagaaa 300

agaaaataat aatagtaacc ataagagcca ggcagaagag gagagaagcg tgaagtgtta 360

accactccgg tgtggaggcc ggcgcctccg ggacagccac ccgccgcgcc acacagcccc 420

ggagcagcct ccgcgcgcgc cccgcctgca gcccggccgg cttaggcgag ccctccttat 480

gcaaagcgcg cagcggagcg gcgagccggg gacgccgcgc accgggccgt gctcctccgg 540

catcctctct gccgctgtca tcaccaccgt caccgccagc cgcggaggac cttcccgagc 600

ctgaagcgcc cgggatcgga gctcagggag gcgagcaggc gaggaggggg cccggggcga 660

gcggaggaga gagagcgaga gagcaagaga gagaaagaga gagagcacat tggcgtgagc 720

agggggaggg agggcgggcg cggggggctg cgggcagggc ggggggtgtg tgcacgttcg 780

agctttcggg ccacagccac cgccgcgcaa gctagaagcg ccctagcccg gcaagctggc 840

tcacccgctg gccacccagc acagcccgct ggcccctctc ctgcagccca tctggcggag 900

cggcggcggc ggtggcggcg gaggcaggag gatggcttca gaactggcaa tgaacaattc 960

cgacctgccc accagtcccc tggccatgga atatgttaat gacttcgatc tgatgaagtt 1020

tgaagtgaaa aaggaaccgg tggagaccga ccgcatcatc agccagtgcg gccgtctcat 1080

cgccgggggc tcgctgtcct ccacccccat gagcacgccc tgcagctcgg tgcccccgtc 1140

ccccagcttc tcggcgccca gcccgggctc gggcagcgaa cagaaggcgc acctggaaga 1200

ctactactgg atgaccggct acccgcagca gctcaacccg gaggcgctgg gcttcagccc 1260

ggaggacgcg gtcgaggcgc tcatcagcaa cagccaccag ctccagggtg gcttcgatgg 1320

ctatgcgcgg ggagcgcagc agctggccgc ggcagcgggg gccggcgccg gcgcctccct 1380

gggcggcagc ggcgaggaga tgggccccgc cgccgccgtg gtgtccgccg tgatcgccgc 1440

ggccgccgcg cagagcggcg cggcacccca ctaccatcac caccaccacc acgccgcggg 1500

gcaccaccac catccgacgg ccggcgcccc gggagccgcg ggcggcgcgt ctgcctctgc 1560

gagcggcgcg ggtggcgcgg gcggcggtgg cccggccagc gccgggggcg gcggcggcgg 1620

aggcggcggc gggggcacgg cgggggcggg gggcgccctg cacccgcacc atgccgcggg 1680

cggcctgcac ttcgacgacc gcttctcgga cgagcagttg gtgaccatgt cggtgcgcga 1740

gctgaaccgg cagctgcgcg gggtcagcaa ggaggaggtg atccgactga agcagaagag 1800

gcggaccctg aaaaaccgcg gctatgccca gtcctgccgc ttcaagaggg tgcagcagag 1860

acacgtcctg gagtcggaga agaaccagct gctgcagcag gtagaccacc tcaagcagga 1920

gatctccagg ctggtgcgcg aaagggacgc ctacaaggag aaatacgaga agctggtgag 1980

caacggcttc cgagaaaacg gctcgagcag cgacaaccct tcctctcccg aatttttcat 2040

gtgagtgtga cacgcgatcc cagctagcca ccttgttaaa tgctccgtgg gggtcccgct 2100

cctgagtggg cttgctagtt gcagggccaa gctcgctcgg ccaccacctc gccccctccc 2160

ctggcgagtt tgctcccctt ggccctgaac agacacacac acacacacac acacacacac 2220

acacacacac acacacacac acaccaagct tgtggtggtg gtgatggctc ttttgaattg 2280

gggagggaaa gggtgtctgg ctcatggatt gccaacctga attcccccgt aacttgctag 2340

cttggttgtt ttgttctttt ttattattat tattattatt acaactacta ctctttttac 2400

accgcctgcc ccacacatac cctggacttg cacaagggtc aaagatctga gcagcatact 2460

tcatgtaggg catagctcac ctttgaaccg ggccatcaat tgcctgtata tatatttttt 2520

cttcttcttc ttcagctcat gagctggtgt tcattctgtg tgtgtgtgtt tgtgtgtttt 2580

attttgtttg gattttcttt tttcttcttc ttcttctttg tttttgttta ttattactat 2640

tactaatttt ttggagcttg ctgtgtttcc cagcctctgc caccccccac ccccacccct 2700

gcaccatgtt cttgagataa aaggaaaaaa aaaaaaagca gggttttttt ttcccctcct 2760

tctgagttct tctgattgag cttgcaaagg aaaaaaaaat gtgaaatgct atagaccttg 2820

cagcgagctg agttccacgg gggttttatc ttagcattgt tatgctaaaa cagaatgaac 2880

taaagccact cctgaacctg ccacaatcaa gcctacatca accttctggg tgtgacttgt 2940

gagttttggc cttatgatgc caaatctgaa agtttagtct gccattaaac aacaaacaaa 3000

caaaaacaca aaaacaaact tactctcatc tcatgcatta cgatgcttgc tactttgtct 3060

tagcaacaat gaactataac agtttcaaag acattatgga aaagagacat tatattaata 3120

aaagaagcct gcatgctgga catgtatggt ataattattt ttttcctttt ttttcccccc 3180

aatttggctt ggaaatggac ttttgaagac ttagcgcatg acatccatac ttttgattta 3240

cagcctcatg acttttttga gttccaaaca acaacagaaa aagtacaacc ccagagcctt 3300
cttcccttga cagtttgctt ctactcctga ctgctttgag ttacaatgaa cttcagcctc 3360
ccgttgtaca tgcactgaag gcgttccttg tcaaagatag cagaatgggc tacaaattta 3420
tatggcaaat attatagaac gtaaattaaa tgttttcttt ttagaaacaa tgatgcctgc 3480
tttggggcac tttaaattgt gctattgcca aaagcagtct aaattttctt tgaaaagaaa 3540
gaaaaaaaaa atctgcccca attatttttt ggttggtttt attttttttt attttatttt 3600
ttctattttt tttttaattt ttttggtttt tggtcattgt caaatgtgga atgctatggg 3660
tttctagtat ataatttaat tctagatttt ataatctgtt agcccagtta aaatgtatgc 3720
tacagataaa ggaatgttat agatacattt gaaagagtta ggtctgtgta gctgtagatt 3780
tttttaaaga ccgatgcact aaattgttta ctgttgtgat gttaaggggg gtagagtttg 3840
caaggggact gtttaaaaag tagctttctg aagcatgtgc ttgcaactta aatataagtt 3900
gggtatgtgt agttcttgct ataccatgga ctgtattgaa aaccaaagta ttaaaagggg 3960
aaatgcccct gtttatatct gtaggggtat tttacattca aaatgtatgt ttttttccaa 4020
aattaaagta tttgggactg aattgcacta agatataacc tgcaagcata taatacaaaa 4080
aaaaatcgca aaaactgttt agaacgctaa taaaatttat gcagttataa aatggcatta 4140
ctgcacagtt ttaaagtgat gcagattttt tacagttatg ttgtggtgca gaactggatt 4200
ttctgtaact taaaaaaatc cacagtttta aaggcaataa ccagtaaatg ttattttcag 4260
ggactgacat cctgtcttag aaagaaagaa agaaagaaag aaagaaagaa agaaagaaag 4320
aaagaaagaa agaaagaaaa ggaaaggaaa tcttaccaca ataaatataa gaaatcttgt 4380
cagttacttt tcttttacat attttgctgt gcaaaattgt tttataactt gagttactaa 4440
ctaaccacgc gtgacgttct acatgcttct ctttcatttt caattctggt tatatcaaga 4500
gaaagaataa tctacaataa taaactgcat tttttttgat tctgttccgt ttcttagtgt 4560
acagattaac tgggtcccat gtcttggagc ttggatatga aatacaaaat gcattctctt 4620
tgatttctga acaagggagt tggaggcagg aattctattc tgagaggcag gagattgaca 4680
ccatatagtc agtaacaata tttaatatta atacaaatta tgacatctta ttttttctcc 4740
tcacagatct tagtttccta ataggctgag ctagtaagga aaaaaagtga gaaagaggga 4800
cagtgagaga gaaagataga gagatagaca gacagacaga cagacagaca gagacacaca 4860
cacacacaca cacacacaca cacacacaca cacactgaga gcacatagga gctttctgaa 4920
caggactcta agcttctgac gaaggagcac agatagaggg actgggacca gcaagtctgg 4980
ctcaccagag gtgcatggga acatatgcat ttaatggtaa agataaataa ttcttgccgt 5040
ttggccaaaa ttgattattt tttcttcttc ccttacatgt tgtgattttc ttgttggcac 5100
agtcctgaca agactaaaca taaacacttt gcacaaatca aagttgtttg attttgctct 5160

tttttccccc tgtcattttt ttttaaactt ttcaagtcac ttgtcctaaa tgggaaaaag 5220

aaagtctatc atttgcacac agagtttcca gttcacacca ggttttatct atattatcta 5280

actatatttc gaatagtcaa agagctgtct ccaagcatat aagctaggga taactacatg 5340

agtcccccaa atataaatat acaaagaaaa caacattttc atggggtgta ggcggtgctc 5400

tgatatattg ctgtttgccc ccaaagcagg ttatgaatgg ggtctaattg aatgatcact 5460

tggaaaaata tctcaattac cttcagttga attcctaaac cctgacccat ttaaaactga 5520

cacaaattcc tgcaaccatg ttacaaatac atggcatttt ctctttcggt aggactccct 5580

gagaattaat cccatagtag acaaagccaa cactgttttg gaaatatagc gaaacacaaa 5640

tgacttttgt ctgattatgt tttcgattct tttttctttt ttaaaatact agctaatatt 5700

gcaacccacc atgcaggcct gatttctaag gaatcgggta gcccttcagg ctggggaatc 5760

ctggcctgtt tctcatgggc cttggctctt ttgcacagaa cacagactgt ttacacttcc 5820

tatgttggcg tcactcggtg agcttagacg tgagtcctca aaacacttag ttctgcttga 5880

cagagaaatc ttaatggcaa aacttctctt ctatgctcta agttagccgt cctcagcttt 5940

agagattttc atcatctgac tacagccaga aaataggcac acattaccag tctggcgagg 6000

aggaaatggg tatatcactt ggacccaaag ctcagcacca gaagaaagag tttaggcaaa 6060

tatattctta ggcacaagag gcatttgtaa cacgatggtc gcagttgttt atcaaagagt 6120

aagacctggg ttggggtggg ggagatgcaa ttatatataa taattaatca ctttgataga 6180

gttctataaa gcagttcaat atcagaagcc agtttgggcc ctggacatta gaaaatgaga 6240

cagcaccatc agaataaaga ggaggcggag tgactggctc aaagcaaaag ttaaccttca 6300

ctgaaaagcg ctttcagaca cgagagactt cagaagataa tactaaacag aactaaacgc 6360

<210> 9
<211> 1113
<212> DNA
<213> Mus musculus

<400> 9
atggcttcag aactggcaat gaacaattcc gacctgccca ccagtcccct ggccatggaa 60

tatgttaatg acttcgatct gatgaagttt gaagtgaaaa aggaaccggt ggagaccgac 120

cgcatcatca gccagtgcgg ccgtctcatc gccgggggct cgctgtcctc cacccccatg 180

agcacgccct gcagctcggt gcccccgtcc cccagcttct cggcgcccag cccgggctcg 240

ggcagcgaac agaaggcgca cctggaagac tactactgga tgaccggcta cccgcagcag 300

ctcaacccgg aggcgctggg cttcagcccg gaggacgcgg tcgaggcgct catcagcaac 360

agccaccagc tccagggtgg cttcgatggc tatgcgcggg gagcgcagca gctggccgcg 420

gcagcggggg ccggcgccgg cgcctccctg ggcggcagcg gcgaggagat gggccccgcc 480

gccgccgtgg tgtccgccgt gatcgccgcg gccgccgcgc agagcggcgc ggcaccccac 540

taccatcacc accaccacca cgccgcgggg caccaccacc atccgacggc cggcgccccg 600

ggagccgcgg gcggcgcgtc tgcctctgcg agcggcgcgg gtggcgcggg cggcggtggc 660

ccggccagcg ccgggggcgg cggcggcgga ggcggcggcg ggggcacggc gggggcgggg 720

ggcgccctgc acccgcacca tgccgcgggc ggcctgcact tcgacgaccg cttctcggac 780

gagcagttgg tgaccatgtc ggtgcgcgag ctgaaccggc agctgcgcgg ggtcagcaag 840

gaggaggtga tccgactgaa gcagaagagg cggaccctga aaaaccgcgg ctatgcccag 900

tcctgccgct tcaagagggt gcagcagaga cacgtcctgg agtcggagaa gaaccagctg 960

ctgcagcagg tagaccacct caagcaggag atctccaggc tggtgcgcga aagggacgcc 1020

tacaaggaga aatacgagaa gctggtgagc aacggcttcc gagaaaacgg ctcgagcagc 1080

gacaaccctt cctctcccga atttttcatg tga 1113

<210> 10
<211> 6360
<212> DNA
<213> Mus musculus

<400> 10
ggagctgtag ctcgggctgc tcgtccgctc ggcctctccc cccaaccctt ttgctctcgg 60

ctttgccttg gcccaggact tgcaattttg ctttaaaaaa ataataagaa agaaaaaaag 120

aaaagaaaag aaaaaccaaa accaaaagaa aaaaagtggg ggaagaaaga acttaactcc 180

cccctccctt gcctccagcc ggcctgcacc tcggtcttgc actttgcacg gagggagagg 240

gtgagagaga gagagagaga gagagagagc gcgcgcacgc gagggatccg gagagagaaa 300

agaaaataat aatagtaacc ataagagcca ggcagaagag gagagaagcg tgaagtgtta 360

accactccgg tgtggaggcc ggcgcctccg ggacagccac ccgccgcgcc acacagcccc 420

ggagcagcct ccgcgcgcgc cccgcctgca gcccggccgg cttaggcgag ccctccttat 480

gcaaagcgcg cagcggagcg gcgagccggg gacgccgcgc accgggccgt gctcctccgg 540

catcctctct gccgctgtca tcaccaccgt caccgccagc cgcggaggac cttcccgagc 600

ctgaagcgcc cgggatcgga gctcagggag gcgagcaggc gaggaggggg cccggggcga 660

gcggaggaga gagagcgaga gagcaagaga gagaaagaga gagagcacat tggcgtgagc 720

agggggaggg agggcgggcg cggggggctg cgggcagggc ggggggtgtg tgcacgttcg 780

agctttcggg ccacagccac cgccgcgcaa gctagaagcg ccctagcccg gcaagctggc 840

tcacccgctg gccacccagc acagcccgct ggcccctctc ctgcagccca tctggcggag 900

cggcggcggc ggtggcggcg gaggcaggag gatggcttca gaactggcaa tgaacaattc 960

cgacctgccc accagtcccc tggccatgga atatgttaat gacttcgatc tgatgaagtt 1020

tgaagtgaaa aaggaaccgg tggagaccga ccgcatcatc agccagtgcg gccgtctcat 1080

cgccgggggc tcgctgtcct ccacccccat gagcacgccc tgcagctcgg tgcccccgtc 1140

ccccagcttc tcggcgccca gcccgggctc gggcagcgaa cagaaggcgc acctggaaga 1200

ctactactgg atgaccggct acccgcagca gctcaacccg gaggcgctgg gcttcagccc 1260

ggaggacgcg gtcgaggcgc tcatcagcaa cagccaccag ctccagggtg gcttcgatgg 1320

ctatgcgcgg ggagcgcagc agctggccgc ggcagcgggg gccggcgccg gcgcctccct 1380

gggcggcagc ggcgaggaga tgggccccgc cgccgccgtg gtgtccgccg tgatcgccgc 1440

ggccgccgcg cagagcggcg cggcacccca ctaccatcac caccaccacc acgccgcggg 1500

gcaccaccac catccgacgg ccggcgcccc gggagccgcg ggcggcgcgt ctgcctctgc 1560

gagcggcgcg ggtggcgcgg gcggcggtgg cccggccagc gccgggggcg gcggcggcgg 1620

aggcggcggc gggggcacgg cgggggcggg gggcgccctg cacccgcacc atgccgcggg 1680

cggcctgcac ttcgacgacc gcttctcgga cgagcagttg gtgaccatgt cggtgcgcga 1740

gctgaaccgg cagctgcgcg gggtcagcaa ggaggaggtg atccgactga agcagaagag 1800

gcggaccctg aaaaaccgcg gctatgccca gtcctgccgc ttcaagaggg tgcagcagag 1860

acacgtcctg gagtcggaga agaaccagct gctgcagcag gtagaccacc tcaagcagga 1920

gatctccagg ctggtgcgcg aaaggacgcc ctacaaggag aaatacgaga agctggtgag 1980

caacggcttc cgagaaaacg gctcgagcag cgacaaccct tcctctcccg aatttttcat 2040

gtgagtgtga cacgcgatcc cagctagcca ccttgttaaa tgctccgtgg gggtcccgct 2100

cctgagtggg cttgctagtt gcagggccaa gctcgctcgg ccaccacctc gccccctccc 2160

ctggcgagtt tgctcccctt ggccctgaac agacacacac acacacacac acacacacac 2220

acacacacac acacacacac acaccaagct tgtggtggtg gtgatggctc ttttgaattg 2280

gggagggaaa gggtgtctgg ctcatggatt gccaacctga attcccccgt aacttgctag 2340

cttggttgtt ttgttctttt ttattattat tattattatt acaactacta ctctttttac 2400

accgcctgcc ccacacatac cctggacttg cacaagggtc aaagatctga gcagcatact 2460

tcatgtaggg catagctcac ctttgaaccg ggccatcaat tgcctgtata tatatttttt 2520

cttcttcttc ttcagctcat gagctggtgt tcattctgtg tgtgtgtgtt tgtgtgtttt 2580

attttgtttg gattttcttt tttcttcttc ttcttctttg tttttgttta ttattactat 2640

tactaatttt ttggagcttg ctgtgtttcc cagcctctgc caccccccac ccccacccct 2700

gcaccatgtt cttgagataa aaggaaaaaa aaaaaaagca gggttttttt ttcccctcct 2760

tctgagttct tctgattgag cttgcaaagg aaaaaaaaat gtgaaatgct atagaccttg 2820

cagcgagctg agttccacgg gggttttatc ttagcattgt tatgctaaaa cagaatgaac 2880

taaagccact cctgaacctg ccacaatcaa gcctacatca accttctggg tgtgacttgt 2940

gagttttggc cttatgatgc caaatctgaa agtttagtct gccattaaac aacaaacaaa 3000

caaaaacaca aaaacaaact tactctcatc tcatgcatta cgatgcttgc tactttgtct 3060

tagcaacaat gaactataac agtttcaaag acattatgga aaagagacat tatattaata 3120

aaagaagcct gcatgctgga catgtatggt ataattattt ttttcctttt ttttcccccc 3180

aatttggctt ggaaatggac ttttgaagac ttagcgcatg acatccatac ttttgattta 3240

cagcctcatg acttttttga gttccaaaca acaacagaaa aagtacaacc ccagagcctt 3300

cttcccttga cagtttgctt ctactcctga ctgctttgag ttacaatgaa cttcagcctc 3360

ccgttgtaca tgcactgaag gcgttccttg tcaaagatag cagaatgggc tacaaattta 3420

tatggcaaat attatagaac gtaaattaaa tgttttcttt ttagaaacaa tgatgcctgc 3480

tttggggcac tttaaattgt gctattgcca aaagcagtct aaattttctt tgaaaagaaa 3540

gaaaaaaaaa atctgcccca attatttttt ggttggtttt attttttttt attttatttt 3600

ttctattttt tttttaattt ttttggtttt tggtcattgt caaatgtgga atgctatggg 3660

tttctagtat ataatttaat tctagatttt ataatctgtt agcccagtta aaatgtatgc 3720

tacagataaa ggaatgttat agatacattt gaaagagtta ggtctgtgta gctgtagatt 3780

tttttaaaga ccgatgcact aaattgttta ctgttgtgat gttaaggggg gtagagtttg 3840

caaggggact gtttaaaaag tagctttctg aagcatgtgc ttgcaactta aatataagtt 3900

gggtatgtgt agttcttgct ataccatgga ctgtattgaa aaccaaagta ttaaaagggg 3960

aaatgcccct gtttatatct gtaggggtat tttacattca aaatgtatgt ttttttccaa 4020

aattaaagta tttgggactg aattgcacta agatataacc tgcaagcata taatacaaaa 4080

aaaaatcgca aaaactgttt agaacgctaa taaaatttat gcagttataa aatggcatta 4140

ctgcacagtt ttaaagtgat gcagattttt tacagttatg ttgtggtgca gaactggatt 4200

ttctgtaact taaaaaaatc cacagtttta aaggcaataa ccagtaaatg ttattttcag 4260

ggactgacat cctgtcttag aaagaaagaa agaaagaaag aaagaaagaa agaaagaaag 4320

aaagaaagaa agaaagaaaa ggaaaggaaa tcttaccaca ataaatataa gaaatcttgt 4380

cagttacttt tcttttacat attttgctgt gcaaaattgt tttataactt gagttactaa 4440

ctaaccacgc gtgacgttct acatgcttct ctttcatttt caattctggt tatatcaaga 4500

gaaagaataa tctacaataa taaactgcat tttttttgat tctgttccgt ttcttagtgt 4560

acagattaac tgggtcccat gtcttggagc ttggatatga aatacaaaat gcattctctt 4620

tgatttctga acaagggagt tggaggcagg aattctattc tgagaggcag gagattgaca 4680

ccatatagtc agtaacaata tttaatatta atacaaatta tgacatctta ttttttctcc 4740

tcacagatct tagtttccta ataggctgag ctagtaagga aaaaaagtga gaaagaggga 4800

cagtgagaga gaaagataga gagatagaca gacagacaga cagacagaca gagacacaca 4860

cacacacaca cacacacaca cacacacaca cacactgaga gcacatagga gctttctgaa 4920

caggactcta agcttctgac gaaggagcac agatagaggg actgggacca gcaagtctgg 4980

ctcaccagag gtgcatggga acatatgcat ttaatggtaa agataaataa ttcttgccgt 5040

ttggccaaaa ttgattattt tttcttcttc ccttacatgt tgtgattttc ttgttggcac 5100

agtcctgaca agactaaaca taaacacttt gcacaaatca aagttgtttg attttgctct 5160

tttttccccc tgtcattttt ttttaaactt ttcaagtcac ttgtcctaaa tgggaaaaag 5220

aaagtctatc atttgcacac agagtttcca gttcacacca ggttttatct atattatcta 5280

actatatttc gaatagtcaa agagctgtct ccaagcatat aagctaggga taactacatg 5340

agtcccccaa atataaatat acaaagaaaa caacattttc atggggtgta ggcggtgctc 5400

tgatatattg ctgtttgccc ccaaagcagg ttatgaatgg ggtctaattg aatgatcact 5460

tggaaaaata tctcaattac cttcagttga attcctaaac cctgacccat ttaaaactga 5520

cacaaattcc tgcaaccatg ttacaaatac atggcatttt ctctttcggt aggactccct 5580

gagaattaat cccatagtag acaaagccaa cactgttttg gaaatatagc gaaacacaaa 5640

tgacttttgt ctgattatgt tttcgattct tttttctttt ttaaaatact agctaatatt 5700

gcaacccacc atgcaggcct gatttctaag gaatcgggta gcccttcagg ctggggaatc 5760

ctggcctgtt tctcatgggc cttggctctt ttgcacagaa cacagactgt ttacacttcc 5820

tatgttggcg tcactcggtg agcttagacg tgagtcctca aaacacttag ttctgcttga 5880

cagagaaatc ttaatggcaa aacttctctt ctatgctcta agttagccgt cctcagcttt 5940

agagattttc atcatctgac tacagccaga aaataggcac acattaccag tctggcgagg 6000

aggaaatggg tatatcactt ggacccaaag ctcagcacca gaagaaagag tttaggcaaa 6060

tatattctta ggcacaagag gcatttgtaa cacgatggtc gcagttgttt atcaaagagt 6120

aagacctggg ttggggtggg ggagatgcaa ttatatataa taattaatca ctttgataga 6180

gttctataaa gcagttcaat atcagaagcc agtttgggcc ctggacatta gaaaatgaga 6240

cagcaccatc agaataaaga ggaggcggag tgactggctc aaagcaaaag ttaaccttca 6300

ctgaaaagcg ctttcagaca cgagagactt cagaagataa tactaaacag aactaaacgc 6360

<210> 11
<211> 370
<212> PRT
<213> Mus musculus

<400> 11

Met Ala Ser Glu Leu Ala Met Asn Asn Ser Asp Leu Pro Thr Ser Pro
1               5                   10                  15

Leu Ala Met Glu Tyr Val Asn Asp Phe Asp Leu Met Lys Phe Glu Val
            20                  25                  30

Lys Lys Glu Pro Val Glu Thr Asp Arg Ile Ile Ser Gln Cys Gly Arg
        35                  40                  45

Leu Ile Ala Gly Gly Ser Leu Ser Ser Thr Pro Met Ser Thr Pro Cys
    50                  55                  60

Ser Ser Val Pro Pro Ser Pro Ser Phe Ser Ala Pro Ser Pro Gly Ser
65                  70                  75                  80

Gly Ser Glu Gln Lys Ala His Leu Glu Asp Tyr Tyr Trp Met Thr Gly
                85                  90                  95

Tyr Pro Gln Gln Leu Asn Pro Glu Ala Leu Gly Phe Ser Pro Glu Asp
            100                 105                 110

Ala Val Glu Ala Leu Ile Ser Asn Ser His Gln Leu Gln Gly Gly Phe
        115                 120                 125

Asp Gly Tyr Ala Arg Gly Ala Gln Gln Leu Ala Ala Ala Ala Gly Ala
    130                 135                 140

Gly Ala Gly Ala Ser Leu Gly Gly Ser Gly Glu Glu Met Gly Pro Ala
145                 150                 155                 160

Ala Ala Val Val Ser Ala Val Ile Ala Ala Ala Ala Ala Gln Ser Gly
                165                 170                 175

Ala Ala Pro His Tyr His His His His His His Ala Ala Gly His His
            180                 185                 190

His His Pro Thr Ala Gly Ala Pro Gly Ala Ala Gly Gly Ala Ser Ala
        195                 200                 205

Ser Ala Ser Gly Ala Gly Gly Ala Gly Gly Gly Gly Pro Ala Ser Ala
    210                 215                 220

Gly Gly Gly Gly Gly Gly Gly Gly Gly Gly Gly Thr Ala Gly Ala Gly
225                 230                 235                 240

Gly Ala Leu His Pro His His Ala Ala Gly Gly Leu His Phe Asp Asp
                245                 250                 255

Arg Phe Ser Asp Glu Gln Leu Val Thr Met Ser Val Arg Glu Leu Asn

260                 265                 270

Arg Gln Leu Arg Gly Val Ser Lys Glu Glu Val Ile Arg Leu Lys Gln
        275                 280                 285

Lys Arg Arg Thr Leu Lys Asn Arg Gly Tyr Ala Gln Ser Cys Arg Phe
    290                 295                 300

Lys Arg Val Gln Gln Arg His Val Leu Glu Ser Glu Lys Asn Gln Leu
305                 310                 315                 320

Leu Gln Gln Val Asp His Leu Lys Gln Glu Ile Ser Arg Leu Val Arg
                325                 330                 335

Glu Arg Asp Ala Tyr Lys Glu Lys Tyr Glu Lys Leu Val Ser Asn Gly
            340                 345                 350

Phe Arg Glu Asn Gly Ser Ser Ser Asp Asn Pro Ser Ser Pro Glu Phe
        355                 360                 365

Phe Met
    370

<210>  12
<211>  4316
<212>  DNA
<213>  Mus musculus

<400>  12
   gtgtgacacg cgatcccagc tagccacctt gttaaatgct ccgtgggggt cccgctcctg      60
   agtgggcttg ctagttgcag ggccaagctc gctcggccac cacctcgccc cctcccctgg     120
   cgagtttgct ccccttggcc ctgaacagac acacacacac acacacacac acacacacac     180
   acacacacac acacacacac caagcttgtg gtggtggtga tggctctttt gaattgggga     240
   gggaaagggt gtctggctca tggattgcca acctgaattc ccccgtaact tgctagcttg     300
   gttgttttgt tcttttttat tattattatt attattacaa ctactactct ttttacaccg     360
   cctgccccac acataccctg gacttgcaca agggtcaaag atctgagcag catacttcat     420
   gtagggcata gctcaccttt gaaccgggcc atcaattgcc tgtatatata ttttttcttc     480
   ttcttcttca gctcatgagc tggtgttcat tctgtgtgtg tgtgtttgtg tgttttattt     540
   tgtttggatt ttcttttttc ttcttcttct tctttgtttt tgtttattat tactattact     600
   aattttttgg agcttgctgt gtttcccagc ctctgccacc ccccaccccc acccctgcac     660
   catgttcttg agataaaagg aaaaaaaaaa aaagcagggt ttttttttcc cctccttctg     720
   agttcttctg attgagcttg caaaggaaaa aaaaatgtga aatgctatag accttgcagc     780
   gagctgagtt ccacgggggt tttatcttag cattgttatg ctaaaacaga atgaactaaa 840
   gccactcctg aacctgccac aatcaagcct acatcaacct tctgggtgtg acttgtgagt 900
   tttggcctta tgatgccaaa tctgaaagtt tagtctgcca ttaaacaaca aacaaacaaa 960
   aacacaaaaa caaacttact ctcatctcat gcattacgat gcttgctact ttgtcttagc 1020
   aacaatgaac tataacagtt tcaaagacat tatggaaaag agacattata ttaataaaag 1080
   aagcctgcat gctggacatg tatggtataa ttattttttt cctttttttt ccccccaatt 1140
   tggcttggaa atggactttt gaagacttag cgcatgacat ccatactttt gatttacagc 1200
   ctcatgactt ttttgagttc caaacaacaa cagaaaaagt acaaccccag agccttcttc 1260
   ccttgacagt ttgcttctac tcctgactgc tttgagttac aatgaacttc agcctcccgt 1320
   tgtacatgca ctgaaggcgt tccttgtcaa agatagcaga atgggctaca aatttatatg 1380
   gcaaatatta tagaacgtaa attaaatgtt ttctttttag aaacaatgat gcctgctttg 1440
   gggcacttta aattgtgcta ttgccaaaag cagtctaaat tttctttgaa aagaaagaaa 1500
   aaaaaaatct gccccaatta ttttttggtt ggttttattt ttttttattt tattttttct 1560
   attttttttt taattttttt ggtttttggt cattgtcaaa tgtggaatgc tatgggtttc 1620
   tagtatataa tttaattcta gattttataa tctgttagcc cagttaaaat gtatgctaca 1680
   gataaaggaa tgttatagat acatttgaaa gagttaggtc tgtgtagctg tagatttttt 1740
   taaagaccga tgcactaaat tgtttactgt tgtgatgtta aggggggtag agtttgcaag 1800
   gggactgttt aaaaagtagc tttctgaagc atgtgcttgc aacttaaata taagttgggt 1860
   atgtgtagtt cttgctatac catggactgt attgaaaacc aaagtattaa aaggggaaat 1920
   gcccctgttt atatctgtag gggtatttta cattcaaaat gtatgttttt ttccaaaatt 1980
   aaagtatttg ggactgaatt gcactaagat ataacctgca agcatataat acaaaaaaaa 2040
   atcgcaaaaa ctgtttagaa cgctaataaa atttatgcag ttataaaatg gcattactgc 2100
   acagttttaa agtgatgcag attttttaca gttatgttgt ggtgcagaac tggattttct 2160
   gtaacttaaa aaaatccaca gttttaaagg caataaccag taaatgttat tttcagggac 2220
   tgacatcctg tcttagaaag aaagaaagaa agaaagaaag aaagaaagaa agaaagaaag 2280
   aaagaaagaa agaaaaggaa aggaaatctt accacaataa atataagaaa tcttgtcagt 2340
   tacttttctt ttacatattt tgctgtgcaa aattgtttta taacttgagt tactaactaa 2400
   ccacgcgtga cgttctacat gcttctcttt cattttcaat tctggttata tcaagagaaa 2460
   gaataatcta caataataaa ctgcattttt tttgattctg ttccgtttct tagtgtacag 2520
   attaactggg tcccatgtct tggagcttgg atatgaaata caaaatgcat tctctttgat 2580
   ttctgaacaa gggagttgga ggcaggaatt ctattctgag aggcaggaga ttgacaccat 2640
   atagtcagta acaatattta atattaatac aaattatgac atcttatttt ttctcctcac 2700
   agatcttagt ttcctaatag gctgagctag taaggaaaaa aagtgagaaa gagggacagt 2760
   gagagagaaa gatagagaga tagacagaca gacagacaga cagacagaga cacacacaca 2820
   cacacacaca cacacacaca cacacacaca ctgagagcac ataggagctt tctgaacagg 2880
   actctaagct tctgacgaag gagcacagat agagggactg ggaccagcaa gtctggctca 2940
   ccagaggtgc atgggaacat atgcatttaa tggtaaagat aaataattct tgccgtttgg 3000
   ccaaaattga ttattttttc ttcttccctt acatgttgtg attttcttgt tggcacagtc 3060
   ctgacaagac taaacataaa cactttgcac aaatcaaagt tgtttgattt tgctcttttt 3120
   tccccctgtc attttttttt aaacttttca agtcacttgt cctaaatggg aaaaagaaag 3180
   tctatcattt gcacacagag tttccagttc acaccaggtt ttatctatat tatctaacta 3240
   tatttcgaat agtcaaagag ctgtctccaa gcatataagc tagggataac tacatgagtc 3300
   ccccaaatat aaatatacaa agaaaacaac attttcatgg ggtgtaggcg gtgctctgat 3360
   atattgctgt ttgcccccaa agcaggttat gaatggggtc taattgaatg atcacttgga 3420
   aaaatatctc aattaccttc agttgaattc ctaaaccctg acccatttaa aactgacaca 3480
   aattcctgca accatgttac aaatacatgg cattttctct ttcggtagga ctccctgaga 3540
   attaatccca tagtagacaa agccaacact gttttggaaa tatagcgaaa cacaaatgac 3600
   ttttgtctga ttatgttttc gattcttttt tcttttttaa aatactagct aatattgcaa 3660
   cccaccatgc aggcctgatt tctaaggaat cgggtagccc ttcaggctgg ggaatcctgg 3720
   cctgtttctc atgggccttg gctcttttgc acagaacaca gactgtttac acttcctatg 3780
   ttggcgtcac tcggtgagct tagacgtgag tcctcaaaac acttagttct gcttgacaga 3840
   gaaatcttaa tggcaaaact tctcttctat gctctaagtt agccgtcctc agctttagag 3900
   attttcatca tctgactaca gccagaaaat aggcacacat taccagtctg gcgaggagga 3960
   aatgggtata tcacttggac ccaaagctca gcaccagaag aaagagttta ggcaaatata 4020
   ttcttaggca caagaggcat ttgtaacacg atggtcgcag ttgtttatca aagagtaaga 4080
   cctgggttgg ggtgggggag atgcaattat atataataat taatcacttt gatagagttc 4140
   tataaagcag ttcaatatca gaagccagtt tgggccctgg acattagaaa atgagacagc 4200
   accatcagaa taaagaggag gcggagtgac tggctcaaag caaaagttaa ccttcactga 4260
   aaagcgcttt cagacacgag agacttcaga agataatact aaacagaact aaacgc 4316

<210> 13
<211> 931
<212> DNA
<213> Mus musculus

<400> 13
ggagctgtag ctcgggctgc tcgtccgctc ggcctctccc cccaaccctt ttgctctcgg 60

ctttgccttg gcccaggact tgcaattttg ctttaaaaaa ataataagaa agaaaaaaag 120

aaaagaaaag aaaaaccaaa accaaaagaa aaaaagtggg ggaagaaaga acttaactcc 180

cccctccctt gcctccagcc ggcctgcacc tcggtcttgc actttgcacg gagggagagg 240

gtgagagaga gagagagaga gagagagagc gcgcgcacgc gagggatccg gagagagaaa 300

agaaaataat aatagtaacc ataagagcca ggcagaagag gagagaagcg tgaagtgtta 360

accactccgg tgtggaggcc ggcgcctccg ggacagccac ccgccgcgcc acacagcccc 420

ggagcagcct ccgcgcgcgc cccgcctgca gcccggccgg cttaggcgag ccctccttat 480

gcaaagcgcg cagcggagcg gcgagccggg gacgccgcgc accgggccgt gctcctccgg 540

catcctctct gccgctgtca tcaccaccgt caccgccagc cgcggaggac cttcccgagc 600

ctgaagcgcc cgggatcgga gctcagggag gcgagcaggc gaggaggggg cccggggcga 660

gcggaggaga gagagcgaga gagcaagaga gagaaagaga gagagcacat tggcgtgagc 720

agggggaggg agggcgggcg cggggggctg cgggcagggc ggggggtgtg tgcacgttcg 780

agctttcggg ccacagccac cgccgcgcaa gctagaagcg ccctagcccg gcaagctggc 840

tcacccgctg gccacccagc acagcccgct ggcccctctc ctgcagccca tctggcggag 900

cggcggcggc ggtggcggcg gaggcaggag g 931

<210> 14
<211> 6360
<212> DNA
<213> Mus musculus

<400> 14
   ggagctgtag ctcgggctgc tcgtccgctc ggcctctccc cccaaccctt ttgctctcgg 60
   ctttgccttg gcccaggact tgcaattttg ctttaaaaaa ataataagaa agaaaaaaag 120
   aaaagaaaag aaaaaccaaa accaaaagaa aaaaagtggg ggaagaaaga acttaactcc 180
   cccctccctt gcctccagcc ggcctgcacc tcggtcttgc actttgcacg gagggagagg 240
   gtgagagaga gagagagaga gagagagagc gcgcgcacgc gagggatccg gagagagaaa 300
   agaaaataat aatagtaacc ataagagcca ggcagaagag gagagaagcg tgaagtgtta 360
   accactccgg tgtggaggcc ggcgcctccg ggacagccac ccgccgcgcc acacagcccc 420
   ggagcagcct ccgcgcgcgc cccgcctgca gcccggccgg cttaggcgag ccctccttat 480
   gcaaagcgcg cagcggagcg gcgagccggg gacgccgcgc accgggccgt gctcctccgg 540
   catcctctct gccgctgtca tcaccaccgt caccgccagc cgcggaggac cttcccgagc 600
   ctgaagcgcc cgggatcgga gctcagggag gcgagcaggc gaggaggggg cccggggcga 660
   gcggaggaga gagagcgaga gagcaagaga gagaaagaga gagagcacat tggcgtgagc 720
   agggggaggg agggcgggcg cggggggctg cgggcagggc ggggggtgtg tgcacgttcg 780
   agctttcggg ccacagccac cgccgcgcaa gctagaagcg ccctagcccg gcaagctggc 840
   tcacccgctg gccacccagc acagcccgct ggcccctctc ctgcagccca tctggcggag 900
   cggcggcggc ggtggcggcg gaggcaggag gatggcttca gaactggcaa tgaacaattc 960
   cgacctgccc accagtcccc tggccatgga atatgttaat gacttcgatc tgatgaagtt 1020
   tgaagtgaaa aaggaaccgg tggagaccga ccgcatcatc agccagtgcg gccgtctcat 1080
   cgccgggggc tcgctgtcct ccacccccat gagcacgccc tgcagctcgg tgcccccgtc 1140
   ccccagcttc tcggcgccca gcccgggctc gggcagcgaa cagaaggcgc acctggaaga 1200
   ctactactgg atgaccggct acccgcagca gctcaacccg gaggcgctgg gcttcagccc 1260
   ggaggacgcg gtcgaggcgc tcatcagcaa cagccaccag ctccagggtg gcttcgatgg 1320
   ctatgcgcgg ggagcgcagc agctggccgc ggcagcgggg gccggcgccg gcgcctccct 1380
   gggcggcagc ggcgaggaga tgggccccgc cgccgccgtg gtgtccgccg tgatcgccgc 1440
   ggccgccgcg cagagcggcg cggcacccca ctaccatcac caccaccacc acgccgcggg 1500
   gcaccaccac catccgacgg ccggcgcccc gggagccgcg ggcggcgcgt ctgcctctgc 1560
   gagcggcgcg ggtggcgcgg gcggcggtgg cccggccagc gccgggggcg gcggcggcgg 1620
   aggcggcggc gggggcacgg cgggggcggg gggcgccctg cacccgcacc atgccgcggg 1680
   cggcctgcac ttcgacgacc gcttctcgga cgagcagttg gtgaccatgt cggtgcgcga 1740
   gctgaaccgg cagctgcgcg gggtcagcaa ggaggaggtg atccgactga agcagaagag 1800
   gcggaccctg aaaaaccgcg gctatgccca gtcctgccgc ttcaagaggg tgcagcagag 1860
   acacgtcctg gagtcggaga agaaccagct gctgcagcag gtagaccacc tcaagcagga 1920
   gatctccagg ctggtgcgcg aaagggacgc ctacaaggag aaatacgaga agctggtgag 1980
   caacggcttc cgagaaaacg gctcgagcag cgacaaccct tcctctcccg aatttttcat 2040
   gtgagtgtga cacgcgatcc cagctagcca ccttgttaaa tgctccgtgg gggtcccgct 2100
   cctgagtggg cttgctagtt gcagggccaa gctcgctcgg ccaccacctc gccccctccc 2160
   ctggcgagtt tgctcccctt ggccctgaac agacacacac acacacacac acacacacac 2220
   acacacacac acacacacac acaccaagct tgtggtggtg gtgatggctc ttttgaattg 2280
   gggagggaaa gggtgtctgg ctcatggatt gccaacctga attcccccgt aacttgctag 2340
   cttggttgtt ttgttctttt ttattattat tattattatt acaactacta ctctttttac 2400
   accgcctgcc ccacacatac cctggacttg cacaagggtc aaagatctga gcagcatact 2460
   tcatgtaggg catagctcac ctttgaaccg ggccatcaat tgcctgtata tatatttttt 2520
   cttcttcttc ttcagctcat gagctggtgt tcattctgtg tgtgtgtgtt tgtgtgtttt 2580
   attttgtttg gattttcttt tttcttcttc ttcttctttg tttttgttta ttattactat 2640
   tactaatttt ttggagcttg ctgtgtttcc cagcctctgc caccccccac ccccacccct 2700
   gcaccatgtt cttgagataa aaggaaaaaa aaaaaaagca gggttttttt ttcccctcct 2760
   tctgagttct tctgattgag cttgcaaagg aaaaaaaaat gtgaaatgct atagaccttg 2820
   cagcgagctg agttccacgg gggttttatc ttagcattgt tatgctaaaa cagaatgaac 2880
   taaagccact cctgaacctg ccacaatcaa gcctacatca accttctggg tgtgacttgt 2940
   gagttttggc cttatgatgc caaatctgaa agtttagtct gccattaaac aacaaacaaa 3000
   caaaaacaca aaaacaaact tactctcatc tcatgcatta cgatgcttgc tactttgtct 3060
   tagcaacaat gaactataac agtttcaaag acattatgga aaagagacat tatattaata 3120
   aaagaagcct gcatgctgga catgtatggt ataattattt ttttcctttt ttttcccccc 3180
   aatttggctt ggaaatggac ttttgaagac ttagcgcatg acatccatac ttttgattta 3240
   cagcctcatg acttttttga gttccaaaca acaacagaaa aagtacaacc ccagagcctt 3300
   cttcccttga cagtttgctt ctactcctga ctgctttgag ttacaatgaa cttcagcctc 3360
   ccgttgtaca tgcactgaag gcgttccttg tcaaagatag cagaatgggc tacaaattta 3420
   tatggcaaat attatagaac gtaaattaaa tgtttctttt ttagaaacaa tgatgcctgc 3480
   tttggggcac tttaaattgt gctattgcca aaagcagtct aaatttcttt tgaaaagaaa 3540
   gaaaaaaaaa atctgcccca attatttttt ggttggtttt attttttttt attttatttt 3600
   ttctattttt tttttaattt ttttggtttt tggtcattgt caaatgtgga atgctatggg 3660
   tttctagtat ataatttaat tctagatttt ataatctgtt agcccagtta aaatgtatgc 3720
   tacagataaa ggaatgttat agatacattt gaaagagtta ggtctgtgta gctgtagatt 3780
   tttttaaaga ccgatgcact aaattgttta ctgttgtgat gttaaggggg gtagagtttg 3840
   caaggggact gtttaaaaag tagctttctg aagcatgtgc ttgcaactta aatataagtt 3900
   gggtatgtgt agttcttgct ataccatgga ctgtattgaa aaccaaagta ttaaaagggg 3960
   aaatgcccct gtttatatct gtaggggtat tttacattca aaatgtatgt ttttttccaa 4020
   aattaaagta tttgggactg aattgcacta agatataacc tgcaagcata taatacaaaa 4080
   aaaaatcgca aaaactgttt agaacgctaa taaaatttat gcagttataa aatggcatta 4140
   ctgcacagtt ttaaagtgat gcagattttt tacagttatg ttgtggtgca gaactggatt 4200
   ttctgtaact taaaaaaatc cacagtttta aaggcaataa ccagtaaatg ttattttcag 4260
   ggactgacat cctgtcttag aaagaaagaa agaaagaaag aaagaaagaa agaaagaaag 4320
   aaagaaagaa agaaagaaaa ggaaaggaaa tcttaccaca ataaatataa gaaatcttgt 4380
   cagttacttt tcttttacat attttgctgt gcaaaattgt tttataactt gagttactaa 4440
   ctaaccacgc gtgacgttct acatgcttct ctttcatttt caattctggt tatatcaaga 4500
   gaaagaataa tctacaataa taaactgcat tttttttgat tctgttccgt ttcttagtgt 4560
   acagattaac tgggtcccat gtcttggagc ttggatatga aatacaaaat gcattctctt 4620
   tgatttctga acaagggagt tggaggcagg aattctattc tgagaggcag gagattgaca 4680
   ccatatagtc agtaacaata tttaatatta atacaaatta tgacatctta ttttttctcc 4740
   tcacagatct tagtttccta ataggctgag ctagtaagga aaaaaagtga gaaagaggga 4800
   cagtgagaga gaaagataga gagatagaca gacagacaga cagacagaca gagacacaca 4860
   cacacacaca cacacacaca cacacacaca cacactgaga gcacatagga gctttctgaa 4920
   caggactcta agcttctgac gaaggagcac agatagaggg actgggacca gcaagtctgg 4980
   ctcaccagag gtgcatggga acatatgcat ttaatggtaa agataaataa ttcttgccgt 5040
   ttggccaaaa ttgattattt tttcttcttc ccttacatgt tgtgattttc ttgttggcac 5100
   agtcctgaca agactaaaca taaacacttt gcacaaatca aagttgtttg attttgctct 5160
   tttttccccc tgtcattttt ttttaaactt ttcaagtcac ttgtcctaaa tgggaaaaag 5220
   aaagtctatc atttgcacac agagtttcca gttcacacca ggttttatct atattatcta 5280
   actatatttc gaatagtcaa agagctgtct ccaagcatat aagctaggga taactacatg 5340
   agtcccccaa atataaatat acaaagaaaa caacattttc atggggtgta ggcggtgctc 5400
   tgatatattg ctgtttgccc ccaaagcagg ttatgaatgg ggtctaattg aatgatcact 5460
   tggaaaaata tctcaattac cttcagttga attcctaaac cctgacccat ttaaaactga 5520
   cacaaattcc tgcaaccatg ttacaaatac atggcatttt ctctttcggt aggactccct 5580
   gagaattaat cccatagtag acaaagccaa cactgttttg gaaatatagc gaaacacaaa 5640
   tgacttttgt ctgattatgt tttcgattct tttttctttt ttaaaatact agctaatatt 5700
   gcaacccacc atgcaggcct gatttctaag gaatcgggta gcccttcagg ctggggaatc 5760
   ctggcctgtt tctcatgggc cttggctctt ttgcacagaa cacagactgt ttacacttcc 5820
   tatgttggcg tcactcggtg agcttagacg tgagtcctca aaacacttag ttctgcttga 5880
   cagagaaatc ttaatggcaa aacttctctt ctatgctcta agttagccgt cctcagcttt 5940
   agagattttc atcatctgac tacagccaga aaataggcac acattaccag tctggcgagg 6000
   aggaaatggg tatatcactt ggacccaaag ctcagcacca gaagaaagag tttaggcaaa 6060
   tatattctta ggcacaagag gcatttgtaa cacgatggtc gcagttgttt atcaaagagt 6120
   aagacctggg ttggggtggg ggagatgcaa ttatatataa taattaatca ctttgataga 6180
   gttctataaa gcagttcaat atcagaagcc agtttgggcc ctggacatta gaaaatgaga 6240
   cagcaccatc agaataaaga ggaggcggag tgactggctc aaagcaaaag ttaaccttca 6300
   ctgaaaagcg ctttcagaca cgagagactt cagaagataa tactaaacag aactaaacgc 6360

<210> 15

<211>  7623
<212>  DNA
<213>  Unknown

<220>
<223>  Artifical sequence

<400>  15
tgaaagaccc cacctgtagg tttggcaagc tagcttaagt aacgccattt tgcaaggcat      60

ggaaaataca taactgagaa tagagaagtt cagatcaagg ttaggaacag agagacagca     120

gaatatgggc caaacaggat atctgtggta agcagttcct gccccggctc agggccaaga     180

acagatggtc cccagatgcg gtcccgccct cagcagtttc tagagaacca tcagatgttt     240

ccagggtgcc ccaaggacct gaaatgaccc tgtgccttat ttgaactaac caatcagttc     300

gcttctcgct tctgttcgcg cgcttctgct ccccgagctc aataaaagag cccacaaccc     360

ctcactcggc gcgccagtcc tccgatagac tgcgtcgccc gggtacccgt attcccaata     420

aagcctcttg ctgtttgcat ccgaatcgtg gactcgctga tccttgggag ggtctcctca     480

gattgattga ctgcccacct cgggggtctt tcatttggag gttccaccga gatttggaga     540

cccctgccca gggaccaccg acccccccgc cgggaggtaa gctggccagc ggtcgtttcg     600

tgtctgtctc tgtctttgtg cgtgtttgtg ccggcatcta atgtttgcgc ctgcgtctgt     660

actagttagc taactagctc tgtatctggc ggacccgtgg tggaactgac gagttctgaa     720

cacccggccg caaccctggg agacgtccca gggactttgg gggccgtttt tgtggcccga     780

cctgaggaag ggagtcgatg tggaatccga ccccgtcagg atatgtggtt ctggtaggag     840

acgagaacct aaaacagttc ccgcctccgt ctgaattttt gctttcggtt tggaaccgaa     900

gccgcgcgtc ttgtctgctg cagcgctgca gcatcgttct gtgttgtctc tgtctgactg     960

tgtttctgta tttgtctgaa aattagggcc agactgttac cactccctta agtttgacct    1020

taggtcactg gaaagatgtc gagcggatcg ctcacaacca gtcggtagat gtcaagaaga    1080

gacgttgggt taccttctgc tctgcagaat ggccaacctt taacgtcgga tggccgcgag    1140

acggcacctt taaccgagac ctcatcaccc aggttaagat caaggtcttt tcacctggcc    1200

cgcatggaca cccagaccag gtcccctaca tcgtgacctg ggaagccttg gcttttgacc    1260

cccctccctg ggtcaagccc tttgtacacc ctaagcctcc gcctcctctt cctccatccg    1320

ccccgtctct cccccttgaa cctcctcgtt cgaccccgcc tcgatcctcc ctttatccag    1380

ccctcactcc ttctctaggc gccggaattc gtttaaacgc caccatggac tacaaagacc    1440

atgacggtga ttataaagat catgacatcg attacaagga tgacgatgac aagcttgctc    1500

gacaagcttc agaactggca atgaacaatt ccgacctgcc caccagtccc ctggccatgg    1560

aatatgttaa tgacttcgat ctgatgaagt ttgaagtgaa aaaggaaccg gtggagaccg    1620

accgcatcat cagccagtgc ggccgtctca tcgccggggg ctcgctgtcc tccaccccca    1680

tgagcacgcc ctgcagctcg gtgcccccgt cccccagctt ctcggcgccc agcccgggct 1740

cgggcagcga acagaaggcg cacctggaag actactactg gatgaccggc tacccgcagc 1800

agctcaaccc ggaggcgctg ggcttcagcc cggaggacgc ggtcgaggcg ctcatcagca 1860

acagccacca gctccagggt ggcttcgatg gctatgcgcg gggagcgcag cagctggccg 1920

cggcagcggg ggccggcgcc ggcgcctccc tgggcggcag cggcgaggag atgggccccg 1980

ccgccgccgt ggtgtccgcc gtgatcgccg cggccgccgc gcagagcggc gcggcacccc 2040

actaccatca ccaccaccac cacgccgcgg ggcaccacca ccatccgacg gccggcgccc 2100

cgggagccgc gggcggcgcg tctgcctctg cgagcggcgc gggtggcgcg ggcggcggtg 2160

gcccggccag cgccgggggc ggcggcggcg gaggcggcgg cgggggcacg gcgggggcgg 2220

ggggcgccct gcacccgcac catgccgcgg gcggcctgca cttcgacgac cgcttctcgg 2280

acgagcagtt ggtgaccatg tcggtgcgcg agctgaaccg gcagctgcgc ggggtcagca 2340

aggaggaggt gatccgactg aagcagaaga ggcggaccct gaaaaaccgc ggctatgccc 2400

agtcctgccg cttcaagagg gtgcagcaga gacacgtcct ggagtcggag aagaaccagc 2460

tgctgcagca ggtagaccac ctcaagcagg agatctccag gctggtgcgc gaaaggacgc 2520

cctacaagga gaaatacgag aagctggtga gcaacggctt ccgagaaaac ggctcgagca 2580

gcgacaaccc ttcctctccc gaatttttca tgtgatctag aggatccgcc cctctccctc 2640

ccccccccct aacgttactg gccgaagccg cttggaataa ggccggtgtg cgtttgtcta 2700

tatgttattt tccaccatat tgccgtcttt tggcaatgtg agggcccgga aacctggccc 2760

tgtcttcttg acgagcattc ctaggggtct ttcccctctc gccaaaggaa tgcaaggtct 2820

gttgaatgtc gtgaaggaag cagttcctct ggaagcttct tgaagacaaa caacgtctgt 2880

agcgaccctt tgcaggcagc ggaacccccc acctggcgac aggtgcctct gcggccaaaa 2940

gccacgtgta taagatacac ctgcaaaggc ggcacaaccc cagtgccacg ttgtgagttg 3000

gatagttgtg gaaagagtca aatggctctc ctcaagcgta ttcaacaagg ggctgaagga 3060

tgcccagaag gtaccccatt gtatgggatc tgatctgggg cctcggtgca catgctttac 3120

atgtgtttag tcgaggttaa aaaaacgtct aggccccccg aaccacgggg acgtggtttt 3180

cctttgaaaa acacgatgat aatatggcca caaccatggt gagcaagggc gaggagctgt 3240

tcaccggggt ggtgcccatc ctggtcgagc tggacggcga cgtaaacggc cacaagttca 3300

gcgtgtccgg cgagggcgag ggcgatgcca cctacggcaa gctgaccctg aagttcatct 3360

gcaccaccgg caagctgccc gtgccctggc ccaccctcgt gaccaccctg acctacggcg 3420

tgcagtgctt cagccgctac cccgaccaca tgaagcagca cgacttcttc aagtccgcca 3480

tgcccgaagg ctacgtccag gagcgcacca tcttcttcaa ggacgacggc aactacaaga 3540

cccgcgccga ggtgaagttc gagggcgaca ccctggtgaa ccgcatcgag ctgaagggca 3600

tcgacttcaa ggaggacggc aacatcctgg ggcacaagct ggagtacaac tacaacagcc 3660

acaacgtcta tatcatggcc gacaagcaga agaacggcat caaggtgaac ttcaagatcc 3720

gccacaacat cgaggacggc agcgtgcagc tcgccgacca ctaccagcag aacaccccca 3780

tcggcgacgg ccccgtgctg ctgcccgaca accactacct gagcacccag tccgccctga 3840

gcaaagaccc caacgagaag cgcgatcaca tggtcctgct ggagttcgtg accgccgccg 3900

ggatcactct cggcatggac gagctgtaca agtaaagcgg ccataaaata aaagatttta 3960

tttagtctcc agaaaaaggg gggaatgaaa gaccccacct gtaggtttgg caagctagct 4020

taagtaacgc cattttgcaa ggcatggaaa atacataact gagaatagag aagttcagat 4080

caaggttagg aacagagaga cagcagaata tgggccaaac aggatatctg tggtaagcag 4140

ttcctgcccc ggctcagggc caagaacaga tggtccccag atgcggtccc gccctcagca 4200

gtttctagag aaccatcaga tgtttccagg gtgccccaag gacctgaaat gaccctgtgc 4260

cttatttgaa ctaaccaatc agttcgcttc tcgcttctgt tcgcgcgctt ctgctccccg 4320

agctcaataa aagagcccac aacccctcac tcggcgcgcc agtcctccga tagactgcgt 4380

cgcccgggta cccgtgtatc caataaaccc tcttgcagtt gcatccgact tgtggtctcg 4440

ctgttccttg ggagggtctc ctctgagtga ttgactaccc gtcagcgggg gtctttcatg 4500

ggtaacagtt tcttgaagtt ggagaacaac attctgaggg taggagtcga atattaagta 4560

atcctgactc aattagccac tgttttgaat ccacatactc caatactcct gaaatagttc 4620

attatggaca gcgcagaaga gctggggaga attaattcgt aatcatggtc atagctgttt 4680

cctgtgtgaa attgttatcc gctcacaatt ccacacaaca tacgagccgg aagcataaag 4740

tgtaaagcct ggggtgccta atgagtgagc taactcacat taattgcgtt gcgctcactg 4800

cccgctttcc agtcgggaaa cctgtcgtgc cagctgcatt aatgaatcgg ccaacgcgcg 4860

gggagaggcg gtttgcgtat tgggcgctct tccgcttcct cgctcactga ctcgctgcgc 4920

tcggtcgttc ggctgcggcg agcggtatca gctcactcaa aggcggtaat acggttatcc 4980

acagaatcag gggataacgc aggaaagaac atgtgagcaa aaggccagca aaaggccagg 5040

aaccgtaaaa aggccgcgtt gctggcgttt ttccataggc tccgcccccc tgacgagcat 5100

cacaaaaatc gacgctcaag tcagaggtgg cgaaacccga caggactata aagataccag 5160

gcgtttcccc ctggaagctc cctcgtgcgc tctcctgttc cgaccctgcc gcttaccgga 5220

tacctgtccg cctttctccc ttcgggaagc gtggcgcttt ctcatagctc acgctgtagg 5280

tatctcagtt cggtgtaggt cgttcgctcc aagctgggct gtgtgcacga accccccgtt 5340

cagcccgacc gctgcgcctt atccggtaac tatcgtcttg agtccaaccc ggtaagacac 5400

gacttatcgc cactggcagc agccactggt aacaggatta gcagagcgag gtatgtaggc 5460

ggtgctacag agttcttgaa gtggtggcct aactacggct acactagaag gacagtattt 5520

ggtatctgcg ctctgctgaa gccagttacc ttcggaaaaa gagttggtag ctcttgatcc 5580

ggcaaacaaa ccaccgctgg tagcggtggt ttttttgttt gcaagcagca gattacgcgc 5640

agaaaaaaag gatctcaaga agatcctttg atcttttcta cggggtctga cgctcagtgg 5700

aacgaaaact cacgttaagg gattttggtc atgagattat caaaaaggat cttcacctag 5760

atccttttaa attaaaaatg aagttttaaa tcaatctaaa gtatatatga gtaaacttgg 5820

tctgacagtt accaatgctt aatcagtgag gcacctatct cagcgatctg tctatttcgt 5880

tcatccatag ttgcctgact ccccgtcgtg tagataacta cgatacggga gggcttacca 5940

tctggcccca gtgctgcaat gataccgcga gacccacgct caccggctcc agatttatca 6000

gcaataaacc agccagccgg aagggccgag cgcagaagtg gtcctgcaac tttatccgcc 6060

tccatccagt ctattaattg ttgccgggaa gctagagtaa gtagttcgcc agttaatagt 6120

ttgcgcaacg ttgttgccat tgctacaggc atcgtggtgt cacgctcgtc gtttggtatg 6180

gcttcattca gctccggttc ccaacgatca aggcgagtta catgatcccc catgttgtgc 6240

aaaaaagcgg ttagctcctt cggtcctccg atcgttgtca gaagtaagtt ggccgcagtg 6300

ttatcactca tggttatggc agcactgcat aattctctta ctgtcatgcc atccgtaaga 6360

tgcttttctg tgactggtga gtactcaacc aagtcattct gagaatagtg tatgcggcga 6420

ccgagttgct cttgcccggc gtcaatacgg gataataccg cgccacatag cagaacttta 6480

aaagtgctca tcattggaaa acgttcttcg gggcgaaaac tctcaaggat cttaccgctg 6540

ttgagatcca gttcgatgta acccactcgt gcacccaact gatcttcagc atcttttact 6600

ttcaccagcg tttctgggtg agcaaaaaca ggaaggcaaa atgccgcaaa aaagggaata 6660

agggcgacac ggaaatgttg aatactcata ctcttccttt ttcaatatta ttgaagcatt 6720

tatcagggtt attgtctcat gagcggatac atatttgaat gtatttagaa aaataaacaa 6780

ataggggttc cgcgcacatt tccccgaaaa gtgccacctg acgtctaaga aaccattatt 6840

atcatgacat taacctataa aaataggcgt atcacgaggc cctttcgtct cgcgcgtttc 6900

ggtgatgacg gtgaaaacct ctgacacatg cagctcccgg agacggtcac agcttgtctg 6960

taagcggatg ccgggagcag acaagcccgt cagggcgcgt cagcgggtgt tggcgggtgt 7020

cggggctggc ttaactatgc ggcatcagag cagattgtac tgagagtgca ccatatgcgg 7080

tgtgaaatac cgcacagatg cgtaaggaga aaataccgca tcaggcgcca ttcgccattc 7140

aggctgcgca actgttggga agggcgatcg gtgcgggcct cttcgctatt acgccagctg 7200

gcgaaagggg gatgtgctgc aaggcgatta agttgggtaa cgccagggtt ttcccagtca 7260

cgacgttgta aaacgacggc gcaaggaatg gtgcatgcaa ggagatggcg cccaacagtc 7320

ccccggccac ggggcctgcc accataccca cgccgaaaca agcgctcatg agcccgaagt 7380

ggcgagcccg atcttcccca tcggtgatgt cggcgatata ggcgccagca accgcacctg 7440

tggcgccggt gatgccggcc acgatgcgtc cggcgtagag gcgattagtc caatttgtta 7500

aagacaggat atcagtggtc caggctctag ttttgactca acaatatcac cagctgaagc 7560

ctatagagta cgagccatag ataaaataaa agattttatt tagtctccag aaaaaggggg 7620

gaa 7623

<210> 16
<211> 10296
<212> DNA
<213> Unknown

<220>
<223> Artificial sequence

<400> 16
cataatgaaa gaccccacct gtaggtttgg caagctagct taagtaacgc cattttgcaa 60

ggcatggaaa aatacataac tgagaataga aaagttcaga tcaaggtcag gaacagatgg 120

aacagctgaa tatgggccaa acaggatatc tgtggtaagc agttcctgcc ccggctcagg 180

gccaagaaca gatggaacag ctgaatatgg gccaaacagg atatctgtgg taagcagttc 240

ctgccccggc tcagggccaa gaacagatgg tccccagatg cggtccagcc ctcagcagtt 300

tctagagaac catcagatgt ttccagggtg ccccaaggac ctgaaatgac cctgtgcctt 360

atttgaacta accaatcagt tcgcttctcg cttctgttcg cgcgcttctg ctccccgagc 420

tcaataaaag agcccacaac ccctcactcg gggcgccagt cctccgattg actgagtcgc 480

ccgggtaccc gtgtatccaa taaaccctct tgcagttgca tccgacttgt ggtctcgctg 540

ttccttggga gggtctcctc tgagtgattg actacccgtc agcgggggtc tttcatttgg 600

gggctcgtcc gggatcggga gacccctgcc cagggaccac cgacccacca ccgggaggta 660

agctggccag caacttatct gtgtctgtcc gattgtctag tgtctatgac tgattttatg 720

cgcctgcgtc ggtactagtt agctaactag ctctgtatct ggcggacccg tggtggaact 780

gacgagttcg gaacacccgg ccgcaaccct gggagacgtc ccagggatct cgggggccgt 840

ttttgtggcc cgacctgagt ccaaaaatcc cgatcgtttt ggactctttg gtgcaccccc 900

cttagaggag ggatatgtgg ttctggtagg agacgagaac ctaaaacagt tcccgcctcc 960

gtctgaattt ttgctttcgg tttgggaccg aagccgcgcc gcgcgtcttg tctgctgcag 1020

catcgttctg tgttgtctct gtctgactgt gtttctgtat ttgtctgaaa atatgggccc 1080

gggccagact gttaccactc ccttaagttt gaccttaggt cactggaaag atgtcgagcg 1140

gatcgctcac aaccagtcgg tagatgtcaa gaagagacgt tgggttacct tctgctctgc 1200

agaatggcca acctttaacg tcggatggcc gcgagacggc acctttaacc gagacctcat 1260

cacccaggtt aagatcaagg tcttttcacc tggcccgcat ggacacccag accaggtccc 1320

ctacatcgtg acctgggaag ccttggcttt tgacccccct ccctgggtca agccctttgt 1380

acaccctaag cctccgcctc ctcttcctcc atccgccccg tctctccccc ttgaacctcc 1440

tcgttcgacc ccgcctcgat cctcccttta tccagccctc actccttctc taggcgcccc 1500

catatggcca tatgagatct tatatggggc acccccgccc cttgtaaact tccctgaccc 1560

tgacatgaca agagttacta acagcccctc tctccaagct cacttacagg ctctctactt 1620

agtccagcac gaagtctgga gacctctggc ggcagcctac caagaacaac tggaccgacc 1680

ggtggtacct cacccttacc gagtcggcga cacagtgtgg gtccgccgac accagactaa 1740

gaacctagaa cctcgctgga aaggacctta cacagtcctg ctgaccaccc ccaccgccct 1800

caaagtagac ggcatcgcag cttggataca cgccgcccac gtgaaggctg ccgaccccgg 1860

gggtggacca tcctctagac tgccggatcc cagtgtggtg gtacgggaat tctaatgccc 1920

aagaagaaga ggaaggtgtc caatttactg accgtacacc aaaatttgcc tgcattaccg 1980

gtcgatgcaa cgagtgatga ggttcgcaag aacctgatgg acatgttcag ggatcgccag 2040

gcgttttctg agcatacctg gaaaatgctt ctgtccgttt gccggtcgtg ggcggcatgg 2100

tgcaagttga ataaccggaa atggtttccc gcagaacctg aagatgttcg cgattatctt 2160

ctatatcttc aggcgcgcgg tctggcagta aaaactatcc agcaacattt gggccagcta 2220

aacatgcttc atcgtcggtc cgggctgcca cgaccaagtg acagcaatgc tgtttcactg 2280

gttatgcggc ggatctgacg ggctggattc ccaatagctc ttttttaaaa tcttggaaac 2340

tttgtccttc gctgaattac gacactgtcc acctttaatt tcctcgaaaa ctccaataac 2400

tctgctgaag ccatgccttg tgttcaggcg cagtatgggt cctcgcctca aggagccagc 2460

cccgcttctc agagctacag ttaccactct tcgggagaat acagctccga tttcttaact 2520

ccagagtttg tcaagtttag catggacctc accaacactg aaattactgc caccacttct 2580

ctccccagct tcagtacctt tatggacaac tacagcacag gctacgacgt caagccacct 2640

tgcttgtacc aaatgcccct gtccggacag cagtcctcca ttaaggtaga agacattcag 2700

atgcacaact accagcaaca cagccacctg ccccctcagt ccgaggagat gatgccacac 2760

agcgggtcgg tttactacaa gccctcttcg cccccgacac ccagcacccc gagcttccag 2820

gtgcagcata gcccgatgtg ggacgatccg ggctcccttc acaacttcca ccagaactac 2880

gtggccacta cgcatatgat cgagcagagg aagacacctg tctcccgcct gtcactcttc 2940

tcctttaagc agtcgccccc gggcactcct gtgtctagct gccagatgcg cttcgacggg 3000

cctctgcacg tccccatgaa cccggagccc gcgggcagcc accacgtagt ggatgggcag 3060

accttcgccg tgcccaaccc cattcgcaag ccggcatcca tgggcttccc gggcctgcag 3120

atcggccacg catcgcagtt gcttgacacg caggtgccct cgccgccgtc ccggggctct 3180

ccctccaatg agggtctgtg cgctgtttgc ggtgacaacg cggcctgtca gcactacggt 3240

gttcgcactt gtgagggctg caaaggtttc tttaagcgca cggtgcaaaa aaacgcgaaa 3300

tatgtgtgtt tagcaaataa aaactgccca gtggacaagc gccgccgaaa ccgttgtcag 3360

tactgtcggt ttcagaagtg cctagctgtt gggatggtta aagaagtggt tcgcacggac 3420

agtttaaaag gccggagagg tcgtttaccc tcgaagccga agagcccaca ggatccctct 3480

cccccctcac ctccggtgag tctgatcagt gccctcgtca gagcccacgt cgattccaat 3540

ccggcaatga ccagcctgga ctattccagg ttccaggcaa accctgacta tcagatgagt 3600

ggagatgata cccaacatat ccagcagttc tacgatctcc tgaccggctc tatggagatc 3660

atcagagggt gggcagagaa gatccctggc tttgctgacc tgcccaaagc cgaccaggac 3720

ctgctttttg aatcagcttt cttagaatta tttgttctgc gcttagcata caggtccaac 3780

ccagtggagg gtaaactcat cttttgcaat ggggtggtct tgcacaggtt gcaatgcgtg 3840

cgtggctttg gggaatggat tgattccatt gttgaattct cctccaactt gcagaatatg 3900

aacatcgaca tttctgcctt ctcctgcatt gctgccctgg ctatggtcac agagagacac 3960

gggctcaagg aacccaagag agtggaagag ctacaaaaca aaattgtaaa ttgtcttaaa 4020

gaccatgtga ctttcaataa tgggggtttg aaccgaccca actacctgtc taaactgttg 4080

gggaagctgc cagaactccg caccctttgc acacagggcc tccagcgcat tttctacctg 4140

aaattggaag acttggtacc accaccagca ataattgaca aacttttcct ggacacctta 4200

cctttctaag accttctccc aagcacgtca aagaatgacg ggctggattc ccaatagctc 4260

ttttttaaaa tcttggaaac tttgtccttc gctgaattac gacactgtcc acctttaatt 4320

tcctcgaaaa ctccaataac tctgctgaag ccatgccttg tgttcaggcg cagtatgggt 4380

cctcgcctca aggagccagc cccgcttctc agagctacag ttaccactct tcgggagaat 4440

acagctccga tttcttaact ccagagtttg tcaagtttag catggacctc accaacactg 4500

aaattactgc caccacttct ctccccagct tcagtacctt tatggacaac tacagcacag 4560

gctacgacgt caagccacct tgcttgtacc aaatgcccct gtccggacag cagtcctcca 4620

ttaaggtaga agacattcag atgcacaact accagcaaca cagccacctg ccccctcagt 4680

ccgaggagat gatgccacac agcgggtcgg tttactacaa gccctcttcg cccccgacac 4740

ccagcacccc gagcttccag gtgcagcata gcccgatgtg ggacgatccg ggctcccttc 4800

acaacttcca ccagaactac gtggccacta cgcatatgat cgagcagagg aagacacctg 4860

tctcccgcct gtcactcttc tcctttaagc agtcgccccc gggcactcct gtgtctagct 4920

gccagatgcg cttcgacggg cctctgcacg tccccatgaa cccggagccc gcgggcagcc 4980

accacgtagt ggatgggcag accttcgccg tgcccaaccc cattcgcaag ccggcatcca 5040

tgggcttccc gggcctgcag atcggccacg catcgcagtt gcttgacacg caggtgccct 5100

cgccgccgtc ccggggctct ccctccaatg agggtctgtg cgctgtttgc ggtgacaacg 5160

cggcctgtca gcactacggt gttcgcactt gtgagggctg caaaggtttc tttaagcgca 5220

cggtgcaaaa aaacgcgaaa tatgtgtgtt tagcaaataa aaactgccca gtggacaagc 5280

gccgccgaaa ccgttgtcag tactgtcggt ttcagaagtg cctagctgtt gggatggtta 5340

aagaagtggt tcgcacggac agtttaaaag gccggagagg tcgtttaccc tcgaagccga 5400

agagcccaca ggatccctct cccccctcac ctccggtgag tctgatcagt gccctcgtca 5460

gagcccacgt cgattccaat ccggcaatga ccagcctgga ctattccagg ttccaggcaa 5520

accctgacta tcagatgagt ggagatgata cccaacatat ccagcagttc tacgatctcc 5580

tgaccggctc tatggagatc atcagaggtt gggcagagaa gatccctggc tttgctgacc 5640

tgcccaaagc cgaccaggac ctgctttttg aatcagcttt cttagaatta tttgttctgc 5700

gcttagcata caggtccaac ccagtggagg gtaaactcat cttttgcaat ggggtggtct 5760

tgcacaggtt gcaatgcgtg cgtggctttg gggaatggat tgattccatt gttgaattct 5820

cctccaactt gcagaatatg aacatcgaca tttctgcctt ctcctgcatt gctgccctgg 5880

ctatggtcac agagagacac gggctcaagg aacccaagag agtggaagag ctacaaaaca 5940

aaattgtaaa ttgtcttaaa gaccatgtga ctttcaataa tgggggtttg aaccgaccca 6000

actacctgtc taaactgttg gggaagctgc cagaactccg caccctttgc acacagggcc 6060

tccagcgcat tttctacctg aaattggaag acttggtacc accaccagca ataattgaca 6120

aacttttcct ggacacctta cctttctaag accttctccc aagcacgtca aagaagcggc 6180

cgctacgtaa attccgcccc ccccccctct ccctcccccc cccctaacgt tactggccga 6240

agccgcttgg aataaggccg gtgtgcgttt gtctatatgt tattttccac catattgccg 6300

tcttttggca atgtgagggc ccggaaacct ggccctgtct tcttgacgag cattcctagg 6360

ggtctttccc ctctcgccaa aggaatgcaa ggtctgttga atgtcgtgaa ggaagcagtt 6420

cctctggaag cttcttgaag acaaacaacg tctgtagcga ccctttgcag gcagcggaac 6480

cccccacctg gcgacaggtg cctctgcggc caaaagccac gtgtataaga tacacctgca 6540

aaggcggcac aaccccagtg ccacgttgtg agttggatag ttgtggaaag agtcaaatgg 6600

ctctcctcaa gcgtattcaa caaggggctg aaggatgccc agaaggtacc ccattgtatg 6660

ggatctgatc tggggcctcg gtgcacatgc tttacatgtg tttagtcgag gttaaaaaac 6720

gtctaggccc cccgaaccac ggggacgtgg ttttcctttg aaaaacacga tgataatatg 6780

gccaccaccc atatgaaaga aaccgctgct gctaaattcg aacgccagca catggacagc 6840

ccagatcagg gtaccctggt gccacgcggt tccatggtga gcaagggcga ggagctgttc 6900

accggggtgg tgcccatcct ggtcgagctg gacggcgacg taaacggcca caagttcagc 6960
gtgtccggcg agggcgaggg cgatgccacc tacggcaagc tgaccctgaa gttcatctgc 7020
accaccggca agctgcccgt gccctggccc accctcgtga ccaccctgac ctacggcgtg 7080
cagtgcttca gccgctaccc cgaccacatg aagcagcacg acttcttcaa gtccgccatg 7140
cccgaaggct acgtccagga gcgcaccatc ttcttcaagg acgacggcaa ctacaagacc 7200
cgcgccgagg tgaagttcga gggcgacacc ctggtgaacc gcatcgagct gaagggcatc 7260
gacttcaagg aggacggcaa catcctgggg cacaagctgg agtacaacta caacagccac 7320
aacgtctata tcatggccga caagcagaag aacggcatca aggtgaactt caagatccgc 7380
cacaacatcg aggacggcag cgtgcagctc gccgaccact accagcagaa cacccccatc 7440
ggcgacggcc ccgtgctgct gcccgacaac cactacctga gcacccagtc cgccctgagc 7500
aaagacccca acgagaagcg cgatcacatg gtcctgctgg agttcgtgac cgccgccggg 7560
atcactctcg gcatggacga gctgtacaag taaagcggcc gccagcacag tggtcgacga 7620
taaaataaaa gattttattt agtctccaga aaaagggggg aatgaaagac cccacctgta 7680
ggtttggcaa gctagcttaa gtaacgccat tttgcaaggc atggaaaaat acataactga 7740
gaatagagaa gttcagatca aggtcaggaa cagatggaac agctgaatat gggccaaaca 7800
ggatatctgt ggtaagcagt tcctgccccg gctcagggcc aagaacagat ggaacagctg 7860
aatatgggcc aaacaggata tctgtggtaa gcagttcctg ccccggctca gggccaagaa 7920
cagatggtcc ccagatgcgg tccagccctc agcagtttct agagaaccat cagatgtttc 7980
cagggtgccc caaggacctg aaatgaccct gtgccttatt tgaactaacc aatcagttcg 8040
cttctcgctt ctgttcgcgc gcttctgctc cccgagctca ataaaagagc ccacaacccc 8100
tcactcgggg cgccagtcct ccgattgact gagtcgcccg ggtacccgtg tatccaataa 8160
accctcttgc agttgcatcc gacttgtggt ctcgctgttc cttgggaggg tctcctctga 8220
gtgattgact acccgtcagc gggggtcttt cacatgcagc atgtatcaaa attaatttgg 8280
ttttttttct taagtattta cattaaatgg ccatagttgc attaatgaat cggccaacgc 8340
gcggggagag gcggtttgcg tattgggcgc tcttccgctt cctcgctcac tgactcgctg 8400
cgctcggtcg ttcggctgcg gcgagcggta tcagctcact caaaggcggt aatacggtta 8460
tccacagaat caggggataa cgcaggaaag aacatgtgag caaaaggcca gcaaaaggcc 8520
aggaaccgta aaaaggccgc gttgctggcg tttttccata ggctccgccc ccctgacgag 8580
catcacaaaa atcgacgctc aagtcagagg tggcgaaacc cgacaggact ataaagatac 8640
caggcgtttc cccctggaag ctccctcgtg cgctctcctg ttccgaccct gccgcttacc 8700
ggatacctgt ccgcctttct cccttcggga agcgtggcgc tttctcatag ctcacgctgt 8760
aggtatctca gttcggtgta ggtcgttcgc tccaagctgg gctgtgtgca cgaacccccc 8820

gttcagcccg accgctgcgc cttatccggt aactatcgtc ttgagtccaa cccggtaaga 8880

cacgacttat cgccactggc agcagccact ggtaacagga ttagcagagc gaggtatgta 8940

ggcggtgcta cagagttctt gaagtggtgg cctaactacg gctacactag aaggacagta 9000

tttggtatct gcgctctgct gaagccagtt accttcggaa aaagagttgg tagctcttga 9060

tccggcaaac aaaccaccgc tggtagcggt ggtttttttg tttgcaagca gcagattacg 9120

cgcagaaaaa aaggatctca agaagatcct ttgatctttt ctacggggtc tgacgctcag 9180

tggaacgaaa actcacgtta agggattttg gtcatgagat tatcaaaaag gatcttcacc 9240

tagatccttt tgcggccggc cgcaaatcaa tctaaagtat atatgagtaa acttggtctg 9300

acagttacca atgcttaatc agtgaggcac ctatctcagc gatctgtcta tttcgttcat 9360

ccatagttgc ctgactcccc gtcgtgtaga taactacgat acgggagggc ttaccatctg 9420

gccccagtgc tgcaatgata ccgcgagacc cacgctcacc ggctccagat ttatcagcaa 9480

taaaccagcc agccggaagg gccgagcgca gaagtggtcc tgcaacttta tccgcctcca 9540

tccagtctat taattgttgc cgggaagcta gagtaagtag ttcgccagtt aatagtttgc 9600

gcaacgttgt tgccattgct acaggcatcg tggtgtcacg ctcgtcgttt ggtatggctt 9660

cattcagctc cggttcccaa cgatcaaggc gagttacatg atcccccatg ttgtgcaaaa 9720

aagcggttag ctccttcggt cctccgatcg ttgtcagaag taagttggcc gcagtgttat 9780

cactcatggt tatggcagca ctgcataatt ctcttactgt catgccatcc gtaagatgct 9840

tttctgtgac tggtgagtac tcaaccaagt cattctgaga atagtgtatg cggcgaccga 9900

gttgctcttg cccggcgtca acacgggata ataccgcgcc acatagcaga actttaaaag 9960

tgctcatcat tggaaaacgt tcttcggggc gaaaactctc aaggatctta ccgctgttga 10020

gatccagttc gatgtaaccc actcgtgcac ccaactgatc ttcagcatct tttactttca 10080

ccagcgtttc tgggtgagca aaaacaggaa ggcaaaatgc cgcaaaaaag ggaataaggg 10140

cgacacggaa atgttgaata ctcatactct tcctttttca atattattga agcatttatc 10200

agggttattg tctcatgagc ggatacatat ttgaatgtat ttagaaaaat aaacaaatag 10260

gggttccgcg cacatttccc cgaaaagtgc cacctg 10296

<210> 17
<211> 19
<212> DNA
<213> Homo sapiens

<400> 17
aaaatcaagt ggggcgatg 19

<210> 18
<211> 20

<212>  DNA
<213>  Homo sapiens

<400>  18
atggcatgga ctgtggtcat                                                    20


<210>  19
<211>  20
<212>  DNA
<213>  Homo sapiens

<400>  19
gctggggctt ccaaaatgtg                                                    20


<210>  20
<211>  20
<212>  DNA
<213>  Homo sapiens

<400>  20
ccctttacgc tgcgtttgat                                                    20


<210>  21
<211>  20
<212>  DNA
<213>  Homo sapiens

<400>  21
taactcggct gaagccatgc                                                    20


<210>  22
<211>  20
<212>  DNA
<213>  Homo sapiens

<400>  22
cttgaggcga ggacccatac                                                    20


<210>  23
<211>  22
<212>  DNA
<213>  Mus musculus

<400>  23
agactgatac atacgcctgc ag                                                 22


<210>  24
<211>  22
<212>  DNA
<213>  Mus musculus

<400>  24
gcaggttcaa atgaatcttc ag                                                 22


<210>  25
<211>  20

<212>  DNA
<213>  Mus musculus

<400>  25
aacatattcc atggccaggg                                                    20


<210>  26
<211>  20
<212>  DNA
<213>  Mus musculus

<400>  26
ggatggcttc agaactggca                                                    20


<210>  27
<211>  20
<212>  DNA
<213>  Mus musculus

<400>  27
ggttgccaag ccttatcgga                                                    20


<210>  28
<211>  20
<212>  DNA
<213>  Mus musculus

<400>  28
acctgctcca ctgccttgct                                                    20


<210>  29
<211>  20
<212>  DNA
<213>  Mus musculus

<400>  29
aaaggccgga caccagtttt                                                    20


<210>  30
<211>  20
<212>  DNA
<213>  Mus musculus

<400>  30
ccggaggcga ttaaggttga                                                    20


<210>  31
<211>  20
<212>  DNA
<213>  Mus musculus

<400>  31
cactacggtg ttcgcacttg                                                    20


<210>  32
<211>  20

<212>  DNA
<213>  Mus musculus

<400>  32
catcccaaca gctaggcact                                                    20


<210>  33
<211>  19
<212>  DNA
<213>  Mus musculus

<400>  33
gacaacactc ttgacgctg                                                     19


<210>  34
<211>  20
<212>  DNA
<213>  Mus musculus

<400>  34
gatgatctcc cctgcctttg                                                    20


<210>  35
<211>  39
<212>  DNA
<213>  Mus musculus

<400>  35
acgcgtcgac aagcttcaga actggcaatg aacaattcc                               39


<210>  36
<211>  42
<212>  DNA
<213>  Mus musculus

<400>  36
cgcggatcct ctagatcaca tgaaaaattc gggagaggaa gg                           42


<210>  37
<211>  18
<212>  DNA
<213>  Unknown

<220>
<223>  Artificial sequence

<400>  37
tcggaaatat accaaagc                                                      18


<210>  38
<211>  35
<212>  DNA
<213>  Unknown

<220>
<223>  Artifical sequence

<400>  38
ttttcctttt gcggccgctt ctttgacgtg cttgg                                35

<210>  39
<211>  9
<212>  PRT
<213>  Unknown

<220>
<223>  Unknown organism

<400>  39

Leu Pro Tyr Leu Gly Trp Leu Val Phe
1               5

<210>  40
<211>  9
<212>  PRT
<213>  Unknown

<220>
<223>  Unknown organism

<400>  40

Lys Ala Val Tyr Asn Phe Ala Thr Cys
1               5